# EUROPEAN PATENT APPLICATION

(11) **EP 2 284 270 A2**
(43) Date of publication of application: **16.02.2011**
(21) Application number: 10185435.4
(22) Date of filing: 13.12.2001
(51) Int. Cl.: C12N 15/12, C12N 15/10, C12N 15/62, C07K 14/47, C40B 40/08, C40B 50/06

(54) **Method for the identification and isolation of binding polypeptides from combinatorial libraries of proteins having the scaffold structure of C-type lectin-like domains**

(30) Priority: 13.12.2000 DK 200001872; 28.02.2001 US 272098 P
(62) Divisional of application: 09160663.2
(71) Applicant: Anaphore, Inc., La Jolla, CA 92037 (US)
(72) Inventor: Etzerodt, Michael, 8382, Hinnerup (DK); Holtet, Thor Las, 8410, Rønde (DK); Graversen, Niels Jonas Heilskov, 8230, Åbyhøj (DK); Thøgersen, Hans Christian, 8381, Mundelstrup (DK)
(74) Representative: Inspicos A/S

(57) **Abstract**

A novel family of protein libraries comprising CTLDs (C-type Lectin-Like Domains) in which internal polypeptide loopregions lining the ligand binding sites in CTLDs have been replaced with ensembles of completely or partially randomized polypeptide segments. Tetranectin CTLDs were chosen as framework for the preferred embodiment of the invention; and versatile phagemid vectors useful in the generation and manipulation of human and murine tetranectin CTLD libraries are disclosed as part of this invention. Tetranectin CTLDs in monomeric as well as in trimeric form are efficiently displayed as gene III fusions in fully functional form by the recombinant fd phage display vector. CTLD derivatives with affinity for new ligands may readily be isolated from libraries of vectors displaying CTLDs, in which loop-regions have been randomised, using one or more rounds of enrichment by screening or selection followed by amplification of the enriched subpopulation in each round. The efficiency with which protein products containing CTLDs with new binding properties can be produced, e.g. by bacterial expression and in vitro refolding, in mono-, tri-, or multimeric formats provides important advantages in terms of simplicity, cost and efficiency of generation, production and diagnostic or therapeutic applications in comparison to recombinant antibody derivatives.

SEQUENCE LISTING

<110> Anaphore, Inc.

<120> Combinatorial libraries of proteins having the scaffold structure
 of C-type lectin-like domains

<130> 16294EP02

<160> 91

<170> PatentIn version 3.5

<210> 1
<211> 571
<212> DNA
<213> Homo sapiens


<220>
<221> CDS
<222> (1)..(564)
<223> FX-htlec encoding insert

<400> 1
gga tcc atc gag ggt agg ggc gag cca cca acc cag aag ccc aag aag 48
Gly Ser Ile Glu Gly Arg Gly Glu Pro Pro Thr Gln Lys Pro Lys Lys
1               5                   10                  15

att gta aat gcc aag aaa gat gtt gtg aac aca aag atg ttt gag gag 96
Ile Val Asn Ala Lys Lys Asp Val Val Asn Thr Lys Met Phe Glu Glu
            20                  25                  30

ctc aag agc cgt ctg gac acc ctg gcc cag gag gtg gcc ctg ctg aag 144
Leu Lys Ser Arg Leu Asp Thr Leu Ala Gln Glu Val Ala Leu Leu Lys
        35                  40                  45

gag cag cag gcc ctg cag acg gtc gtc ctg aag ggg acc aag gtg cac 192
Glu Gln Gln Ala Leu Gln Thr Val Val Leu Lys Gly Thr Lys Val His
    50                  55                  60

atg aaa gtc ttt ctg gcc ttc acc cag acg aag acc ttc cac gag gcc 240
Met Lys Val Phe Leu Ala Phe Thr Gln Thr Lys Thr Phe His Glu Ala
65                  70                  75                  80

agc gag gac tgc atc tcg cgc ggg ggc acc ctg agc acc cct cag act 288
Ser Glu Asp Cys Ile Ser Arg Gly Gly Thr Leu Ser Thr Pro Gln Thr
                85                  90                  95

ggc tcg gag aac gac gcc ctg tat gag tac ctg cgc cag agc gtg ggc 336
Gly Ser Glu Asn Asp Ala Leu Tyr Glu Tyr Leu Arg Gln Ser Val Gly
            100                 105                 110

aac gag gcc gag atc tgg ctg ggc ctc aac gac atg gcg gcc gag ggc 384
Asn Glu Ala Glu Ile Trp Leu Gly Leu Asn Asp Met Ala Ala Glu Gly
        115                 120                 125

acc tgg gtg gac atg acc ggt acc cgc atc gcc tac aag aac tgg gag 432
Thr Trp Val Asp Met Thr Gly Thr Arg Ile Ala Tyr Lys Asn Trp Glu
    130                 135                 140

act gag atc acc gcg caa ccc gat ggc ggc aag acc gag aac tgc gcg 480
Thr Glu Ile Thr Ala Gln Pro Asp Gly Gly Lys Thr Glu Asn Cys Ala
145                 150                 155                 160

gtc ctg tca ggc gcg gcc aac ggc aag tgg ttc gac aag cgc tgc cgc 528
Val Leu Ser Gly Ala Ala Asn Gly Lys Trp Phe Asp Lys Arg Cys Arg
                165                 170                 175

gat caa ttg ccc tac atc tgc cag ttc ggg atc gtg taagctt 571
Asp Gln Leu Pro Tyr Ile Cys Gln Phe Gly Ile Val
            180                 185

<210> 2
<211> 188
<212> PRT
<213> Homo sapiens

<400> 2

Gly Ser Ile Glu Gly Arg Gly Glu Pro Pro Thr Gln Lys Pro Lys Lys
1               5                   10                  15

Ile Val Asn Ala Lys Lys Asp Val Val Asn Thr Lys Met Phe Glu Glu
            20                  25                  30

Leu Lys Ser Arg Leu Asp Thr Leu Ala Gln Glu Val Ala Leu Leu Lys
        35                  40                  45

Glu Gln Gln Ala Leu Gln Thr Val Val Leu Lys Gly Thr Lys Val His
    50                  55                  60

Met Lys Val Phe Leu Ala Phe Thr Gln Thr Lys Thr Phe His Glu Ala
65                  70                  75                  80

Ser Glu Asp Cys Ile Ser Arg Gly Gly Thr Leu Ser Thr Pro Gln Thr
                85                  90                  95

Gly Ser Glu Asn Asp Ala Leu Tyr Glu Tyr Leu Arg Gln Ser Val Gly
            100                 105                 110

Asn Glu Ala Glu Ile Trp Leu Gly Leu Asn Asp Met Ala Ala Glu Gly
        115                 120                 125

Thr Trp Val Asp Met Thr Gly Thr Arg Ile Ala Tyr Lys Asn Trp Glu
    130                 135                 140

Thr Glu Ile Thr Ala Gln Pro Asp Gly Gly Lys Thr Glu Asn Cys Ala
145                 150                 155                 160

Val Leu Ser Gly Ala Ala Asn Gly Lys Trp Phe Asp Lys Arg Cys Arg
                165                 170                 175

Asp Gln Leu Pro Tyr Ile Cys Gln Phe Gly Ile Val
            180                 185

<210> 3
<211> 436
<212> DNA
<213> Homo sapiens

<220>

<221>  CDS
<222>  (1)..(429)
<223>  FX-htCTLD encoding insert

<400>  3
gga tcc atc gag ggt agg gcc ctg cag acg gtc gtc ctg aag ggg acc      48
Gly Ser Ile Glu Gly Arg Ala Leu Gln Thr Val Val Leu Lys Gly Thr
1               5                   10                  15

aag gtg cac atg aaa gtc ttt ctg gcc ttc acc cag acg aag acc ttc      96
Lys Val His Met Lys Val Phe Leu Ala Phe Thr Gln Thr Lys Thr Phe
            20                  25                  30

cac gag gcc agc gag gac tgc atc tcg cgc ggg ggc acc ctg agc acc      144
His Glu Ala Ser Glu Asp Cys Ile Ser Arg Gly Gly Thr Leu Ser Thr
        35                  40                  45

cct cag act ggc tcg gag aac gac gcc ctg tat gag tac ctg cgc cag      192
Pro Gln Thr Gly Ser Glu Asn Asp Ala Leu Tyr Glu Tyr Leu Arg Gln
    50                  55                  60

agc gtg ggc aac gag gcc gag atc tgg ctg ggc ctc aac gac atg gcg      240
Ser Val Gly Asn Glu Ala Glu Ile Trp Leu Gly Leu Asn Asp Met Ala
65                  70                  75                  80

gcc gag ggc acc tgg gtg gac atg acc ggt acc cgc atc gcc tac aag      288
Ala Glu Gly Thr Trp Val Asp Met Thr Gly Thr Arg Ile Ala Tyr Lys
                85                  90                  95

aac tgg gag act gag atc acc gcg caa ccc gat ggc ggc aag acc gag      336
Asn Trp Glu Thr Glu Ile Thr Ala Gln Pro Asp Gly Gly Lys Thr Glu
            100                 105                 110

aac tgc gcg gtc ctg tca ggc gcg gcc aac ggc aag tgg ttc gac aag      384
Asn Cys Ala Val Leu Ser Gly Ala Ala Asn Gly Lys Trp Phe Asp Lys
        115                 120                 125

cgc tgc cgc gat caa ttg ccc tac atc tgc cag ttc ggg atc gtg          429
Arg Cys Arg Asp Gln Leu Pro Tyr Ile Cys Gln Phe Gly Ile Val
    130                 135                 140

taagctt                                                              436


<210>  4
<211>  143
<212>  PRT
<213>  Homo sapiens

<400>  4

Gly Ser Ile Glu Gly Arg Ala Leu Gln Thr Val Val Leu Lys Gly Thr
1               5                   10                  15


Lys Val His Met Lys Val Phe Leu Ala Phe Thr Gln Thr Lys Thr Phe
            20                  25                  30


His Glu Ala Ser Glu Asp Cys Ile Ser Arg Gly Gly Thr Leu Ser Thr
        35                  40                  45


Pro Gln Thr Gly Ser Glu Asn Asp Ala Leu Tyr Glu Tyr Leu Arg Gln
    50                  55                  60


Ser Val Gly Asn Glu Ala Glu Ile Trp Leu Gly Leu Asn Asp Met Ala

65                    70                    75                    80

Ala Glu Gly Thr Trp Val Asp Met Thr Gly Thr Arg Ile Ala Tyr Lys
                85                  90                  95

Asn Trp Glu Thr Glu Ile Thr Ala Gln Pro Asp Gly Gly Lys Thr Glu
            100                 105                 110

Asn Cys Ala Val Leu Ser Gly Ala Ala Asn Gly Lys Trp Phe Asp Lys
        115                 120                 125

Arg Cys Arg Asp Gln Leu Pro Tyr Ile Cys Gln Phe Gly Ile Val
    130                 135                 140

<210>  5
<211>  47
<212>  DNA
<213>  Artificial Sequence

<220>
<223>  Description of Artificial Sequence: Primer

<400>  5
cggctgagcg gcccagccgg ccatggccga gccaccaacc cagaagc                    47

<210>  6
<211>  27
<212>  DNA
<213>  Artificial Sequence

<220>
<223>  Description of Artificial Sequence: Primer

<400>  6
cctgcggccg ccacgatccc gaactgg                                          27

<210>  7
<211>  43
<212>  DNA
<213>  Artificial Sequence

<220>
<223>  Description of Artificial Sequence: Primer

<400>  7
cggctgagcg gcccagccgg ccatggccgc cctgcagacg gtc                        43

<210>  8
<211>  570
<212>  DNA
<213>  Homo sapiens

<220>
<221>  CDS
<222>  (8)..(565)
<223>  PhTN encoding insert

<400>  8
ggcccag ccg gcc atg gcc gag cca cca acc cag aag ccc aag aag att        49

Pro Ala Met Ala Glu Pro Pro Thr Gln Lys Pro Lys Lys Ile
1               5                   10

gta aat gcc aag aaa gat gtt gtg aac aca aag atg ttt gag gag ctc     97
Val Asn Ala Lys Lys Asp Val Val Asn Thr Lys Met Phe Glu Glu Leu
15                  20                  25                  30

aag agc cgt ctg gac acc ctg gcc cag gag gtg gcc ctg ctg aag gag     145
Lys Ser Arg Leu Asp Thr Leu Ala Gln Glu Val Ala Leu Leu Lys Glu
                35                  40                  45

cag cag gcc ctg cag acg gtc tgc ctg aag ggg acc aag gtg cac atg     193
Gln Gln Ala Leu Gln Thr Val Cys Leu Lys Gly Thr Lys Val His Met
            50                  55                  60

aaa tgc ttt ctg gcc ttc acc cag acg aag acc ttc cac gag gcc agc     241
Lys Cys Phe Leu Ala Phe Thr Gln Thr Lys Thr Phe His Glu Ala Ser
        65                  70                  75

gag gac tgc atc tcg cgc ggg ggc acc ctg agc acc cct cag act ggc     289
Glu Asp Cys Ile Ser Arg Gly Gly Thr Leu Ser Thr Pro Gln Thr Gly
    80                  85                  90

tcg gag aac gac gcc ctg tat gag tac ctg cgc cag agc gtg ggc aac     337
Ser Glu Asn Asp Ala Leu Tyr Glu Tyr Leu Arg Gln Ser Val Gly Asn
95                  100                 105                 110

gag gcc gag atc tgg ctg ggc ctc aac gac atg gcg gcc gag ggc acc     385
Glu Ala Glu Ile Trp Leu Gly Leu Asn Asp Met Ala Ala Glu Gly Thr
                115                 120                 125

tgg gtg gac atg acc ggc gcc cgc atc gcc tac aag aac tgg gag act     433
Trp Val Asp Met Thr Gly Ala Arg Ile Ala Tyr Lys Asn Trp Glu Thr
            130                 135                 140

gag atc acc gcg caa ccc gat ggc ggc aag acc gag aac tgc gcg gtc     481
Glu Ile Thr Ala Gln Pro Asp Gly Gly Lys Thr Glu Asn Cys Ala Val
        145                 150                 155

ctg tca ggc gcg gcc aac ggc aag tgg ttc gac aag cgc tgc cgc gat     529
Leu Ser Gly Ala Ala Asn Gly Lys Trp Phe Asp Lys Arg Cys Arg Asp
    160                 165                 170

cag ctg ccc tac atc tgc cag ttc ggg atc gtg gcg gccgc               570
Gln Leu Pro Tyr Ile Cys Gln Phe Gly Ile Val Ala
175                 180                 185


<210>  9
<211>  186
<212>  PRT
<213>  Homo sapiens

<400>  9

Pro Ala Met Ala Glu Pro Pro Thr Gln Lys Pro Lys Lys Ile Val Asn
1               5                   10                  15


Ala Lys Lys Asp Val Val Asn Thr Lys Met Phe Glu Glu Leu Lys Ser
            20                  25                  30


Arg Leu Asp Thr Leu Ala Gln Glu Val Ala Leu Leu Lys Glu Gln Gln
        35                  40                  45


Ala Leu Gln Thr Val Cys Leu Lys Gly Thr Lys Val His Met Lys Cys

50                  55                  60

Phe Leu Ala Phe Thr Gln Thr Lys Thr Phe His Glu Ala Ser Glu Asp
65                  70                  75                  80

Cys Ile Ser Arg Gly Gly Thr Leu Ser Thr Pro Gln Thr Gly Ser Glu
                85                  90                  95

Asn Asp Ala Leu Tyr Glu Tyr Leu Arg Gln Ser Val Gly Asn Glu Ala
            100                 105                 110

Glu Ile Trp Leu Gly Leu Asn Asp Met Ala Ala Glu Gly Thr Trp Val
        115                 120                 125

Asp Met Thr Gly Ala Arg Ile Ala Tyr Lys Asn Trp Glu Thr Glu Ile
    130                 135                 140

Thr Ala Gln Pro Asp Gly Gly Lys Thr Glu Asn Cys Ala Val Leu Ser
145                 150                 155                 160

Gly Ala Ala Asn Gly Lys Trp Phe Asp Lys Arg Cys Arg Asp Gln Leu
                165                 170                 175

Pro Tyr Ile Cys Gln Phe Gly Ile Val Ala
            180                 185

<210>  10
<211>  438
<212>  DNA
<213>  Homo sapiens

<220>
<221>  CDS
<222>  (8)..(433)
<223>  PhTN3 encoding insert

<400>  10
ggcccag ccg gcc atg gcc gcc ctg cag acg gtc tgc ctg aag ggg acc      49
        Pro Ala Met Ala Ala Leu Gln Thr Val Cys Leu Lys Gly Thr
        1               5                   10

aag gtg cac atg aaa tgc ttt ctg gcc ttc acc cag acg aag acc ttc      97
Lys Val His Met Lys Cys Phe Leu Ala Phe Thr Gln Thr Lys Thr Phe
15                  20                  25                  30

cac gag gcc agc gag gac tgc atc tcg cgc ggg ggc acc ctg agc acc      145
His Glu Ala Ser Glu Asp Cys Ile Ser Arg Gly Gly Thr Leu Ser Thr
                35                  40                  45

cct cag act ggc tcg gag aac gac gcc ctg tat gag tac ctg cgc cag      193
Pro Gln Thr Gly Ser Glu Asn Asp Ala Leu Tyr Glu Tyr Leu Arg Gln
            50                  55                  60

agc gtg ggc aac gag gcc gag atc tgg ctg ggc ctc aac gac atg gcg      241
Ser Val Gly Asn Glu Ala Glu Ile Trp Leu Gly Leu Asn Asp Met Ala
        65                  70                  75

gcc gag ggc acc tgg gtg gac atg acc ggc gcc cgc atc gcc tac aag      289

Ala Glu Gly Thr Trp Val Asp Met Thr Gly Ala Arg Ile Ala Tyr Lys
    80                  85                  90

aac tgg gag act gag atc acc gcg caa ccc gat ggc ggc aag acc gag        337
Asn Trp Glu Thr Glu Ile Thr Ala Gln Pro Asp Gly Gly Lys Thr Glu
95                  100                 105                 110

aac tgc gcg gtc ctg tca ggc gcg gcc aac ggc aag tgg ttc gac aag        385
Asn Cys Ala Val Leu Ser Gly Ala Ala Asn Gly Lys Trp Phe Asp Lys
                115                 120                 125

cgc tgc cgc gat cag ctg ccc tac atc tgc cag ttc ggg atc gtg gcg        433
Arg Cys Arg Asp Gln Leu Pro Tyr Ile Cys Gln Phe Gly Ile Val Ala
            130                 135                 140

gccgc                                                                  438


<210>  11
<211>  142
<212>  PRT
<213>  Homo sapiens

<400>  11

Pro Ala Met Ala Ala Leu Gln Thr Val Cys Leu Lys Gly Thr Lys Val
1               5                   10                  15

His Met Lys Cys Phe Leu Ala Phe Thr Gln Thr Lys Thr Phe His Glu
            20                  25                  30

Ala Ser Glu Asp Cys Ile Ser Arg Gly Gly Thr Leu Ser Thr Pro Gln
        35                  40                  45

Thr Gly Ser Glu Asn Asp Ala Leu Tyr Glu Tyr Leu Arg Gln Ser Val
    50                  55                  60

Gly Asn Glu Ala Glu Ile Trp Leu Gly Leu Asn Asp Met Ala Ala Glu
65                  70                  75                  80

Gly Thr Trp Val Asp Met Thr Gly Ala Arg Ile Ala Tyr Lys Asn Trp
                85                  90                  95

Glu Thr Glu Ile Thr Ala Gln Pro Asp Gly Gly Lys Thr Glu Asn Cys
            100                 105                 110

Ala Val Leu Ser Gly Ala Ala Asn Gly Lys Trp Phe Asp Lys Arg Cys
        115                 120                 125

Arg Asp Gln Leu Pro Tyr Ile Cys Gln Phe Gly Ile Val Ala
    130                 135                 140


<210>  12
<211>  570
<212>  DNA
<213>  Homo sapiens


<220>

<221>  CDS
<222>  (8)..(565)
<223>  Phtlec encoding insert

<400>  12
ggcccag ccg gcc atg gcc gag cca cca acc cag aag ccc aag aag att      49
        Pro Ala Met Ala Glu Pro Pro Thr Gln Lys Pro Lys Lys Ile
        1               5                   10

gta aat gcc aag aaa gat gtt gtg aac aca aag atg ttt gag gag ctc      97
Val Asn Ala Lys Lys Asp Val Val Asn Thr Lys Met Phe Glu Glu Leu
15                  20                  25                  30

aag agc cgt ctg gac acc ctg gcc cag gag gtg gcc ctg ctg aag gag      145
Lys Ser Arg Leu Asp Thr Leu Ala Gln Glu Val Ala Leu Leu Lys Glu
                35                  40                  45

cag cag gcc ctg cag acg gtc gtc ctg aag ggg acc aag gtg cac atg      193
Gln Gln Ala Leu Gln Thr Val Val Leu Lys Gly Thr Lys Val His Met
            50                  55                  60

aaa gtc ttt ctg gcc ttc acc cag acg aag acc ttc cac gag gcc agc      241
Lys Val Phe Leu Ala Phe Thr Gln Thr Lys Thr Phe His Glu Ala Ser
        65                  70                  75

gag gac tgc atc tcg cgc ggg ggc acc ctg agc acc cct cag act ggc      289
Glu Asp Cys Ile Ser Arg Gly Gly Thr Leu Ser Thr Pro Gln Thr Gly
    80                  85                  90

tcg gag aac gac gcc ctg tat gag tac ctg cgc cag agc gtg ggc aac      337
Ser Glu Asn Asp Ala Leu Tyr Glu Tyr Leu Arg Gln Ser Val Gly Asn
95                  100                 105                 110

gag gcc gag atc tgg ctg ggc ctc aac gac atg gcg gcc gag ggc acc      385
Glu Ala Glu Ile Trp Leu Gly Leu Asn Asp Met Ala Ala Glu Gly Thr
                115                 120                 125

tgg gtg gac atg acc ggt acc cgc atc gcc tac aag aac tgg gag act      433
Trp Val Asp Met Thr Gly Thr Arg Ile Ala Tyr Lys Asn Trp Glu Thr
            130                 135                 140

gag atc acc gcg caa ccc gat ggc ggc aag acc gag aac tgc gcg gtc      481
Glu Ile Thr Ala Gln Pro Asp Gly Gly Lys Thr Glu Asn Cys Ala Val
        145                 150                 155

ctg tca ggc gcg gcc aac ggc aag tgg ttc gac aag cgc tgc cgc gat      529
Leu Ser Gly Ala Ala Asn Gly Lys Trp Phe Asp Lys Arg Cys Arg Asp
    160                 165                 170

caa ttg ccc tac atc tgc cag ttc ggg atc gtg gcg gccgc                570
Gln Leu Pro Tyr Ile Cys Gln Phe Gly Ile Val Ala
175                 180                 185


<210>  13
<211>  186
<212>  PRT
<213>  Homo sapiens

<400>  13

Pro Ala Met Ala Glu Pro Pro Thr Gln Lys Pro Lys Lys Ile Val Asn
1               5                   10                  15


Ala Lys Lys Asp Val Val Asn Thr Lys Met Phe Glu Glu Leu Lys Ser
            20                  25                  30

Arg Leu Asp Thr Leu Ala Gln Glu Val Ala Leu Leu Lys Glu Gln Gln
        35                  40                  45

Ala Leu Gln Thr Val Val Leu Lys Gly Thr Lys Val His Met Lys Val
    50                  55                  60

Phe Leu Ala Phe Thr Gln Thr Lys Thr Phe His Glu Ala Ser Glu Asp
65                  70                  75                  80

Cys Ile Ser Arg Gly Gly Thr Leu Ser Thr Pro Gln Thr Gly Ser Glu
                85                  90                  95

Asn Asp Ala Leu Tyr Glu Tyr Leu Arg Gln Ser Val Gly Asn Glu Ala
            100                 105                 110

Glu Ile Trp Leu Gly Leu Asn Asp Met Ala Ala Glu Gly Thr Trp Val
        115                 120                 125

Asp Met Thr Gly Thr Arg Ile Ala Tyr Lys Asn Trp Glu Thr Glu Ile
    130                 135                 140

Thr Ala Gln Pro Asp Gly Gly Lys Thr Glu Asn Cys Ala Val Leu Ser
145                 150                 155                 160

Gly Ala Ala Asn Gly Lys Trp Phe Asp Lys Arg Cys Arg Asp Gln Leu
                165                 170                 175

Pro Tyr Ile Cys Gln Phe Gly Ile Val Ala
            180                 185

<210>  14
<211>  438
<212>  DNA
<213>  Homo sapiens

<220>
<221>  CDS
<222>  (8)..(433)
<223>  PhtCTLD encoding insert

<400>  14
ggcccag ccg gcc atg gcc gcc ctg cag acg gtc gtc ctg aag ggg acc      49
        Pro Ala Met Ala Ala Leu Gln Thr Val Val Leu Lys Gly Thr
        1               5                   10

aag gtg cac atg aaa gtc ttt ctg gcc ttc acc cag acg aag acc ttc      97
Lys Val His Met Lys Val Phe Leu Ala Phe Thr Gln Thr Lys Thr Phe
15                  20                  25                  30

cac gag gcc agc gag gac tgc atc tcg cgc ggg ggc acc ctg agc acc      145
His Glu Ala Ser Glu Asp Cys Ile Ser Arg Gly Gly Thr Leu Ser Thr
                35                  40                  45

cct cag act ggc tcg gag aac gac gcc ctg tat gag tac ctg cgc cag      193
Pro Gln Thr Gly Ser Glu Asn Asp Ala Leu Tyr Glu Tyr Leu Arg Gln
            50                  55                  60

agc gtg ggc aac gag gcc gag atc tgg ctg ggc ctc aac gac atg gcg     241
Ser Val Gly Asn Glu Ala Glu Ile Trp Leu Gly Leu Asn Asp Met Ala
        65                  70                  75

gcc gag ggc acc tgg gtg gac atg acc ggt acc cgc atc gcc tac aag     289
Ala Glu Gly Thr Trp Val Asp Met Thr Gly Thr Arg Ile Ala Tyr Lys
    80                  85                  90

aac tgg gag act gag atc acc gcg caa ccc gat ggc ggc aag acc gag     337
Asn Trp Glu Thr Glu Ile Thr Ala Gln Pro Asp Gly Gly Lys Thr Glu
95                  100                 105                 110

aac tgc gcg gtc ctg tca ggc gcg gcc aac ggc aag tgg ttc gac aag     385
Asn Cys Ala Val Leu Ser Gly Ala Ala Asn Gly Lys Trp Phe Asp Lys
                115                 120                 125

cgc tgc cgc gat caa ttg ccc tac atc tgc cag ttc ggg atc gtg gcg     433
Arg Cys Arg Asp Gln Leu Pro Tyr Ile Cys Gln Phe Gly Ile Val Ala
            130                 135                 140

gccgc                                                               438


<210>  15
<211>  142
<212>  PRT
<213>  Homo sapiens

<400>  15

Pro Ala Met Ala Ala Leu Gln Thr Val Val Leu Lys Gly Thr Lys Val
1               5                   10                  15

His Met Lys Val Phe Leu Ala Phe Thr Gln Thr Lys Thr Phe His Glu
            20                  25                  30

Ala Ser Glu Asp Cys Ile Ser Arg Gly Gly Thr Leu Ser Thr Pro Gln
        35                  40                  45

Thr Gly Ser Glu Asn Asp Ala Leu Tyr Glu Tyr Leu Arg Gln Ser Val
    50                  55                  60

Gly Asn Glu Ala Glu Ile Trp Leu Gly Leu Asn Asp Met Ala Ala Glu
65                  70                  75                  80

Gly Thr Trp Val Asp Met Thr Gly Thr Arg Ile Ala Tyr Lys Asn Trp
                85                  90                  95

Glu Thr Glu Ile Thr Ala Gln Pro Asp Gly Gly Lys Thr Glu Asn Cys
            100                 105                 110

Ala Val Leu Ser Gly Ala Ala Asn Gly Lys Trp Phe Asp Lys Arg Cys
        115                 120                 125

Arg Asp Gln Leu Pro Tyr Ile Cys Gln Phe Gly Ile Val Ala
    130                 135                 140

<210>  16

<211>  555
<212>  DNA
<213>  Mus musculus

<400>  16
ggaattcgag tcacccactc ccaaggccaa gaaggctgca aatgccaaga aagatttggt      60

gagctcaaag atgtcgagga gctcaagaac aggatggatg tcctggccca ggaggtggcc     120

ctgctgaagg agaagcaggc cttacagact gtggtcctga agggcaccaa ggtgaacttg     180

aaggtcctcc tggccttcac ccaaccgaag accttccatg aggcgagcga ggactgcatc     240

tcgcaagggg gcacgctggg caccccgcag tcagagctag agaacgaggc gctgttcgag     300

tacgcgcgcc acagcgtggg caacgatgcg gagatctggc tgggcctcaa cgacatggcc     360

gcggaaggcg cctgggtgga catgaccggt accctcctgg cctacaagaa ctgggagacg     420

gagatcacga cgcaacccga cggcggcaaa gccgagaact gcgccgccct gtctggcgca     480

gccaacggca agtggttcga caagcgatgc cgcgatcaat tgccctacat ctgccagttt     540

gccattgtga agctt                                                      555

<210>  17
<211>  77
<212>  DNA
<213>  Artificial Sequence

<220>
<223>  Description of Artificial Sequence: oligonucleotide

<400>  17
cggaattcga gtcacccact cccaaggcca agaaggctgc aaatgccaag aaagatttgg      60

tgagctcaaa gatgttc                                                     77

<210>  18
<211>  94
<212>  DNA
<213>  Artificial Sequence

<220>
<223>  Description of Artificial Sequence: oligonucleotide

<400>  18
gcggatccag gcctgcttct ccttcagcag ggccacctcc tgggccagga catccatcct      60

gttcttgagc tcctcgaaca tctttgagct cacc                                  94

<210>  19
<211>  97
<212>  DNA
<213>  Artificial Sequence

<220>
<223>  Description of Artificial Sequence: oligonucleotide

<400>  19
gcaggcctta cagactgtgt gcctgaaggg caccaaggtg aacttgaagt gcctcctggc      60

cttcacccaa ccgaagacct tccatgaggc gagcgag                               97

<210> 20
<211> 93
<212> DNA
<213> Artificial Sequence

<220>
<223> Description of Artificial Sequence: oligonucleotide

<400> 20
ccgcatgctt cgaacagcgc ctcgttctct agctctgact gcggggtgcc cagcgtgccc 60

ccttgcgaga tgcagtcctc gctcgcctca tgg 93

<210> 21
<211> 61
<212> DNA
<213> Artificial Sequence

<220>
<223> Description of Artificial Sequence: oligonucleotide

<400> 21
ggttcgaata cgcgcgccac agcgtgggca acgatgcgga gatctaaatg ctcccaattg 60

c 61

<210> 22
<211> 55
<212> DNA
<213> Artificial Sequence

<220>
<223> Description of Artificial Sequence: oligonucleotide

<400> 22
ccaagcttca caatggcaaa ctggcagatg tagggcaatt gggagcattt agatc 55

<210> 23
<211> 86
<212> DNA
<213> Artificial Sequence

<220>
<223> Description of Artificial Sequence: oligonucleotide

<400> 23
cggagatctg gctgggcctc aacgacatgg ccgcggaagg cgcctgggtg gacatgaccg 60

gtaccctcct ggcctacaag aactgg 86

<210> 24
<211> 130
<212> DNA
<213> Artificial Sequence

<220>
<223> Description of Artificial Sequence: oligonucleotide

<400> 24
gggcaattga tcgcggcatc gcttgtcgaa cctcttgccg ttggctgcgc cagacagggc 60

ggcgcagttc tcggctttgc cgccgtcggg ttgcgtcgtg atctccgtct cccagttctt 120

gtaggccagg 130

<210>  25
<211>  40
<212>  DNA
<213>  Artificial Sequence

<220>
<223>  Description of Artificial Sequence: Primer

<400>  25
ctgggatcca tccagggtcg cgagtcaccc actcccaagg                              40


<210>  26
<211>  27
<212>  DNA
<213>  Artificial Sequence

<220>
<223>  Description of Artificial Sequence: Primer

<400>  26
ccgaagctta cacaatggca aactggc                                            27


<210>  27
<211>  39
<212>  DNA
<213>  Artificial Sequence

<220>
<223>  Description of Artificial Sequence: Primer

<400>  27
ctgggatcca tccagggtcg cgccttacag actgtggtc                               39


<210>  28
<211>  568
<212>  DNA
<213>  Mus musculus


<220>
<221>  CDS
<222>  (1)..(561)
<223>  FX-mtlec encoding insert

<400>  28
gga tcc atc cag ggt cgc gag tca ccc act ccc aag gcc aag aag gct      48
Gly Ser Ile Gln Gly Arg Glu Ser Pro Thr Pro Lys Ala Lys Lys Ala
1               5                   10                  15

gca aat gcc aag aaa gat ttg gtg agc tca aag atg ttc gag gag ctc      96
Ala Asn Ala Lys Lys Asp Leu Val Ser Ser Lys Met Phe Glu Glu Leu
            20                  25                  30

aag aac agg atg gat gtc ctg gcc cag gag gtg gcc ctg ctg aag gag     144
Lys Asn Arg Met Asp Val Leu Ala Gln Glu Val Ala Leu Leu Lys Glu
        35                  40                  45

aag cag gcc tta cag act gtg gtc ctg aag ggc acc aag gtg aac ttg     192
Lys Gln Ala Leu Gln Thr Val Val Leu Lys Gly Thr Lys Val Asn Leu
    50                  55                  60

aag gtc ctc ctg gcc ttc acc caa ccg aag acc ttc cat gag gcg agc     240
Lys Val Leu Leu Ala Phe Thr Gln Pro Lys Thr Phe His Glu Ala Ser

65                  70                  75                  80

gag gac tgc atc tcg caa ggg ggc acg ctg ggc acc ccg cag tca gag      288
Glu Asp Cys Ile Ser Gln Gly Gly Thr Leu Gly Thr Pro Gln Ser Glu
                85                  90                  95

cta gag aac gag gcg ctg ttc gag tac gcg cgc cac agc gtg ggc aac      336
Leu Glu Asn Glu Ala Leu Phe Glu Tyr Ala Arg His Ser Val Gly Asn
            100                 105                 110

gat gcg gag atc tgg ctg ggc ctc aac gac atg gcc gcg gaa ggc gcc      384
Asp Ala Glu Ile Trp Leu Gly Leu Asn Asp Met Ala Ala Glu Gly Ala
        115                 120                 125

tgg gtg gac atg acc ggt acc ctc ctg gcc tac aag aac tgg gag acg      432
Trp Val Asp Met Thr Gly Thr Leu Leu Ala Tyr Lys Asn Trp Glu Thr
    130                 135                 140

gag atc acg acg caa ccc gac ggc ggc aaa gcc gag aac tgc gcc gcc      480
Glu Ile Thr Thr Gln Pro Asp Gly Gly Lys Ala Glu Asn Cys Ala Ala
145                 150                 155                 160

ctg tct ggc gca gcc aac ggc aag tgg ttc gac aag cga tgc cgc gat      528
Leu Ser Gly Ala Ala Asn Gly Lys Trp Phe Asp Lys Arg Cys Arg Asp
                165                 170                 175

caa ttg ccc tac atc tgc cag ttt gcc att gtg taagctt                  568
Gln Leu Pro Tyr Ile Cys Gln Phe Ala Ile Val
            180                 185


<210>  29
<211>  187
<212>  PRT
<213>  Mus musculus

<400>  29

Gly Ser Ile Gln Gly Arg Glu Ser Pro Thr Pro Lys Ala Lys Lys Ala
1               5                   10                  15


Ala Asn Ala Lys Lys Asp Leu Val Ser Ser Lys Met Phe Glu Glu Leu
            20                  25                  30


Lys Asn Arg Met Asp Val Leu Ala Gln Glu Val Ala Leu Leu Lys Glu
        35                  40                  45


Lys Gln Ala Leu Gln Thr Val Val Leu Lys Gly Thr Lys Val Asn Leu
    50                  55                  60


Lys Val Leu Leu Ala Phe Thr Gln Pro Lys Thr Phe His Glu Ala Ser
65                  70                  75                  80


Glu Asp Cys Ile Ser Gln Gly Gly Thr Leu Gly Thr Pro Gln Ser Glu
                85                  90                  95


Leu Glu Asn Glu Ala Leu Phe Glu Tyr Ala Arg His Ser Val Gly Asn
            100                 105                 110


Asp Ala Glu Ile Trp Leu Gly Leu Asn Asp Met Ala Ala Glu Gly Ala
        115                 120                 125

Trp Val Asp Met Thr Gly Thr Leu Leu Ala Tyr Lys Asn Trp Glu Thr
    130                 135                 140

Glu Ile Thr Thr Gln Pro Asp Gly Gly Lys Ala Glu Asn Cys Ala Ala
145                 150                 155                 160

Leu Ser Gly Ala Ala Asn Gly Lys Trp Phe Asp Lys Arg Cys Arg Asp
                165                 170                 175

Gln Leu Pro Tyr Ile Cys Gln Phe Ala Ile Val
            180                 185

<210> 30
<211> 436
<212> DNA
<213> Mus musculus

<220>
<221> CDS
<222> (1)..(429)
<223> FX-mtCTLD encoding insert

<400> 30
gga tcc atc cag ggt cgc gcc tta cag act gtg gtc ctg aag ggc acc 48
Gly Ser Ile Gln Gly Arg Ala Leu Gln Thr Val Val Leu Lys Gly Thr
1               5                   10                  15

aag gtg aac ttg aag gtc ctc ctg gcc ttc acc caa ccg aag acc ttc 96
Lys Val Asn Leu Lys Val Leu Leu Ala Phe Thr Gln Pro Lys Thr Phe
            20                  25                  30

cat gag gcg agc gag gac tgc atc tcg caa ggg ggc acg ctg ggc acc 144
His Glu Ala Ser Glu Asp Cys Ile Ser Gln Gly Gly Thr Leu Gly Thr
        35                  40                  45

ccg cag tca gag cta gag aac gag gcg ctg ttc gag tac gcg cgc cac 192
Pro Gln Ser Glu Leu Glu Asn Glu Ala Leu Phe Glu Tyr Ala Arg His
    50                  55                  60

agc gtg ggc aac gat gcg gag atc tgg ctg ggc ctc aac gac atg gcc 240
Ser Val Gly Asn Asp Ala Glu Ile Trp Leu Gly Leu Asn Asp Met Ala
65                  70                  75                  80

gcg gaa ggc gcc tgg gtg gac atg acc ggt acc ctc ctg gcc tac aag 288
Ala Glu Gly Ala Trp Val Asp Met Thr Gly Thr Leu Leu Ala Tyr Lys
                85                  90                  95

aac tgg gag acg gag atc acg acg caa ccc gac ggc ggc aaa gcc gag 336
Asn Trp Glu Thr Glu Ile Thr Thr Gln Pro Asp Gly Gly Lys Ala Glu
            100                 105                 110

aac tgc gcc gcc ctg tct ggc gca gcc aac ggc aag tgg ttc gac aag 384
Asn Cys Ala Ala Leu Ser Gly Ala Ala Asn Gly Lys Trp Phe Asp Lys
        115                 120                 125

cga tgc cgc gat caa ttg ccc tac atc tgc cag ttt gcc att gtg 429
Arg Cys Arg Asp Gln Leu Pro Tyr Ile Cys Gln Phe Ala Ile Val
    130                 135                 140

taagctt 436

<210> 31
<211> 143
<212> PRT
<213> Mus musculus

<400> 31

Gly Ser Ile Gln Gly Arg Ala Leu Gln Thr Val Val Leu Lys Gly Thr
1               5                   10                  15

Lys Val Asn Leu Lys Val Leu Leu Ala Phe Thr Gln Pro Lys Thr Phe
            20                  25                  30

His Glu Ala Ser Glu Asp Cys Ile Ser Gln Gly Gly Thr Leu Gly Thr
        35                  40                  45

Pro Gln Ser Glu Leu Glu Asn Glu Ala Leu Phe Glu Tyr Ala Arg His
    50                  55                  60

Ser Val Gly Asn Asp Ala Glu Ile Trp Leu Gly Leu Asn Asp Met Ala
65                  70                  75                  80

Ala Glu Gly Ala Trp Val Asp Met Thr Gly Thr Leu Leu Ala Tyr Lys
                85                  90                  95

Asn Trp Glu Thr Glu Ile Thr Thr Gln Pro Asp Gly Gly Lys Ala Glu
            100                 105                 110

Asn Cys Ala Ala Leu Ser Gly Ala Ala Asn Gly Lys Trp Phe Asp Lys
        115                 120                 125

Arg Cys Arg Asp Gln Leu Pro Tyr Ile Cys Gln Phe Ala Ile Val
    130                 135                 140

<210> 32
<211> 47
<212> DNA
<213> Artificial Sequence

<220>
<223> Description of Artificial Sequence: Primer

<400> 32
cggctgagcg gcccagccgg ccatggccga gtcacccact cccaagg 47

<210> 33
<211> 27
<212> DNA
<213> Artificial Sequence

<220>
<223> Description of Artificial Sequence: Primer

<400> 33
cctgcggccg ccacgatccc gaactgg 27

<210> 34
<211> 46
<212> DNA
<213> Artificial Sequence

<220>
<223> Description of Artificial Sequence: Primer

<400> 34
cggctgagcg gcccagccgg ccatggccgc cttacagact gtggtc 46

<210> 35
<211> 570
<212> DNA
<213> Mus musculus

<220>
<221> CDS
<222> (8)..(565)
<223> Pmtlec encoding insert

<400> 35
ggcccag ccg gcc atg gcc gag tca ccc act ccc aag gcc aag aag gct 49
Pro Ala Met Ala Glu Ser Pro Thr Pro Lys Ala Lys Lys Ala
1               5                   10

gca aat gcc aag aaa gat ttg gtg agc tca aag atg ttc gag gag ctc 97
Ala Asn Ala Lys Lys Asp Leu Val Ser Ser Lys Met Phe Glu Glu Leu
15                  20                  25                  30

aag aac agg atg gat gtc ctg gcc cag gag gtg gcc ctg ctg aag gag 145
Lys Asn Arg Met Asp Val Leu Ala Gln Glu Val Ala Leu Leu Lys Glu
                35                  40                  45

aag cag gcc tta cag act gtg gtc ctg aag ggc acc aag gtg aac ttg 193
Lys Gln Ala Leu Gln Thr Val Val Leu Lys Gly Thr Lys Val Asn Leu
            50                  55                  60

aag gtc ctc ctg gcc ttc acc caa ccg aag acc ttc cat gag gcg agc 241
Lys Val Leu Leu Ala Phe Thr Gln Pro Lys Thr Phe His Glu Ala Ser
        65                  70                  75

gag gac tgc atc tcg caa ggg ggc acg ctg ggc acc ccg cag tca gag 289
Glu Asp Cys Ile Ser Gln Gly Gly Thr Leu Gly Thr Pro Gln Ser Glu
    80                  85                  90

cta gag aac gag gcg ctg ttc gag tac gcg cgc cac agc gtg ggc aac 337
Leu Glu Asn Glu Ala Leu Phe Glu Tyr Ala Arg His Ser Val Gly Asn
95                  100                 105                 110

gat gcg gag atc tgg ctg ggc ctc aac gac atg gcc gcg gaa ggc gcc 385
Asp Ala Glu Ile Trp Leu Gly Leu Asn Asp Met Ala Ala Glu Gly Ala
                115                 120                 125

tgg gtg gac atg acc ggt acc ctc ctg gcc tac aag aac tgg gag acg 433
Trp Val Asp Met Thr Gly Thr Leu Leu Ala Tyr Lys Asn Trp Glu Thr
            130                 135                 140

gag atc acg acg caa ccc gac ggc ggc aaa gcc gag aac tgc gcc gcc 481
Glu Ile Thr Thr Gln Pro Asp Gly Gly Lys Ala Glu Asn Cys Ala Ala
        145                 150                 155

ctg tct ggc gca gcc aac ggc aag tgg ttc gac aag cga tgc cgc gat 529
Leu Ser Gly Ala Ala Asn Gly Lys Trp Phe Asp Lys Arg Cys Arg Asp
    160                 165                 170

caa ttg ccc tac atc tgc cag ttt gcc att gtg gcg gccgc                    570
Gln Leu Pro Tyr Ile Cys Gln Phe Ala Ile Val Ala
175                 180                 185

<210>  36
<211>  186
<212>  PRT
<213>  Mus musculus

<400>  36

Pro Ala Met Ala Glu Ser Pro Thr Pro Lys Ala Lys Lys Ala Ala Asn
1               5                   10                  15

Ala Lys Lys Asp Leu Val Ser Ser Lys Met Phe Glu Glu Leu Lys Asn
            20                  25                  30

Arg Met Asp Val Leu Ala Gln Glu Val Ala Leu Leu Lys Glu Lys Gln
        35                  40                  45

Ala Leu Gln Thr Val Val Leu Lys Gly Thr Lys Val Asn Leu Lys Val
    50                  55                  60

Leu Leu Ala Phe Thr Gln Pro Lys Thr Phe His Glu Ala Ser Glu Asp
65                  70                  75                  80

Cys Ile Ser Gln Gly Gly Thr Leu Gly Thr Pro Gln Ser Glu Leu Glu
                85                  90                  95

Asn Glu Ala Leu Phe Glu Tyr Ala Arg His Ser Val Gly Asn Asp Ala
            100                 105                 110

Glu Ile Trp Leu Gly Leu Asn Asp Met Ala Ala Glu Gly Ala Trp Val
        115                 120                 125

Asp Met Thr Gly Thr Leu Leu Ala Tyr Lys Asn Trp Glu Thr Glu Ile
    130                 135                 140

Thr Thr Gln Pro Asp Gly Gly Lys Ala Glu Asn Cys Ala Ala Leu Ser
145                 150                 155                 160

Gly Ala Ala Asn Gly Lys Trp Phe Asp Lys Arg Cys Arg Asp Gln Leu
                165                 170                 175

Pro Tyr Ile Cys Gln Phe Ala Ile Val Ala
            180                 185

<210>  37
<211>  438
<212>  DNA
<213>  Mus musculus

<220>
<221>  CDS

<222>   (8)..(433)
<223>   Pmt CTLD encoding insert

<400>   37
ggcccag ccg gcc atg gcc gcc tta cag act gtg gtc ctg aag ggc acc        49
        Pro Ala Met Ala Ala Leu Gln Thr Val Val Leu Lys Gly Thr
        1               5                   10

aag gtg aac ttg aag gtc ctc ctg gcc ttc acc caa ccg aag acc ttc        97
Lys Val Asn Leu Lys Val Leu Leu Ala Phe Thr Gln Pro Lys Thr Phe
15                  20                  25                  30

cat gag gcg agc gag gac tgc atc tcg caa ggg ggc acg ctg ggc acc        145
His Glu Ala Ser Glu Asp Cys Ile Ser Gln Gly Gly Thr Leu Gly Thr
                35                  40                  45

ccg cag tca gag cta gag aac gag gcg ctg ttc gag tac gcg cgc cac        193
Pro Gln Ser Glu Leu Glu Asn Glu Ala Leu Phe Glu Tyr Ala Arg His
            50                  55                  60

agc gtg ggc aac gat gcg gag atc tgg ctg ggc ctc aac gac atg gcc        241
Ser Val Gly Asn Asp Ala Glu Ile Trp Leu Gly Leu Asn Asp Met Ala
        65                  70                  75

gcg gaa ggc gcc tgg gtg gac atg acc ggt acc ctc ctg gcc tac aag        289
Ala Glu Gly Ala Trp Val Asp Met Thr Gly Thr Leu Leu Ala Tyr Lys
    80                  85                  90

aac tgg gag acg gag atc acg acg caa ccc gac ggc ggc aaa gcc gag        337
Asn Trp Glu Thr Glu Ile Thr Thr Gln Pro Asp Gly Gly Lys Ala Glu
95                  100                 105                 110

aac tgc gcc gcc ctg tct ggc gca gcc aac ggc aag tgg ttc gac aag        385
Asn Cys Ala Ala Leu Ser Gly Ala Ala Asn Gly Lys Trp Phe Asp Lys
                115                 120                 125

cga tgc cgc gat caa ttg ccc tac atc tgc cag ttt gcc att gtg gcg        433
Arg Cys Arg Asp Gln Leu Pro Tyr Ile Cys Gln Phe Ala Ile Val Ala
            130                 135                 140

gccgc                                                                  438


<210>   38
<211>   142
<212>   PRT
<213>   Mus musculus

<400>   38

Pro Ala Met Ala Ala Leu Gln Thr Val Val Leu Lys Gly Thr Lys Val
1               5                   10                  15

Asn Leu Lys Val Leu Leu Ala Phe Thr Gln Pro Lys Thr Phe His Glu
            20                  25                  30

Ala Ser Glu Asp Cys Ile Ser Gln Gly Gly Thr Leu Gly Thr Pro Gln
        35                  40                  45

Ser Glu Leu Glu Asn Glu Ala Leu Phe Glu Tyr Ala Arg His Ser Val
    50                  55                  60

Gly Asn Asp Ala Glu Ile Trp Leu Gly Leu Asn Asp Met Ala Ala Glu
65                  70                  75                  80

Gly Ala Trp Val Asp Met Thr Gly Thr Leu Leu Ala Tyr Lys Asn Trp
                85                  90                  95

Glu Thr Glu Ile Thr Thr Gln Pro Asp Gly Gly Lys Ala Glu Asn Cys
            100                 105                 110

Ala Ala Leu Ser Gly Ala Ala Asn Gly Lys Trp Phe Asp Lys Arg Cys
        115                 120                 125

Arg Asp Gln Leu Pro Tyr Ile Cys Gln Phe Ala Ile Val Ala
    130                 135                 140

<210>  39
<211>  116
<212>  DNA
<213>  Artificial Sequence

<220>
<223>  Description of Artificial Sequence: oligonucleotide

<220>
<221>  misc_feature
<222>  (17)..(18)
<223>  randomised

<220>
<221>  misc_feature
<222>  (20)..(21)
<223>  randomised

<220>
<221>  misc_feature
<222>  (23)..(24)
<223>  randomised

<220>
<221>  misc_feature
<222>  (26)..(27)
<223>  randomised

<220>
<221>  misc_feature
<222>  (29)..(30)
<223>  randomised

<220>
<221>  misc_feature
<222>  (32)..(33)
<223>  randomised

<220>
<221>  misc_feature
<222>  (44)..(45)
<223>  randomised

<220>
<221>  misc_feature
<222>  (47)..(48)
<223>  randomised

<220>

<221>   misc_feature
<222>   (50)..(51)
<223>   randomised

<220>
<221>   misc_feature
<222>   (53)..(54)
<223>   randomised

<220>
<221>   misc_feature
<222>   (98)..(99)
<223>   randomised

<400>   39
cgcctacaag aactggnnsn nsnnsnnsnn snnscaaccc gatnnsnnsn nsnnsgagaa      60

ctgcgcggtc ctgtcaggcg cggccaacgg caagtggnns gacaagcgct gccgcg         116


<210>   40
<211>   31
<212>   DNA
<213>   Artificial Sequence

<220>
<223>   Description of Artificial Sequence: oligonucleotide

<400>   40
gaccggtacc cgcatcgcct acaagaactg g                                     31


<210>   41
<211>   30
<212>   DNA
<213>   Artificial Sequence

<220>
<223>   Description of Artificial Sequence: oligonucleotide

<400>   41
gtagggcaat tgatcgcggc agcgcttgtc                                       30


<210>   42
<211>   94
<212>   DNA
<213>   Artificial Sequence

<220>
<223>   Description of Artificial Sequence: oligonucleotide


<220>
<221>   misc_feature
<222>   (17)..(18)
<223>   randomised

<220>
<221>   misc_feature
<222>   (20)..(21)
<223>   randomised

<220>
<221>   misc_feature
<222>   (23)..(24)
<223>   randomised

<220>
<221> misc_feature
<222> (29)..(30)
<223> randomised

<220>
<221> misc_feature
<222> (32)..(33)
<223> randomised

<400> 42
gctgggcctc aacgacnnsn nsnnsgagnn snnstgggtg gacatgaccg gtacccgcat 60

cgcctacaag aactgggaga ctgagatcac cgcg 94


<210> 43
<211> 102
<212> DNA
<213> Artificial Sequence

<220>
<223> Description of Artificial Sequence: oligonucleotide


<220>
<221> misc_feature
<222> (63)..(64)
<223> randomised

<220>
<221> misc_feature
<222> (66)..(67)
<223> randomised

<220>
<221> misc_feature
<222> (69)..(70)
<223> randomised

<220>
<221> misc_feature
<222> (72)..(73)
<223> randomised

<400> 43
cgcggcagcg cttgtcgaac cacttgccgt tggccgcgcc tgacaggacc gcgcagttct 60

csnnsnnsnn snnatcgggt tgcgcggtga tctcagtctc cc 102


<210> 44
<211> 31
<212> DNA
<213> Artificial Sequence

<220>
<223> Description of Artificial Sequence: oligonucleotide

<400> 44
cgaggccgag atctggctgg gcctcaacga c 31


<210> 45
<211> 31
<212> DNA
<213> Artificial Sequence

<220>
<223>  Description of Artificial Sequence: oligonucleotide

<400>  45
gggcaacgag gccgagatct ggctgggcct c                                    31


<210>  46
<211>  19
<212>  DNA
<213>  Artificial Sequence

<220>
<223>  Description of Artificial Sequence: oligonucleotide

<400>  46
cctgaccctg cagcgcttg                                                  19


<210>  47
<211>  81
<212>  DNA
<213>  Artificial Sequence

<220>
<223>  Description of Artificial Sequence: oligonucleotide


<220>
<221>  misc_feature
<222>  (26)..(27)
<223>  randomised

<220>
<221>  misc_feature
<222>  (29)..(30)
<223>  randomised

<220>
<221>  misc_feature
<222>  (32)..(33)
<223>  randomised

<220>
<221>  misc_feature
<222>  (35)..(36)
<223>  randomised

<220>
<221>  misc_feature
<222>  (38)..(39)
<223>  randomised

<220>
<221>  misc_feature
<222>  (41)..(42)
<223>  randomised

<400>  47
cgagatctgg ctgggcctca acgacnnsnn snnsnnsnns nnsgagggca cctgggtgga     60

catgaccggt acccgcatcg c                                               81


<210>  48
<211>  78
<212>  DNA
<213>  Artificial Sequence

<220>
<223>  Description of Artificial Sequence: oligonucleotide

<220>
<221>  misc_feature
<222>  (26)..(27)
<223>  randomised

<220>
<221>  misc_feature
<222>  (29)..(30)
<223>  randomised

<220>
<221>  misc_feature
<222>  (32)..(33)
<223>  randomised

<220>
<221>  misc_feature
<222>  (35)..(36)
<223>  randomised

<220>
<221>  misc_feature
<222>  (38)..(39)
<223>  randomised

<400>  48
cgagatctgg ctgggcctca acgacnnsnn snnsnnsnns gagggcacct gggtggacat      60

gaccggtacc cgcatcgc                                                    78

<210>  49
<211>  94
<212>  DNA
<213>  Artificial Sequence

<220>
<223>  Description of Artificial Sequence: oligonucleotide

<220>
<221>  misc_feature
<222>  (17)..(18)
<223>  randomised

<220>
<221>  misc_feature
<222>  (20)..(21)
<223>  randomised

<220>
<221>  misc_feature
<222>  (23)..(24)
<223>  randomised

<220>
<221>  misc_feature
<222>  (29)..(30)
<223>  randomised

<220>
<221>  misc_feature
<222>  (32)..(33)

<223>  randomised

<400>  49
gctgggcctc aacgacnnsn nsnnsgagnn snnstgggtg gacatgaccg gtacccgcat      60

cgcctacaag aactgggaga ctgagatcac cgcg                                  94


<210>  50
<211>  18
<212>  DNA
<213>  Artificial Sequence

<220>
<223>  Description of Artificial Sequence: oligonucleotide

<400>  50
gcgatgcggg taccggtc                                                    18


<210>  51
<211>  89
<212>  DNA
<213>  Artificial Sequence

<220>
<223>  Description of Artificial Sequence: oligonucleotide


<220>
<221>  misc_feature
<222>  (54)..(55)
<223>  randomised

<220>
<221>  misc_feature
<222>  (57)..(58)
<223>  randomised

<220>
<221>  misc_feature
<222>  (60)..(61)
<223>  randomised

<220>
<221>  misc_feature
<222>  (63)..(64)
<223>  randomised

<220>
<221>  misc_feature
<222>  (66)..(67)
<223>  randomised

<220>
<221>  misc_feature
<222>  (69)..(70)
<223>  randomised

<400>  51
gcatcgccta caagaactgg gagactgaga tcaccgcgca acccgatggc ggcnnsnnsn      60

nsnnsnnsnn sgagaactgc gcggtcctg                                        89


<210>  52
<211>  86
<212>  DNA

<213>  Artificial Sequence

<220>
<223>  Description of Artificial Sequence: oligonucleotide


<220>
<221>  misc_feature
<222>  (54)..(55)
<223>  randomised

<220>
<221>  misc_feature
<222>  (57)..(58)
<223>  randomised

<220>
<221>  misc_feature
<222>  (60)..(61)
<223>  randomised

<220>
<221>  misc_feature
<222>  (63)..(64)
<223>  randomised

<220>
<221>  misc_feature
<222>  (66)..(67)
<223>  randomised

<400>  52
gcatcgccta caagaactgg gagactgaga tcaccgcgca acccgatggc ggcnnsnnsn      60

nsnnsnnsga gaactgcgcg gtcctg                                           86


<210>  53
<211>  34
<212>  DNA
<213>  Artificial Sequence

<220>
<223>  Description of Artificial Sequence: oligonucleotide

<400>  53
catgaccggt acccgcatcg cctacaagaa ctgg                                  34


<210>  54
<211>  66
<212>  DNA
<213>  Artificial Sequence

<220>
<223>  Description of Artificial Sequence: oligonucleotide

<400>  54
cctgaccctg cagcgcttgt cgaaccactt gccgttggcc gcgcctgaca ggaccgcgca      60

gttctc                                                                 66


<210>  55
<211>  45
<212>  DNA
<213>  Artificial Sequence

<220>
<223>  Description of Artificial Sequence: oligonucleotide

<400>  55
ggtacctaag tgacgatatc ctgacctaac tgcagggatc aattg                    45


<210>  56
<211>  343
<212>  DNA
<213>  Homo sapiens


<220>
<221>  CDS
<222>  (8)..(274)
<223>  Human PhtCPB insert

<400>  56
ggcccag ccg gcc atg gcc gcc ctc cag acg gtc tgc ctg aag ggg acc      49
        Pro Ala Met Ala Ala Leu Gln Thr Val Cys Leu Lys Gly Thr
        1               5                   10

aag gtg cac atg aaa tgc ttt ctg gcc ttc acc cag acg aag acc ttc      97
Lys Val His Met Lys Cys Phe Leu Ala Phe Thr Gln Thr Lys Thr Phe
15                  20                  25                  30

cac gag gcc agc gag gac tgc atc tcg cgc ggg ggc acc ctg agc acc      145
His Glu Ala Ser Glu Asp Cys Ile Ser Arg Gly Gly Thr Leu Ser Thr
                35                  40                  45

cct cag act ggc tcg gag aac gac gcc ctg tat gag tac ctg cgc cag      193
Pro Gln Thr Gly Ser Glu Asn Asp Ala Leu Tyr Glu Tyr Leu Arg Gln
            50                  55                  60

agc gtg ggc aac gag gcc gag atc tgg ctg ggc ctc aac gac atg gcg      241
Ser Val Gly Asn Glu Ala Glu Ile Trp Leu Gly Leu Asn Asp Met Ala
        65                  70                  75

gcc gag ggc acc tgg gtg gac atg acc ggt acc taagtgacga tatcctgacc    294
Ala Glu Gly Thr Trp Val Asp Met Thr Gly Thr
    80                  85

taactgcagg gatcaattgc cctacatctg ccagttcggg atcgtgtag                343


<210>  57
<211>  89
<212>  PRT
<213>  Homo sapiens

<400>  57

Pro Ala Met Ala Ala Leu Gln Thr Val Cys Leu Lys Gly Thr Lys Val
1               5                   10                  15


His Met Lys Cys Phe Leu Ala Phe Thr Gln Thr Lys Thr Phe His Glu
            20                  25                  30


Ala Ser Glu Asp Cys Ile Ser Arg Gly Gly Thr Leu Ser Thr Pro Gln
        35                  40                  45


Thr Gly Ser Glu Asn Asp Ala Leu Tyr Glu Tyr Leu Arg Gln Ser Val
    50                  55                  60

Gly Asn Glu Ala Glu Ile Trp Leu Gly Leu Asn Asp Met Ala Ala Glu
65                  70                  75                  80

Gly Thr Trp Val Asp Met Thr Gly Thr
                85

<210>  58
<211>  405
<212>  DNA
<213>  Rattus rattus

<220>
<221>  CDS
<222>  (8)..(400)
<223>  Rat PrMBP insert

<400>  58
ggcccag ccg gcc atg gcc aac aag ttg cat gcc ttc tcc atg ggt aaa      49
        Pro Ala Met Ala Asn Lys Leu His Ala Phe Ser Met Gly Lys
        1               5                   10

aag tct ggg aag aag ttc ttt gtg acc aac cat gaa agg atg ccc ttt      97
Lys Ser Gly Lys Lys Phe Phe Val Thr Asn His Glu Arg Met Pro Phe
15                  20                  25                  30

tcc aaa gtc aag gcc ctg tgc tca gag ctc cga ggc act gtg gct atc      145
Ser Lys Val Lys Ala Leu Cys Ser Glu Leu Arg Gly Thr Val Ala Ile
                35                  40                  45

ccc aag aat gct gag gag aac aag gcc atc caa gaa gtg gct aaa acc      193
Pro Lys Asn Ala Glu Glu Asn Lys Ala Ile Gln Glu Val Ala Lys Thr
            50                  55                  60

tct gcc ttc cta ggc atc acg gac gag gtg act gaa ggc caa ttc atg      241
Ser Ala Phe Leu Gly Ile Thr Asp Glu Val Thr Glu Gly Gln Phe Met
        65                  70                  75

tat gtg aca ggg ggg agg ctc acc tac agc aac tgg aaa aag gat gag      289
Tyr Val Thr Gly Gly Arg Leu Thr Tyr Ser Asn Trp Lys Lys Asp Glu
    80                  85                  90

ccc aat gac cat ggc tct ggg gaa gac tgt gtc act ata gta gac aac      337
Pro Asn Asp His Gly Ser Gly Glu Asp Cys Val Thr Ile Val Asp Asn
95                  100                 105                 110

ggt ctg tgg aat gac atc tcc tgc caa gct tcc cac acg gct gtc tgc      385
Gly Leu Trp Asn Asp Ile Ser Cys Gln Ala Ser His Thr Ala Val Cys
                115                 120                 125

gag ttc cca gcc gcg gccgc                                            405
Glu Phe Pro Ala Ala
            130

<210>  59
<211>  131
<212>  PRT
<213>  Rattus rattus

<400>  59

Pro Ala Met Ala Asn Lys Leu His Ala Phe Ser Met Gly Lys Lys Ser
1               5                   10                  15

Gly Lys Lys Phe Phe Val Thr Asn His Glu Arg Met Pro Phe Ser Lys
            20                  25                  30

Val Lys Ala Leu Cys Ser Glu Leu Arg Gly Thr Val Ala Ile Pro Lys
        35                  40                  45

Asn Ala Glu Glu Asn Lys Ala Ile Gln Glu Val Ala Lys Thr Ser Ala
    50                  55                  60

Phe Leu Gly Ile Thr Asp Glu Val Thr Glu Gly Gln Phe Met Tyr Val
65                  70                  75                  80

Thr Gly Gly Arg Leu Thr Tyr Ser Asn Trp Lys Lys Asp Glu Pro Asn
                85                  90                  95

Asp His Gly Ser Gly Glu Asp Cys Val Thr Ile Val Asp Asn Gly Leu
            100                 105                 110

Trp Asn Asp Ile Ser Cys Gln Ala Ser His Thr Ala Val Cys Glu Phe
        115                 120                 125

Pro Ala Ala
    130

<210>  60
<211>  408
<212>  DNA
<213>  Homo sapiens

<220>
<221>  CDS
<222>  (8)..(403)
<223>  Human PhSP-D insert

<400>  60
ggcccag ccg gcc atg gcc aag aaa gtt gag ctc ttc cca aat ggc caa      49
        Pro Ala Met Ala Lys Lys Val Glu Leu Phe Pro Asn Gly Gln
        1               5                   10

agt gtg ggg gag aag att ttc aag aca gca ggc ttt gta aaa cca ttt      97
Ser Val Gly Glu Lys Ile Phe Lys Thr Ala Gly Phe Val Lys Pro Phe
15                  20                  25                  30

acg gag gca cag ctg ctg tgc aca cag gct ggt gga cag ttg gcc tct      145
Thr Glu Ala Gln Leu Leu Cys Thr Gln Ala Gly Gly Gln Leu Ala Ser
                35                  40                  45

cca cgc tct gcc gct gag aat gcc gcc ttg caa cag ctg gtc gta gct      193
Pro Arg Ser Ala Ala Glu Asn Ala Ala Leu Gln Gln Leu Val Val Ala
            50                  55                  60

aag aac gag gct gct ttc ctg agc atg act gat tcc aag aca gag ggc      241
Lys Asn Glu Ala Ala Phe Leu Ser Met Thr Asp Ser Lys Thr Glu Gly
        65                  70                  75

aag ttc acc tac ccc aca gga gag tcc ctg gtc tat tcc aac tgg gcc      289
Lys Phe Thr Tyr Pro Thr Gly Glu Ser Leu Val Tyr Ser Asn Trp Ala
    80                  85                  90

cca ggg gag ccc aac gat gat ggc ggg tca gag gac tgt gtg gag atc 337
Pro Gly Glu Pro Asn Asp Asp Gly Gly Ser Glu Asp Cys Val Glu Ile
95                  100                 105                 110

ttc acc aat ggc aag tgg aat gac agg gct tgt gga gaa aag cgt ctt 385
Phe Thr Asn Gly Lys Trp Asn Asp Arg Ala Cys Gly Glu Lys Arg Leu
                115                 120                 125

gtg gtc tgc gag ttc gcg gccgc 408
Val Val Cys Glu Phe Ala
            130

<210> 61
<211> 132
<212> PRT
<213> Homo sapiens

<400> 61

Pro Ala Met Ala Lys Lys Val Glu Leu Phe Pro Asn Gly Gln Ser Val
1               5                   10                  15

Gly Glu Lys Ile Phe Lys Thr Ala Gly Phe Val Lys Pro Phe Thr Glu
            20                  25                  30

Ala Gln Leu Leu Cys Thr Gln Ala Gly Gly Gln Leu Ala Ser Pro Arg
        35                  40                  45

Ser Ala Ala Glu Asn Ala Ala Leu Gln Gln Leu Val Val Ala Lys Asn
    50                  55                  60

Glu Ala Ala Phe Leu Ser Met Thr Asp Ser Lys Thr Glu Gly Lys Phe
65                  70                  75                  80

Thr Tyr Pro Thr Gly Glu Ser Leu Val Tyr Ser Asn Trp Ala Pro Gly
                85                  90                  95

Glu Pro Asn Asp Asp Gly Gly Ser Glu Asp Cys Val Glu Ile Phe Thr
            100                 105                 110

Asn Gly Lys Trp Asn Asp Arg Ala Cys Gly Glu Lys Arg Leu Val Val
        115                 120                 125

Cys Glu Phe Ala
    130

<210> 62
<211> 49
<212> DNA
<213> Artificial Sequence

<220>
<223> Description of Artificial Sequence: oligonucleotide

<400> 62
cggctgagcg gcccagccgg ccatggccaa caagttgcat gccttctcc 49

<210>  63
<211>  34
<212>  DNA
<213>  Artificial Sequence

<220>
<223>  Description of Artificial Sequence: oligonucleotide

<400>  63
gcactcctgc ggccgcggct gggaactcgc agac                                    34


<210>  64
<211>  48
<212>  DNA
<213>  Artificial Sequence

<220>
<223>  Description of Artificial Sequence: oligonucleotide

<400>  64
cggctgagcg gcccagccgg ccatggccaa gaaagttgag ctcttccc                     48


<210>  65
<211>  36
<212>  DNA
<213>  Artificial Sequence

<220>
<223>  Description of Artificial Sequence: oligonucleotide

<400>  65
gcactcctgc ggccgcgaac tcgcagacca caagac                                  36


<210>  66
<211>  65
<212>  DNA
<213>  Artificial Sequence

<220>
<223>  Description of Artificial Sequence: oligonucleotide


<220>
<221>  misc_feature
<222>  (32)..(33)
<223>  randomised

<220>
<221>  misc_feature
<222>  (35)..(36)
<223>  randomised

<220>
<221>  misc_feature
<222>  (38)..(39)
<223>  randomised

<220>
<221>  misc_feature
<222>  (41)..(42)
<223>  randomised

<220>
<221>  misc_feature

<222>  (44)..(45)
<223>  randomised

<400>  66
gccaccggtg acgtagatga attggccttc snnsnnsnns nnsnngtccg tgatgcctag      60

gaagg                                                                  65


<210>  67
<211>  68
<212>  DNA
<213>  Artificial Sequence

<220>
<223>  Description of Artificial Sequence: oligonucleotide


<220>
<221>  misc_feature
<222>  (32)..(33)
<223>  randomised

<220>
<221>  misc_feature
<222>  (35)..(36)
<223>  randomised

<220>
<221>  misc_feature
<222>  (38)..(39)
<223>  randomised

<220>
<221>  misc_feature
<222>  (41)..(42)
<223>  randomised

<220>
<221>  misc_feature
<222>  (44)..(45)
<223>  randomised

<220>
<221>  misc_feature
<222>  (47)..(48)
<223>  randomised

<400>  67
gccaccggtg acgtagatga attggccttc snnsnnsnns nnsnnsnngt ccgtgatgcc      60

taggaagg                                                               68


<210>  68
<211>  62
<212>  DNA
<213>  Artificial Sequence

<220>
<223>  Description of Artificial Sequence: oligonucleotide


<220>
<221>  misc_feature
<222>  (23)..(24)
<223>  randomised

<220>
<221> misc_feature
<222> (26)..(27)
<223> randomised

<220>
<221> misc_feature
<222> (29)..(30)
<223> randomised

<220>
<221> misc_feature
<222> (32)..(33)
<223> randomised

<220>
<221> misc_feature
<222> (35)..(36)
<223> randomised

<220>
<221> misc_feature
<222> (38)..(39)
<223> randomised

<220>
<221> misc_feature
<222> (41)..(42)
<223> randomised

<400> 68
gccaccggtg acgtagatga asnnsnnsnn snnsnnsnns nncgtgatgc ctaggaaggc 60

ag 62


<210> 69
<211> 40
<212> DNA
<213> Artificial Sequence

<220>
<223> Description of Artificial Sequence: oligonucleotide

<400> 69
ccagttgctg tatttcaggc tgccaccggt gacgtagatg 40


<210> 70
<211> 34
<212> DNA
<213> Artificial Sequence

<220>
<223> Description of Artificial Sequence: oligonucleotide

<400> 70
gcctgaaata cagcaactgg aagaaagacg aacc 34


<210> 71
<211> 68
<212> DNA
<213> Artificial Sequence

<220>
<223> Description of Artificial Sequence: oligonucleotide

<220>
<221> misc_feature
<222> (32)..(33)
<223> randomised

<220>
<221> misc_feature
<222> (35)..(36)
<223> randomised

<220>
<221> misc_feature
<222> (38)..(39)
<223> randomised

<220>
<221> misc_feature
<222> (41)..(42)
<223> randomised

<220>
<221> misc_feature
<222> (44)..(45)
<223> randomised

<400> 71
ctggaagaaa gacgaaccga atgaccatgg cnnsnnsnns nnsnnsgaag actgtgtcac 60

tatagtag 68


<210> 72
<211> 71
<212> DNA
<213> Artificial Sequence

<220>
<223> Description of Artificial Sequence: oligonucleotide


<220>
<221> misc_feature
<222> (32)..(33)
<223> randomised

<220>
<221> misc_feature
<222> (35)..(36)
<223> randomised

<220>
<221> misc_feature
<222> (38)..(39)
<223> randomised

<220>
<221> misc_feature
<222> (41)..(42)
<223> randomised

<220>
<221> misc_feature
<222> (44)..(45)
<223> randomised

<220>
<221> misc_feature

<222>  (47)..(48)
<223>  randomised

<400>  72
ctggaagaaa gacgaaccga atgaccatgg cnnsnnsnns nnsnnsnnsg aagactgtgt      60

cactatagta g                                                          71


<210>  73
<211>  59
<212>  DNA
<213>  Artificial Sequence

<220>
<223>  Description of Artificial Sequence: oligonucleotide


<220>
<221>  misc_feature
<222>  (23)..(24)
<223>  randomised

<220>
<221>  misc_feature
<222>  (26)..(27)
<223>  randomised

<220>
<221>  misc_feature
<222>  (29)..(30)
<223>  randomised

<220>
<221>  misc_feature
<222>  (32)..(33)
<223>  randomised

<220>
<221>  misc_feature
<222>  (35)..(36)
<223>  randomised

<400>  73
ctggaagaaa gacgaaccga atnnsnnsnn snnsnnsgaa gactgtgtca ctatagtag      59


<210>  74
<211>  17
<212>  DNA
<213>  Artificial Sequence

<220>
<223>  Description of Artificial Sequence: oligonucleotide

<400>  74
cggctgagcg gcccagc                                                    17


<210>  75
<211>  17
<212>  DNA
<213>  Artificial Sequence

<220>
<223>  Description of Artificial Sequence: oligonucleotide

<400>  75

gcactcctgc ggccgcg                                                    17


<210>  76
<211>  69
<212>  DNA
<213>  Artificial Sequence

<220>
<223>  Description of Artificial Sequence: oligonucleotide


<220>
<221>  misc_feature
<222>  (35)..(36)
<223>  randomised

<220>
<221>  misc_feature
<222>  (38)..(39)
<223>  randomised

<220>
<221>  misc_feature
<222>  (41)..(42)
<223>  randomised

<220>
<221>  misc_feature
<222>  (44)..(45)
<223>  randomised

<220>
<221>  misc_feature
<222>  (47)..(48)
<223>  randomised

<400>  76
ctcaccggtc ggatacgtga acttgccctc tgtsnnsnns nnsnnsnnat cagtcatgct      60

caggaaagc                                                              69


<210>  77
<211>  72
<212>  DNA
<213>  Artificial Sequence

<220>
<223>  Description of Artificial Sequence: oligonucleotide


<220>
<221>  misc_feature
<222>  (35)..(36)
<223>  randomised

<220>
<221>  misc_feature
<222>  (38)..(39)
<223>  randomised

<220>
<221>  misc_feature
<222>  (41)..(42)
<223>  randomised

<220>

<221>  misc_feature
<222>  (44)..(45)
<223>  randomised

<220>
<221>  misc_feature
<222>  (47)..(48)
<223>  randomised

<220>
<221>  misc_feature
<222>  (50)..(51)
<223>  randomised

<400>  77
ctcaccggtc ggatacgtga acttgccctc tgtsnnsnns nnsnnsnnsn natcagtcat      60

gctcaggaaa gc                                                          72


<210>  78
<211>  60
<212>  DNA
<213>  Artificial Sequence

<220>
<223>  Description of Artificial Sequence: oligonucleotide


<220>
<221>  misc_feature
<222>  (23)..(24)
<223>  randomised

<220>
<221>  misc_feature
<222>  (26)..(27)
<223>  randomised

<220>
<221>  misc_feature
<222>  (29)..(30)
<223>  randomised

<220>
<221>  misc_feature
<222>  (32)..(33)
<223>  randomised

<220>
<221>  misc_feature
<222>  (35)..(36)
<223>  randomised

<220>
<221>  misc_feature
<222>  (38)..(39)
<223>  randomised

<220>
<221>  misc_feature
<222>  (41)..(42)
<223>  randomised

<400>  78
ctcaccggtc ggatacgtga asnnsnnsnn snnsnnsnns nnagtcatgc tcaggaaagc      60

<210> 79
<211> 39
<212> DNA
<213> Artificial Sequence

<220>
<223> Description of Artificial Sequence: oligonucleotide

<400> 79
cagttggaat agaccaggga ctcaccggtc ggatacgtg 39

<210> 80
<211> 65
<212> DNA
<213> Artificial Sequence

<220>
<223> Description of Artificial Sequence: oligonucleotide

<220>
<221> misc_feature
<222> (30)..(31)
<223> randomised

<220>
<221> misc_feature
<222> (33)..(34)
<223> randomised

<220>
<221> misc_feature
<222> (36)..(37)
<223> randomised

<220>
<221> misc_feature
<222> (39)..(40)
<223> randomised

<220>
<221> misc_feature
<222> (42)..(43)
<223> randomised

<400> 80
gggccccagg ggagcccaac gatgatggcn nsnnsnnsnn snnsgaggac tgtgtggaga 60

tcttc 65

<210> 81
<211> 68
<212> DNA
<213> Artificial Sequence

<220>
<223> Description of Artificial Sequence: oligonucleotide

<220>
<221> misc_feature
<222> (30)..(31)
<223> randomised

<220>
<221> misc_feature

<222>   (33)..(34)
<223>   randomised

<220>
<221>   misc_feature
<222>   (36)..(37)
<223>   randomised

<220>
<221>   misc_feature
<222>   (39)..(40)
<223>   randomised

<220>
<221>   misc_feature
<222>   (42)..(43)
<223>   randomised

<220>
<221>   misc_feature
<222>   (45)..(46)
<223>   randomised

<400>   81
gggccccagg ggagcccaac gatgatggcn nsnnsnnsnn snnsnnsgag gactgtgtgg        60

agatcttc                                                                  68


<210>   82
<211>   68
<212>   DNA
<213>   Artificial Sequence

<220>
<223>   Description of Artificial Sequence: oligonucleotide


<220>
<221>   misc_feature
<222>   (30)..(31)
<223>   randomised

<220>
<221>   misc_feature
<222>   (33)..(34)
<223>   randomised

<220>
<221>   misc_feature
<222>   (36)..(37)
<223>   randomised

<220>
<221>   misc_feature
<222>   (39)..(40)
<223>   randomised

<220>
<221>   misc_feature
<222>   (42)..(43)
<223>   randomised

<220>
<221>   misc_feature
<222>   (45)..(46)
<223>   randomised

<400>  82
gggccccagg ggagcccaac gatgatggcn nsnnsnnsnn snnsnnsgag gactgtgtgg      60

agatcttc                                                                68


<210>  83
<211>  56
<212>  DNA
<213>  Artificial Sequence

<220>
<223>  Description of Artificial Sequence: oligonucleotide


<220>
<221>  misc_feature
<222>  (21)..(22)
<223>  randomised

<220>
<221>  misc_feature
<222>  (24)..(25)
<223>  randomised

<220>
<221>  misc_feature
<222>  (27)..(28)
<223>  randomised

<220>
<221>  misc_feature
<222>  (30)..(31)
<223>  randomised

<220>
<221>  misc_feature
<222>  (33)..(34)
<223>  randomised

<400>  83
gggccccagg ggagcccaac nnsnnsnnsn nsnnsgagga ctgtgtggag atcttc          56


<210>  84
<211>  77
<212>  DNA
<213>  Artificial Sequence

<220>
<223>  Description of Artificial Sequence: oligonucleotide


<220>
<221>  misc_feature
<222>  (21)..(22)
<223>  randomised

<220>
<221>  misc_feature
<222>  (24)..(25)
<223>  randomised

<220>
<221>  misc_feature
<222>  (27)..(28)
<223>  randomised

<220>
<221> misc_feature
<222> (30)..(31)
<223> randomised

<220>
<221> misc_feature
<222> (33)..(34)
<223> randomised

<220>
<221> misc_feature
<222> (36)..(37)
<223> randomised

<400> 84
gcatcgccta caagaactgg nnsnnsnnsn nsnnsnnsca acccgatggc ggcaagaccg 60

agaactgcgc ggtcctg 77


<210> 85
<211> 83
<212> DNA
<213> Artificial Sequence

<220>
<223> Description of Artificial Sequence: oligonucleotide


<220>
<221> misc_feature
<222> (24)..(25)
<223> randomised

<220>
<221> misc_feature
<222> (27)..(28)
<223> randomised

<220>
<221> misc_feature
<222> (30)..(31)
<223> randomised

<220>
<221> misc_feature
<222> (33)..(34)
<223> randomised

<220>
<221> misc_feature
<222> (36)..(37)
<223> randomised

<220>
<221> misc_feature
<222> (39)..(40)
<223> randomised

<400> 85
gcatcgccta caagaactgg gagnnsnnsn nsnnsnnsnn sgcgcaaccc gatggcggca 60

agaccgagaa ctgcgcggtc ctg 83


<210> 86
<211> 80

<212>  DNA
<213>  Artificial Sequence

<220>
<223>  Description of Artificial Sequence: oligonucleotide


<220>
<221>  misc_feature
<222>  (24)..(25)
<223>  randomised

<220>
<221>  misc_feature
<222>  (27)..(28)
<223>  randomised

<220>
<221>  misc_feature
<222>  (30)..(31)
<223>  randomised

<220>
<221>  misc_feature
<222>  (33)..(34)
<223>  randomised

<220>
<221>  misc_feature
<222>  (36)..(37)
<223>  randomised

<400>  86
gcatcgccta caagaactgg gagnnsnnsn nsnnsnnsgc gcaacccgat ggcggcaaga      60

ccgagaactg cgcggtcctg                                                  80


<210>  87
<211>  75
<212>  DNA
<213>  Artificial Sequence

<220>
<223>  Description of Artificial Sequence: oligonucleotide


<220>
<221>  misc_feature
<222>  (38)..(39)
<223>  randomised

<220>
<221>  misc_feature
<222>  (41)..(42)
<223>  randomised

<220>
<221>  misc_feature
<222>  (44)..(45)
<223>  randomised

<220>
<221>  misc_feature
<222>  (47)..(48)
<223>  randomised

<220>

<221>  misc_feature
<222>  (50)..(51)
<223>  randomised

<220>
<221>  misc_feature
<222>  (53)..(54)
<223>  randomised

<220>
<221>  misc_feature
<222>  (56)..(57)
<223>  randomised

<400>  87
gtagggcaat tgatcgctgc agcgcttgtc gaaccasnns nnsnnsnnsn nsnnsnncag      60

gaccgcgcag ttctc                                                       75


<210>  88
<211>  84
<212>  DNA
<213>  Artificial Sequence

<220>
<223>  Description of Artificial Sequence: oligonucleotide


<220>
<221>  misc_feature
<222>  (44)..(45)
<223>  randomised

<220>
<221>  misc_feature
<222>  (47)..(48)
<223>  randomised

<220>
<221>  misc_feature
<222>  (50)..(51)
<223>  randomised

<220>
<221>  misc_feature
<222>  (53)..(54)
<223>  randomised

<220>
<221>  misc_feature
<222>  (56)..(57)
<223>  randomised

<220>
<221>  misc_feature
<222>  (59)..(60)
<223>  randomised

<400>  88
gtagggcaat tgatcgctgc agcgcttgtc gaaccacttg ccsnnsnnsn nsnnsnnsnn      60

gcctgacagg accgcgcagt tctc                                             84


<210>  89
<211>  81
<212>  DNA

<213>  Artificial Sequence

<220>
<223>  Description of Artificial Sequence: oligonucleotide


<220>
<221>  misc_feature
<222>  (44)..(45)
<223>  randomised

<220>
<221>  misc_feature
<222>  (47)..(48)
<223>  randomised

<220>
<221>  misc_feature
<222>  (50)..(51)
<223>  randomised

<220>
<221>  misc_feature
<222>  (53)..(54)
<223>  randomised

<220>
<221>  misc_feature
<222>  (56)..(57)
<223>  randomised

<400>  89
gtagggcaat tgatcgctgc agcgcttgtc gaaccacttg ccsnnsnnsn nsnnsnngcc      60

tgacaggacc gcgcagttct c                                                81


<210>  90
<211>  20
<212>  DNA
<213>  Artificial Sequence

<220>
<223>  Description of Artificial Sequence: oligonucleotide

<400>  90
gtagggcaat tgatcgctgc                                                  20


<210>  91
<211>  34
<212>  DNA
<213>  Artificial Sequence

<220>
<223>  Description of Artificial Sequence: oligonucleotide

<400>  91
catgaccggt acccgcatcg cctacaagaa ctgg                                  34

## Description

### FIELD OF THE INVENTION

This invention describes a system which relates to the generation of randomised libraries of ligand-binding protein units derived from proteins containing the so-called C-type lectin like domain (CTLD) of which the carbohydrate recognition domain (CRD) of C-type lectins represents one example of a family of this protein domain.

### BACKGROUND OF THE INVENTION

The C-type lectin-like domain (CTLD) is a protein domain family which has been identified in a number of proteins isolated from many animal species (reviewed in Drickamer and Taylor (1993) and Drickamer (1999)). Initially, the CTLD domain was identified as a domain common to the so-called C-type lectins (calcium-dependent carbohydrate binding proteins) and named "Carbohydrate Recognition Domain" ("CRD"). More recently, it has become evident that this domain is shared among many eukaryotic proteins, of which several do not bind sugar moieties, and hence, the canonical domain has been named as CTLD.

CTLDs have been reported to bind a wide diversity of compounds, including carbohydrates, lipids, proteins, and even ice [Aspberg et al. (1997), Bettler et al. (1992), Ewart et al. (1998), Graversen et al. (1998), Mizumo et al. (1997), Sano et al. (1998), and Tormo et al. (1999)]. Only one copy of the CTLD is present in some proteins, whereas other proteins contain from two to multiple copies of the domain. In the physiologically functional unit multiplicity in the number of CTLDs is often achieved by assembling single copy protein protomers into larger structures.

The CTLD consists of approximately 120 amino acid residues and, characteristically, contains two or three intra-chain disulfide bridges. Although the similarity at the amino acid sequence level between CTLDs from different proteins is relatively low, the 3D-structures of a number of CTLDs have been found to be highly conserved, with the structural variability essentially confined to a so-called loop-region, often defined by up to five loops. Several CTLDs contain either one or two binding sites for calcium and most of the side chains which interact with calcium are located in the loop-region.

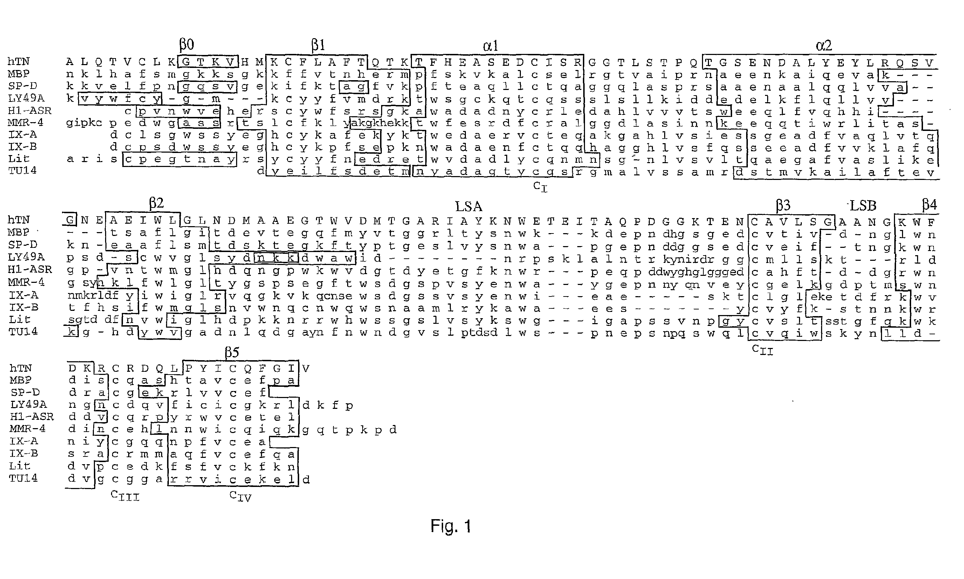

On the basis of CTLDs for which 3D structural information is available, it has been inferred that the canonical CTLD is structurally characterised by seven main secondary-structure elements (i.e. five β-strands and two α-helices) sequentially appearing in the order β1; α1; α2; β2; β3; β4; and β5 (Fig. 1, and references given therein). In all CTLDs, for which 3D structures have been determined, the β-strands are arranged in two anti-parallel β-sheets, one composed of β1 and β5, the other composed of β2, β3 and β4. An additional β-strand, β0, often precedes β1 in the sequence and, where present, forms an additional strand integrating with the β1, β5-sheet. Further, two disulfide bridges, one connecting α1 and β5 (C_{I}-C_{IV}, Fig. 1) and one connecting β3 and the polypeptide segment connecting β4 and β5 (C_{II}-C_{III}, Fig. 1) are invariantly found in all CTLDs characterised so far. In the CTLD 3D-structure, these conserved secondary structure elements form a compact scaffold for a number of loops, which in the present context collectively are referred to as the "loop-region", protruding out from the core. These loops are in the primary structure of the CTLDs organised in two segments, loop segment A, LSA, and loop segment B, LSB. LSA represents the long polypeptide segment connecting β2 and β3 which often lacks regular secondary structure and contains up to four loops. LSB represents the polypeptide segment connecting the β-strands β3 and β4. Residues in LSA, together with single residues in β4, have been shown to specify the Ca²⁺- and ligand-binding sites of several CTLDs, including that of tetranectin. E.g. mutagenesis studies, involving substitution of single or a few residues, have shown, that changes in binding specificity, Ca²⁺-sensitivity and/or affinity can be accommodated by CTLD domains [Weis and Drickamer (1996), Chiba et al. (1999), Graversen et al. (2000)].

As noted above, overall sequence similarities between CTLDs are often limited, as assessed e.g. by aligning a prospective CTLD sequence with the group of structure-characterized CTLDs presented in Fig. 1, using sequence alignment procedures and analysis tools in common use in the field of protein science. In such an alignment, typically 22-30% of the residues of the prospective CTLD will be identical with the corresponding residue in at least one of the structure-characterized CTLDs. The sequence alignment shown in Fig. 1 was strictly elucidated from actual 3D structure data, so the fact that the polypeptide segments of corresponding structural elements of the framework also exhibit strong sequence similarities provide a set of direct sequence-structure signatures, which can readily be inferred from the sequence alignment.

The implication is that also CTLDs, for which precise 3D structural information is not yet available, can nonetheless be used as frameworks in the construction of new classes of CTLD libraries. The specific additional steps involved in preparing starting materials for the construction of such a new class of CTLD library on the basis of a CTLD, for which no precise 3D structure is available, would be the following: (1) Alignment of the sequence of the new CTLD with the sequence shown in Fig. 1; and (2) Assignment of approximate locations of framework structural elements as guided by the sequence alignment, observing any requirement for minor adjustment of the alignment to ensure precise alignment of the four canonical cysteine residues involved in the formation of the two conserved disulfide bridges (C_{I}-C_{IV} and C_{II}-C_{III}, in Fig. 1) . The main objective of these steps would be to identify the sequence location of the loop-region of the new CTLD, as flanked in the sequence by segments corresponding to the β2-, β3- and β4-strands. To provide further guidance in this the results of an analysis of the sequences of 29 bona fide CTLDs are given in Table 1 below in the form of typical tetrapeptide sequences, and their consensus sequences, found as parts of CTLD β2- and β3-strands, and the precise location of the β4-strand by position and sequence characteristics as elucidated.

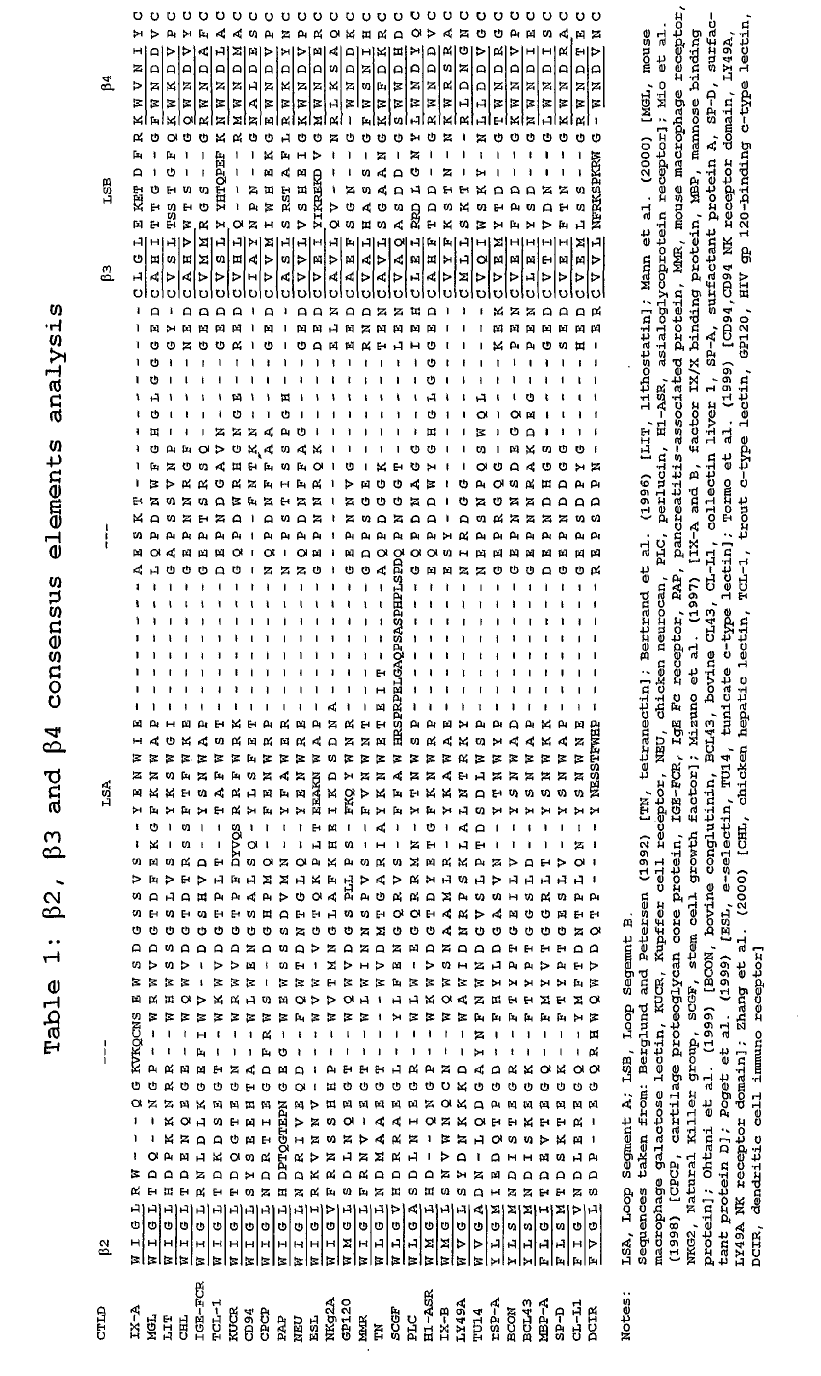

Of the 29 β2-strands,
14 were found to conform to the consensus sequence WIGX (of which 12 were WIGL sequences, 1 was a WIGI sequence and 1 was a WIGV sequence);
3 were found to conform to the consensus sequence WLGX (of which 1 was a WLGL sequence, 1 was a WLGV sequence and 1 was a WLGA sequence);
3 were found to be WMGL sequences;
3 were found to conform to the consensus sequence YLXM (of which 2 were YLSM sequences and 1 was an YLGM sequence);
2 were found to conform to the consensus sequence WVGX (of which 1 was a WVGL sequence and 1 was a WVGA sequence); and
the sequences of the remaining 4 β2-strands in the collection were FLGI, FVGL, FIGV and FLSM sequences, respectively.

Therefore, it is concluded that the four-residue β2 consensus sequence ("β2cseq") may be specified as follows:
Residue 1: An aromatic residue, most preferably Trp, less preferably Phe and least preferably Tyr.
Residue 2: An aliphatic or non-polar residue, most preferably Ile, less preferably Leu or Met and least preferably Val.
Residue 3: An aliphatic or hydrophilic residue, most preferably Gly and least preferably Ser.
Residue 4: An aliphatic or non-polar residue, most preferably Leu and less preferably Met, Val or Ile.

Accordingly the β2 consensus sequence may be summarized as follows:
β2cseq: (W,Y,F)-(I,L,V,M)-(G,S)-(L,M,V,I),
where the underlined residue denotes the most commonly found residue at that sequence position.

All 29 β3-strands analysed are initiated with the Cys_{II} residue canonical for all known CTLD sequences, and of the 29 β3-strands,
5 were found to conform to the consensus sequence CVXI (of which 3 were CVEI sequences, 1 was a CVTI sequence and 1 was a CVQI sequence);
4 were found to conform to the consensus sequence CVXM (of which 2 were CVEM sequences, 1 was a CVVM sequence and 1 was a CVMM sequence);
6 were found to conform to the consensus sequence CVXL (of which 2 were CVVL sequences, 2 were a CVSL sequence, 1 was a CVHL sequence and 1 was CVAL sequence);
3 were found to conform to the consensus sequence CAXL (of which 2 were CAVL sequences and 1 was a CASL sequence);
2 were found to conform to the consensus sequence CAXF (of which 1 was 1 CAHF sequence and 1 was a CAEF sequence);
2 were found to conform to the consensus sequence CLXL (of which 1 was a CLEL sequence and 1 was a CLGL sequence); and
the sequences of the remaining 7 β3-strands in the collection were CVYF, CVAQ, CAHV, CAHI, CLEI, CIAY, and CMLL sequences, respectively.

Therefore, it is concluded that the four-residue β3 consensus sequence ("β3cseq") may be specified as follows:
Residue 1: Cys, being the canonical Cys_{II} residue of CTLDs
Residue 2: An aliphatic or non-polar residue, most preferably Val, less preferably Ala or Leu and least preferably Ile or Met
Residue 3: Most commonly an aliphatic or charged residue, which most preferably is Glu
Residue 4: Most commonly an aliphatic, non-polar, or aromatic residue, most preferably Leu or Ile, less preferably Met or Phe and least preferably Tyr or Val.

Accordingly the β3 consensus sequence may be summarized as follows:
β3cseq: (C)-(V,A,L,I,M)-(E,X)-(L,I,M,F,Y,V),
where the underlined residue denotes the most commonly found residue at that sequence position.

It is observed from the known 3D-structures of CTLDs (Fig. 1), that the β4-strands most often are comprised by five residues located in the primary structure at positions -6 to -2 relative to the canonical Cys_{III} residue of all known CTLDs, and less often are comprised by four residues located at positions -5 to -2 relative to the canonical Cys_{III} residue of all known CTLDs. The residue located at position -3, relative to Cys_{III}, is involved in co-ordination of the site 2 calcium ion in CTLDs housing this site, and this notion is reflected in the observation, that of the 29 CTLD sequences analysed in Table 1, 27 have an Asp-residue or an Asn-residue at this position, whereas 2 CTLDs have a Ser at this position. From the known CTLD 3D-structures it is also noted, that the residue located at position -5, relative to the Cys_{III} residue, is involved in the formation of the hydrophobic core of the CTLD scaffold. This notion is reflected in the observation, that of the 29 CTLD sequences analysed 25 have a Trp-residue, 3 have a Leu-residue, and 1 an Ala-residue at this position. 18 of the 29 CTLD sequences analysed have an Asn-residue at position -4. Further, 19 of the 29 β4-strand segments are preceded by a Gly residue.

Of the 29 central three residue motifs located at positions -5, -4 and -3 relative to the canonical Cys_{III} residue in the β4-strand:
22 were of the sequence WXD (18 were WND, 2 were WKD, 1 was WFD and 1 was WWD),
2 were of the sequence WXN (1 was WVN and 1 was WSN),
and the remaining 5 motifs (WRS, LDD, LDN, LKS and ALD) were each represented once in the analysis.

It has now been found that each member of the family of CTLD domains represents an attractive opportunity for the construction of new protein libraries from which members with affinity for new ligand targets can be identified and isolated using screening or selection methods. Such libraries may be constructed by combining a CTLD framework structure in which the CTLD's loop-region is partially or completely replaced with one or more randomised polypeptide segments.

One such system, where the protein used as scaffold is tetranectin or the CTLD domain of tetranectin, is envisaged as a system of particular interest, not least because the stability of the trimeric complex of tetranectin protomers is very high (International Patent Application Publication No. WO 98/56906 A2).

Tetranectin is a trimeric glycoprotein [Holtet et al. (1997), Nielsen et al. (1997)], which has been isolated from human plasma and found to be present in the extracellular matrix in certain tissues. Tetranectin is known to bind calcium, complex polysaccharides, plasminogen, fibrinogen/fibrin, and apolipoprotein (a). The interaction with plasminogen and apolipoprotein (a) is mediated by the so-called kringle 4 protein domain therein. This interaction is known to be sensitive to calcium and to derivatives of the amino acid lysine [Graversen et al. (1998)].

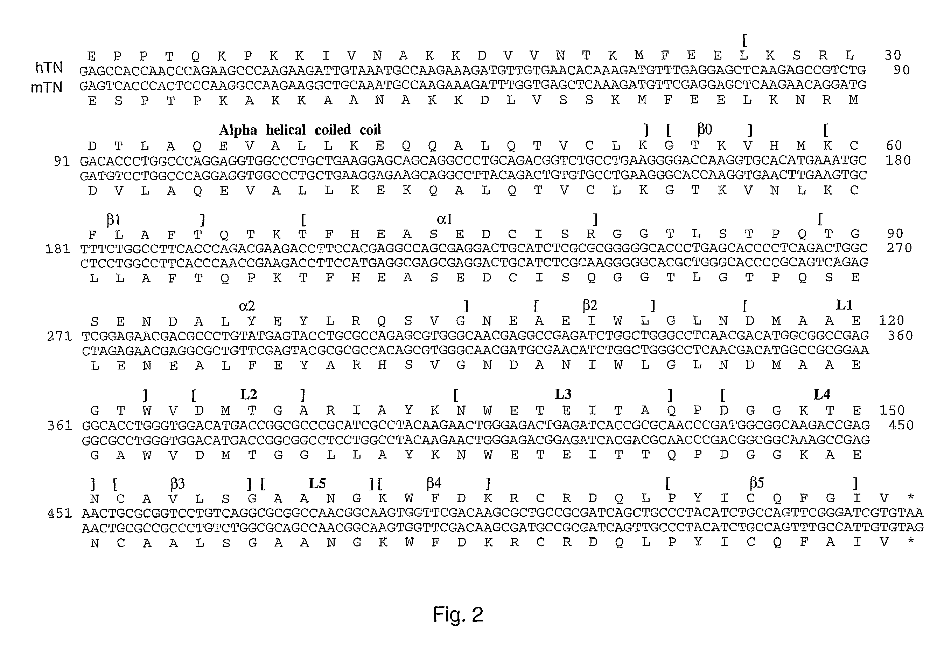

A human tetranectin gene has been characterised, and both human and murine tetranectin cDNA clones have been isolated. Both the human and the murine mature protein comprise 181 amino acid residues (Fig. 2). The 3D-structures of full length recombinant human tetranectin and of the isolated tetranectin CTLD have been determined independently in two separate studies [Nielsen et al. (1997) and Kastrup et al. (1998)]. Tetranectin is a two- or possibly three-domain protein, i.e. the main part of the polypeptide chain comprises the CTLD (amino acid residues Gly53 to Val181), whereas the region Leu26 to Lys52 encodes an alpha-helix governing trimerisation of the protein via the formation of a homotrimeric parallel coiled coil. The polypeptide segment Glu1 to Glu25 contains the binding site for complex polysaccharides (Lys6 to Lys15) [Lorentsen et al. (2000)] and appears to contribute to stabilisation of the trimeric structure [Holtet et al. (1997)]. The two amino acid residues Lys148 and Glu150, localised in loop 4, and Asp165 (localised in β4) have been shown to be of critical importance for plasminogen kringle 4 binding, whereas the residues Ile140 (in loop 3) and Lys166 and Arg167 (in β4) have been shown to be of some importance [Graversen et al. (1998)]. Substitution of Thr149 (in loop 4) with an aromatic residue has been shown to significantly increase affinity of tetranectin to kringle 4 and to increase affinity for plasminogen kringle 2 to a level comparable to the affinity of wild type tetranectin for kringle 4 [Graversen et al. (2000)].

### OBJECT OF THE INVENTION

The object of the invention is to provide a new practicable method for the generation of useful protein products endowed with binding sites able to bind substance of interest with high affinity and specificity.

The invention describes one way in which such new and useful protein products may advantageously be obtained by applying standard combinatorial protein chemistry methods, commonly used in the recombinant antibody field, to generate randomised combinatorial libraries of protein modules, in which each member contains an essentially common core structure similar to that of a CTLD.

The variation of binding site configuration among naturally occurring CTLDs shows that their common core structure can accommodate many essentially different configurations of the ligand binding site. CTLDs are therefore particularly well suited to serve as a basis for constructing such new and useful protein products with desired binding properties.

In terms of practical application, the new artificial CTLD protein products can be employed in applications in which antibody products are presently used as key reagents in technical biochemical assay systems or medical in vitro or *in vivo* diagnostic assay systems or as active components in therapeutic compositions.

In terms of use as components of in vitro assay systems, the artificial CTLD protein products are preferable to antibody derivatives as each binding site in the new protein product is harboured in a single structurally autonomous protein domain. CTLD domains are resistant to proteolysis, and neither stability nor access to the ligand-binding site is compromised by the attachment of other protein domains to the N- or C-terminus of the CTLD. Accordingly, the CTLD binding module may readily be utilized as a building block for the construction of modular molecular assemblies, e.g. harbouring multiple CLTDs of identical or nonidentical specificity in addition to appropriate reporter modules like peroxidases, phosphatases or any other signal-mediating moiety.

In terms of *in vivo* use as essential component of compositions to be used for *in vivo* diagnostic or therapeutic purposes, artificial CTLD protein products constructed on the basis of human CTLDs are virtually identical to the corresponding natural CTLD protein already present in the body, and are therefore expected to elicit minimal immunological response in the patient. Single CTLDs are about half the mass of the smallest functional antibody derivative, the single-chain Fv derivative, and this small size may in some applications be advantageous as it may provide better tissue penetration and distribution, as well as a shorter half-life in circulation. Multivalent formats of CTLD proteins, e.g. corresponding to the complete tetranectin trimer or the further multimerized collectins, like e.g. mannose binding protein, provide increased binding capacity and avidity and longer circulation half-life.

One particular advantage of the preferred embodiment of the invention, arises from the fact that mammalian tetranectins, as exemplified by murine and human tetranectin, are of essentially identical structure. This conservation among species is of great practical importance as it allows straightforward swapping of polypeptide segments defining ligand-binding specificity between e.g. murine and human tetranectin derivatives. The option of facile swapping of species genetic background between tetranectin derivatives is in marked contrast to the well-known complications of effecting the "humanisation" of murine antibody derivatives.

Further advantages of the invention are:

The availability of a general and simple procedure for reliable conversion of an initially selected protein derivative into a final protein product, which without further reformatting may be produced in bacteria (e.g. *Escherichia coli*) both in small and in large scale (International Patent Application Publication No. WO 94/18227 A2).

The option of including several identical or non-identical binding sites in the same functional protein unit by simple and general means, thereby enabling the exploitation even of weak affinities by means of avidity in the interaction, or the construction of bi- or heterofunctional molecular assemblies (International Patent Application Publication No. WO 98/56906 A2).

The possibility of modulating binding by addition or removal of divalent metal ions (e.g. calcium ions) in combinational libraries with one or more preserved metal binding site(s) in the CTLDs.

### SUMMARY OF THE INVENTION

The present invention provides a great number of novel and useful proteins each being a protein having the scaffold structure of C-type lectin-like domains (CTLD), said protein comprising a variant of a model CTLD wherein the α-helices and β-strands and connecting segments are conserved to such a degree that the scaffold structure of the CTLD is substantially maintained, while the loop region is altered by amino acid substitution, deletion, insertion or any combination thereof, with the proviso that said protein is not any of the known CTLD loop derivatives of C-type lectin-like proteins or C-type lectins listed in the following Table 2.

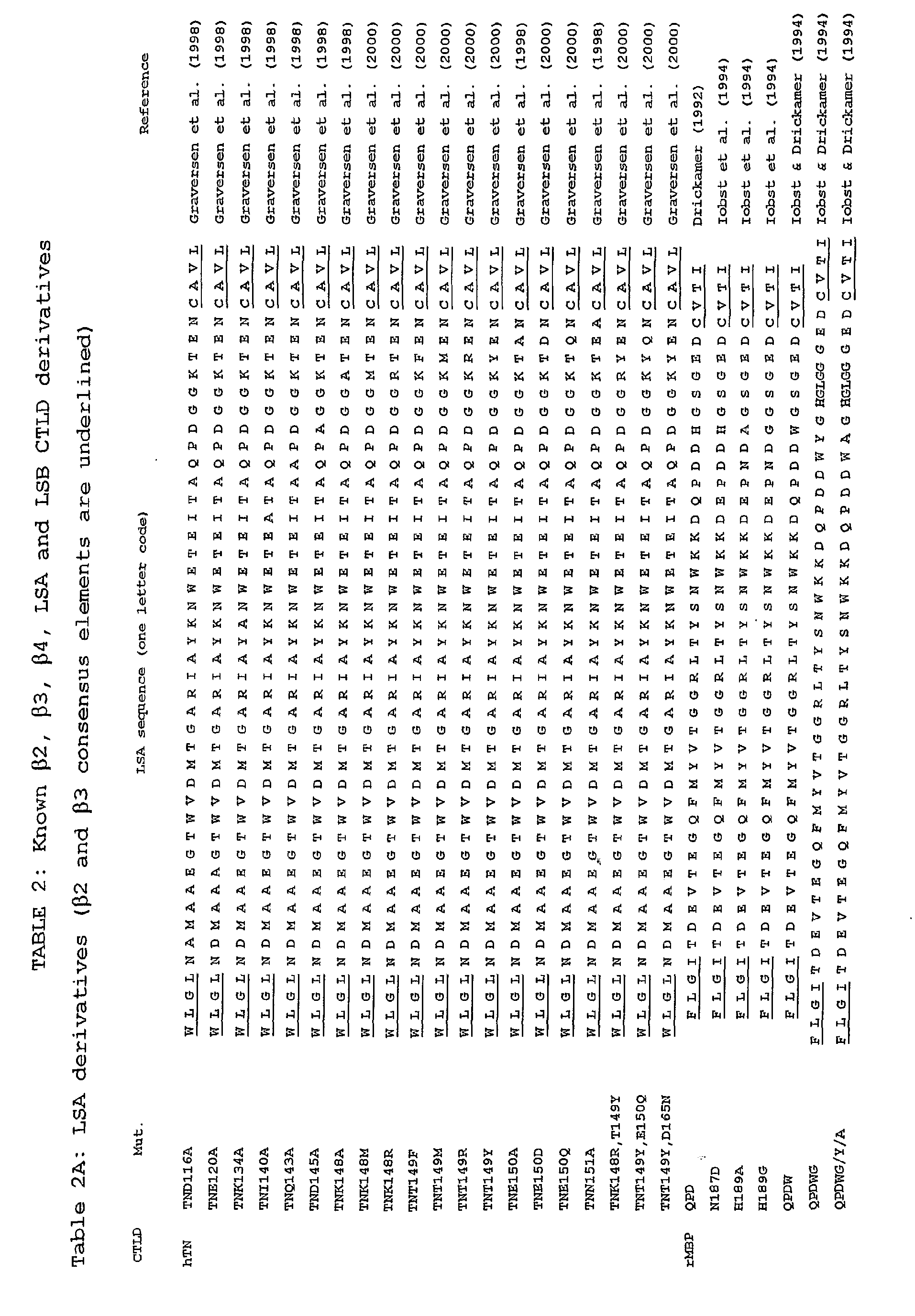

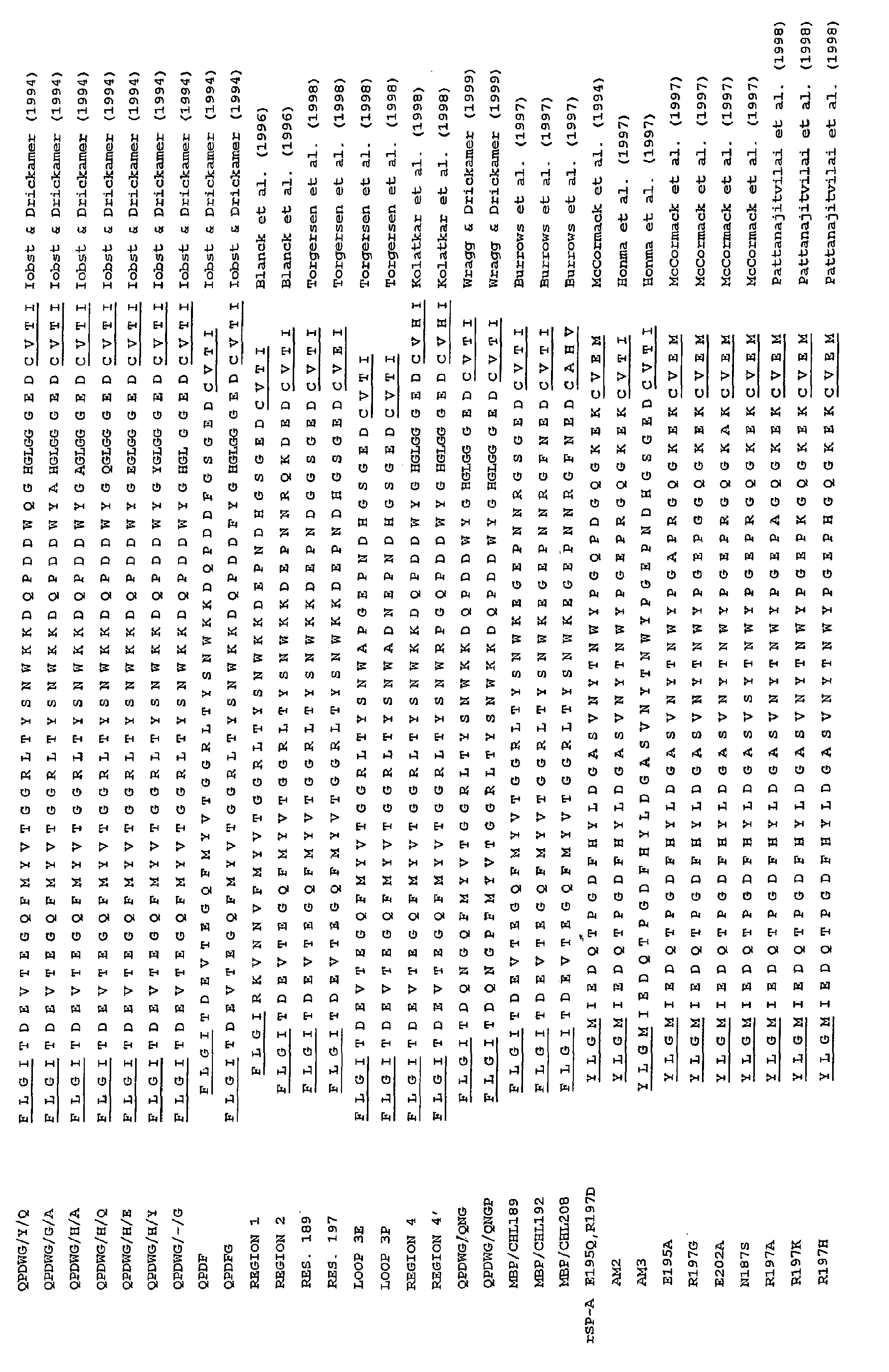

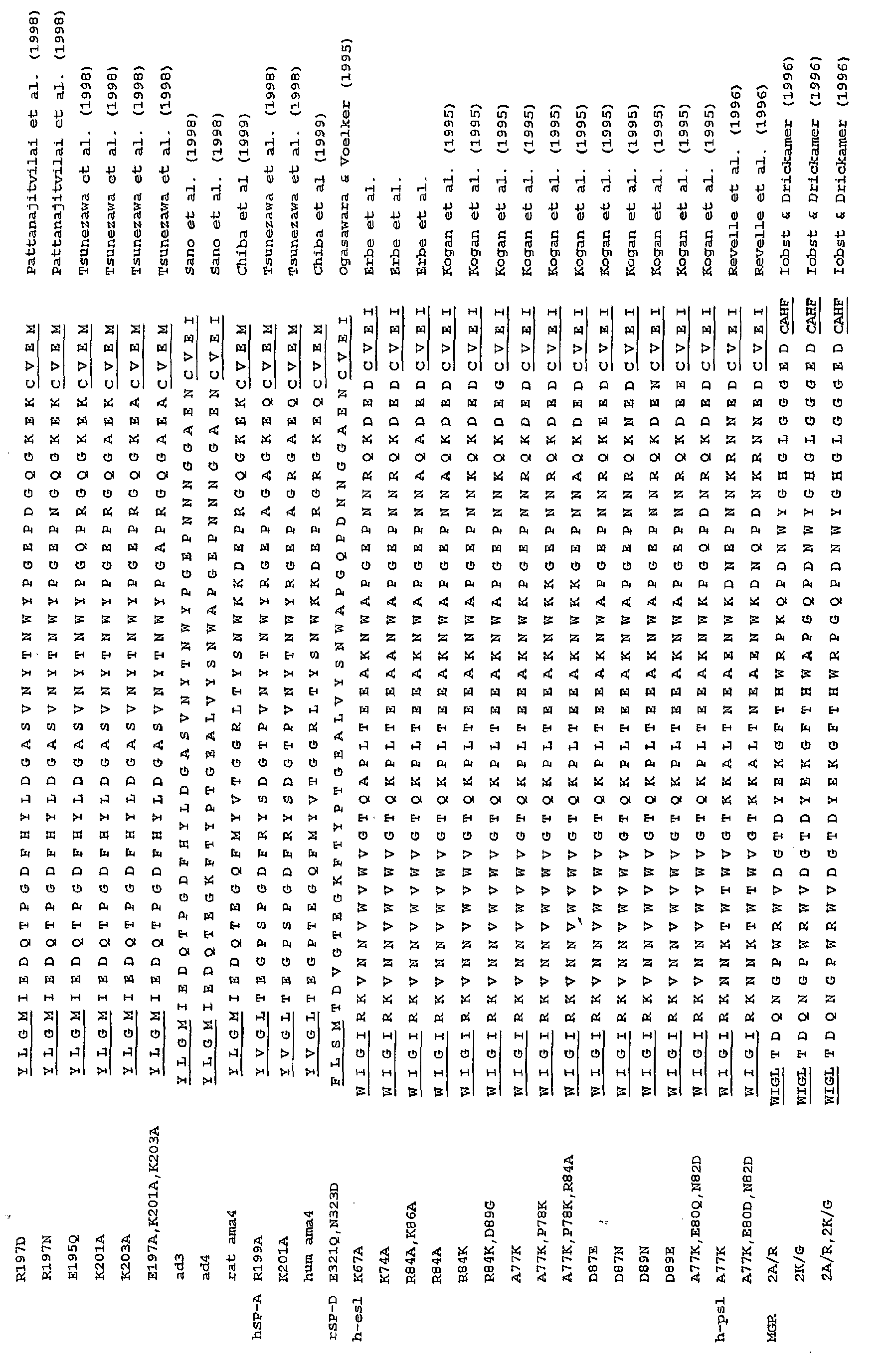

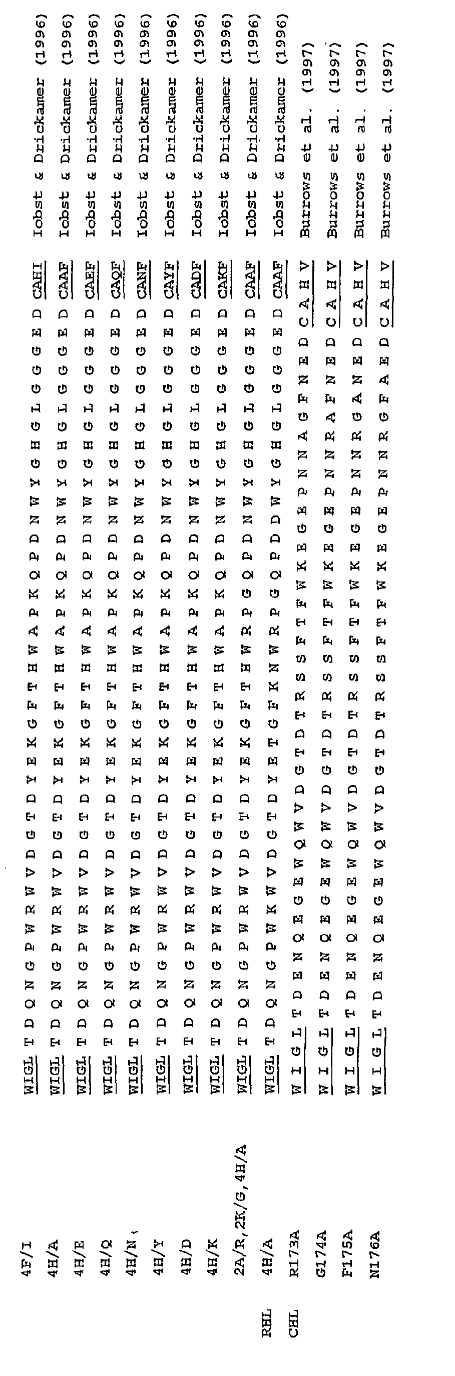

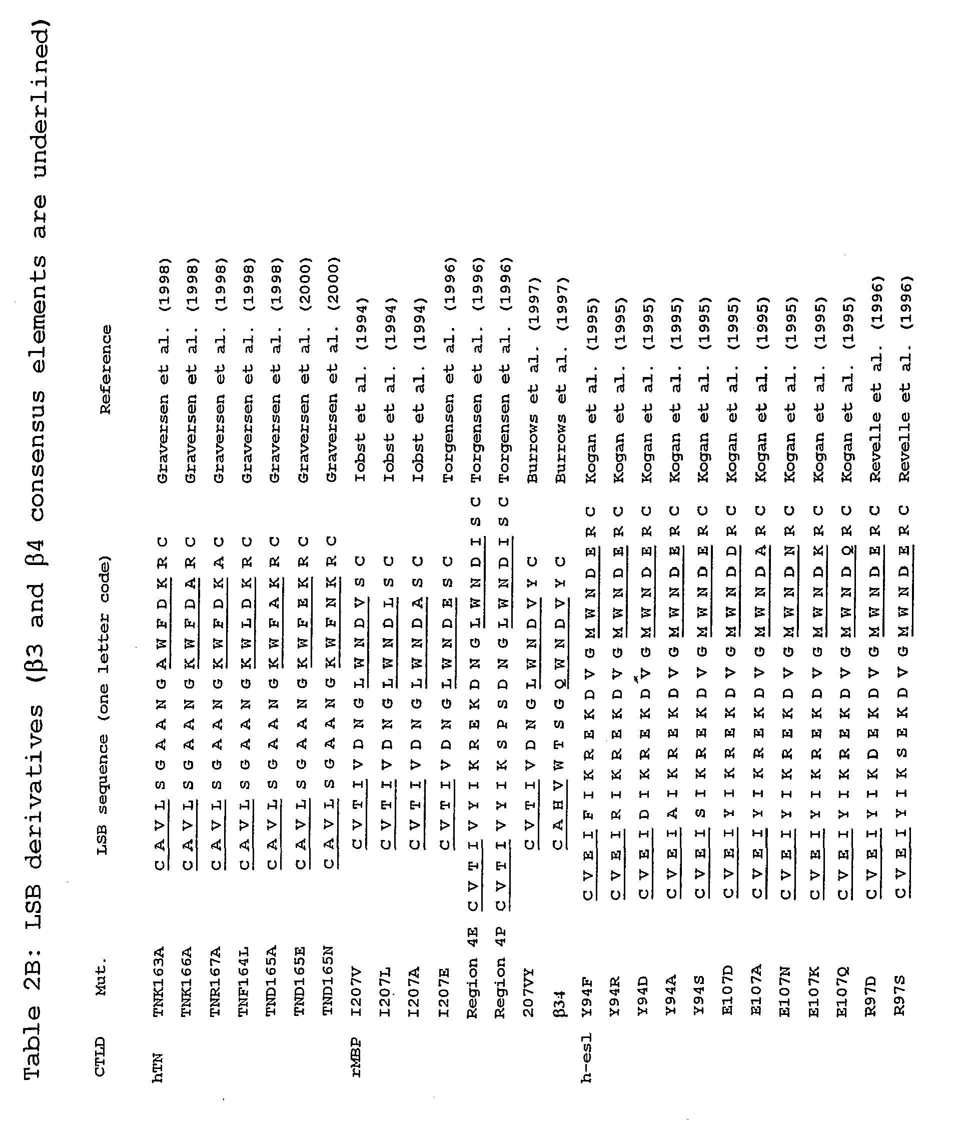

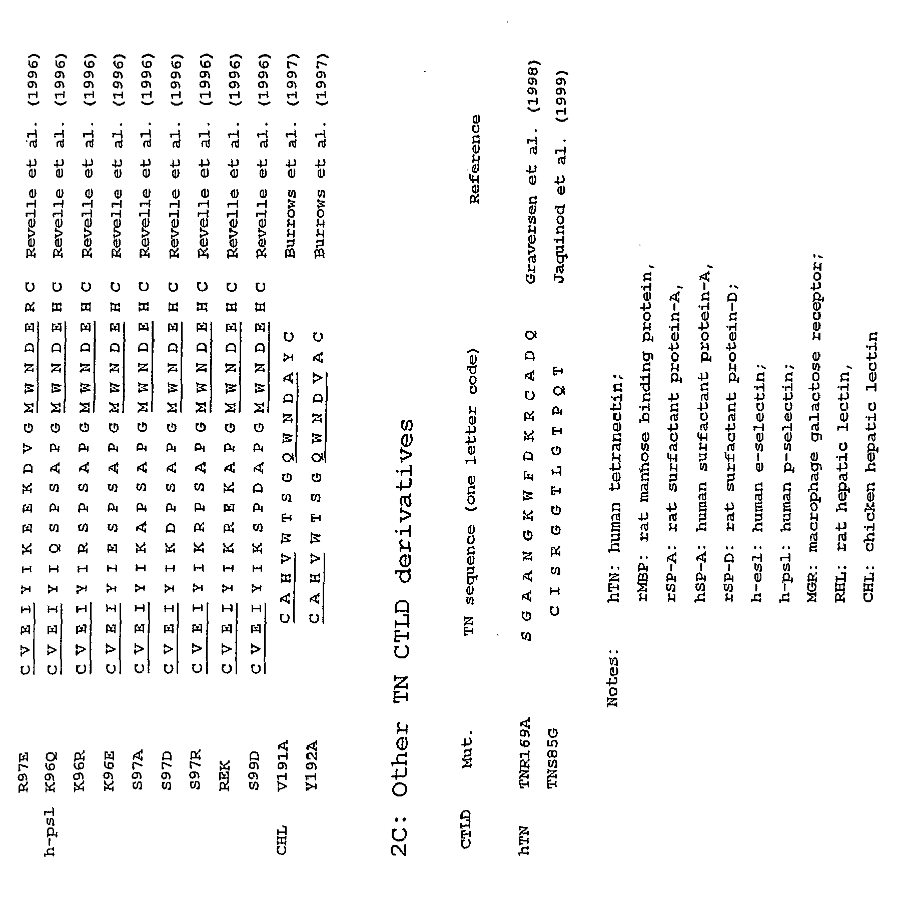

Normally the model CTLD is defined by having a 3D structure that conforms to the secondary-structure arrangement illustrated in Fig. 1 characterized by the following main secondary structure elements:
five β-strands and two α-helices sequentially appearing in the order β1, α1*,* α2, β2, β3, β4, and β5, the β-strands being arranged in two anti-parallel β-sheets, one composed of β1 and β5, the other composed of β2, β3 and β4,
at least two disulfide bridges, one connecting α1 and β5 and one connecting β3 and the polypeptide segment connecting β4 and β5,
a loop region consisting of two polypeptide segments, loop segment A (LSA) connecting β2 and β3 and comprising typically 15-70 or, less typically, 5-14 amino acid residues, and loop segment B (LSB) connecting β3 and β4 and comprising typically 5-12 or less typically, 2-4 amino acid residues.

However, also a CTLD, for which no precise 3D structure is available, can be used as a model CTLD, such CTLD being defined by showing sequence similarity to a previously recognised member of the CTLD family as expressed by an amino acid sequence identity of at least 22 %, preferably at least 25 % and more preferably at least 30 %, and by containing the cysteine residues necessary for establishing the conserved two-disulfide bridge topology (i.e. Cys_{I}, Cys_{II}, Cys_{III} and Cys_{IV}). The loop region, consisting of the loop segments LSA and LSB, and its flanking β-strand structural elements can then be identified by inspection of the sequence alignment with the collection of CTLDs shown in Fig. 1, which provides identification of the sequence locations of the β2- and β3-strands with the further corroboration provided by comparison of these sequences with the four-residue consensus sequences, β2cseq and β3cseq, and the β4 strand segment located typically at positions -6 to -2 and less typically at positions -5 to -2 relative to the conserved Cys_{III} residue and with the characteristic residues at positions -5 and -3 as elucidated from Table 1 and deducted above under BACKGROUND OF THE INVENTION.

The same considerations apply for determining whether in a model CTLD the α-helices and β-strands and connecting segments are conserved to such a degree that the scaffold structure of the CTLD is substantially maintained.

It may be desirable that up to 10, preferably up to 4, and more preferably 1 or 2, amino acid residues are substituted, deleted or inserted in the α-helices and/or β-strands and/or connecting segments of the model CTLD. In particular, changes of up to 4 residues may be made in the β-strands of the model CTLD as a consequence of the introduction of recognition sites for one or more restriction endonucleases in the nucleotide sequence encoding the CTLD to facilitate the excision of part or all of the loop region and the insertion of an altered amino acid sequence instead while the scaffold structure of the CTLD is substantially maintained.

Of particular interest are proteins wherein the model CTLD is that of a tetranectin. Well known tetranectins the CTLDs of which can be used as model CTLDs are human tetranectin and murine tetranectin. The proteins according to the invention thus comprise variants of such model CTLDs.

The proteins according to the invention may comprise N-terminal and/or C-terminal extensions of the CTLD variant, and such extensions may for example contain effector, enzyme, further binding and/or multimerising functions. In particular, said extension may be the non-CTLD-portions of a native C-type lectin-like protein or C-type lectin or a "soluble" variant thereof lacking a functional transmembrane domain.

The proteins according to the invention may also be multimers of a moiety comprising the CTLD variant, e.g. derivatives of the native tetranectin trimer.

In a preferred aspect the present invention provides a combinatorial library of proteins having the scaffold structure of C-type lectin-like domains (CTLD), said proteins comprising variants of a model CTLD wherein the α-helices and β-strands are conserved to such a degree that the scaffold structure of the CTLD is substantially maintained, while the loop region or parts of the loop region of the CTLD is randomised with respect to amino acid sequence and/or number of amino acid residues.

The proteins making up such a library comprise variants of model CTLDs defined as for the above proteins according to the invention, and the variants may include the changes stated for those proteins.

In particular, the combinatorial library according to the invention may consist of proteins wherein the model CTLD is that of a tetranectin, e.g. that of human tetranectin or that of murine tetranectin.

The combinatorial library according to the invention may consist of proteins comprising N-terminal and/or C-terminal extensions of the CTLD variant, and such extensions may for example contain effector, enzyme, further binding and/or multimerising functions. In particular, said extensions may be the non-CTLD-portions of a native C-type lectin-like protein or C-type lectin or a "soluble" variant thereof lacking a functional transmembrane domain.

The combinatorial library according to the invention may also consist of proteins that are multimers of a moiety comprising the CTLD variant, e.g. derivatives of the native tetranectin trimer.

The present invention also provides derivatives of a native tetranectin wherein up to 10, preferably up to 4, and more preferably 1 or 2, amino acid residues are substituted, deleted or inserted in the α-helices and/or β-strands and/or connecting segments of its CTLD as well as nucleic acids encoding such derivatives. Specific derivatives appear from SEQ ID Nos: 02, 04, 09, 11, 13, 15, 29, 31, 36, and 38; and nucleic acids comprising nucleotide inserts encoding specific tetranectin derivatives appear from SEQ ID Nos: 12, 14, 35, and 37.

The invention comprises a method of constructing a tetranectin derivative adapted for the preparation of a combinatorial library according to the invention, wherein the nucleic acid encoding the tetranectin derivative has been modified to generate endonuclease restriction sites within nucleic acid segments encoding β2, β3 or β4, or up to 30 nucleotides upstream or downstream in the sequence from any nucleotide which belongs to a nucleic acid segment encoding β2, β3 or β4.

The invention also comprises the use of a nucleotide sequence encoding a tetranectin, or a derivative thereof wherein the scaffold structure of its CTLD is substantially maintained, for preparing a library of nucleotide sequences encoding related proteins by randomising part or all of the nucleic acid sequence encoding the loop region of its CTLD.

Further, the present invention provides nucleic acid comprising any nucleotide sequence encoding a protein according to the invention.

In particular, the invention provides a library of nucleic acids encoding proteins of a combinatorial library according to the invention, in which the members of the ensemble of nucleic acids, that collectively constitute said library of nucleic acids, are able to be expressed in a display system, which provides for a logical, physical or chemical link between entities displaying phenotypes representing properties of the displayed expression products and their corresponding genotypes.

In such a library the display system may be selected from
(I) a phage display system such as
   (1) a filamentous phage fd in which the library of nucleic acids is inserted into
      (a) a phagemid vector,
      (b) the viral genome of a phage
      (c) purified viral nucleic acid in purified single- or double-stranded form, or
   (2) a phage lambda in which the library is inserted into
      (a) purified phage lambda DNA, or
      (b) the nucleic acid in lambda phage particles; or
(II) a viral display system in which the library of nucleic acids is inserted into the viral nucleic acid of a eukaryotic virus such as baculovirus; or
(III) a cell-based display system in which the library of nucleic acids is inserted into, or adjoined to, a nucleic acid carrier able to integrate either into the host genome or into an extrachromosomal element able to maintain and express itself within the cell and suitable for cell-surface display on the surface of
   (a) bacterial cells,
   (b) yeast cells, or
   (c) mammalian cells; or
(IV) a nucleic acid entity suitable for ribosome linked display into which the library of nucleic acid is inserted; or
(V) a plasmid suitable for plasmid linked display into which the library of nucleic acid is inserted.

A well-known and useful display system is the "Recombinant Phage Antibody System" with the phagemid vector "pCANTAB 5E" supplied by Amersham Pharmacia Biotech (code no. 27-9401-01).

Further, the present invention provides a method of preparing a protein according to the invention, wherein the protein comprises at least one or more, identical or not identical, CTLD domains with novel loop-region sequences which has (have) been isolated from one or more CTLD libraries by screening or selection. At least one such CTLD domain may have been further modified by mutagenesis; and the protein containing at least one CTLD domain may have been assembled from two or more components by chemical or enzymatic coupling or crosslinking.

Also, the present invention provides a method of preparing a combinatorial library according to the invention comprising the following steps:
1) inserting nucleic acid encoding a protein comprising a model CTLD into a suitable vector,
2) if necessary, introducing restriction endonuclease recognition sites by site directed mutagenesis, said recognition sites being properly located in the sequence at or close to the ends of the sequence encoding the loop region of the CTLD or part thereof,
3) excising the DNA fragment encoding the loop region or part thereof by use of the proper restriction endonucleases,
4) ligating mixtures of DNA fragments into the restricted vector, and
5) inducing the vector to express randomised proteins having the scaffold structure of CTLDs in a suitable medium.

In a further aspect, the present invention provides a method of screening a combinatorial library according to the invention for binding to a specific target which comprises the following steps:
1) expressing a nucleic acids library according to any one of claims 59-61 to display the library of proteins in the display system;
2) contacting the collection of entities displayed with a suitably tagged target substance for which isolation of a CTLD-derived exhibiting affinity for said target substance is desired;
3) harvesting subpopulations of the entities displayed that exhibit affinity for said target substance by means of affinity-based selective extractions, utilizing the tag to which said target substance is conjugated or physically attached or adhering to as a vehicle or means of affinity purification, a procedure commonly referred to in the field as "affinity panning", followed by re-amplification of the sub-library;
4) isolating progressively better binders by repeated rounds of panning and re-amplification until a suitably small number of good candidate binders is obtained; and,
5) if desired, isolating each of the good candidates as an individual clone and subjecting it to ordinary functional and structural characterisation in preparation for final selection of one or more preferred product clones.

In a still further aspect, the present invention provides a method of reformatting a protein according to the invention or selected from a combinatorial library according to the invention and containing a CTLD variant exhibiting desired binding properties, in a desired alternative species-compatible framework by excising the nucleic acid fragment encoding the loop region-substituting polypeptide and any required single framework mutations from the nucleic acid encoding said protein using PCR technology, site directed mutagenesis or restriction enzyme digestion and inserting said nucleic acid fragment into the appropriate location(s) in a display- or protein expression vector that harbours a nucleic acid sequence encoding the desired alternative CTLD framework.

### BRIEF DESCRIPTION OF THE DRAWINGS

**Fig. 1** shows an alignment of the amino acid sequences of ten CTLDs of known 3D-structure. The sequence locations of main secondary structure elements are indicated above each sequence, labelled in sequential numerical order as "αN", denoting α-helix number N, and "βM", denoting β-strand number M.
   The four cysteine residues involved in the formation of the two conserved disulfide bridges of CTLDs are indicated and enumerated in the Figure as "C_{I}", "C_{II}", "C_{III}" and "C_{IV}", respectively. The two conserved disulfide bridges are C_{I}-C_{IV} and C_{II}-C_{III}, respectively.
   The ten C-type lectins are
   - hTN:: human tetranectin [Nielsen et al. (1997)];
   - MBP:: mannose binding protein [Weis et al. (1991); She- riff et al. (1994)];
   - SP-D:: surfactant protein D [Håkansson et al. (1999)];
   - LY49A:: NK receptor LY49A [Tormo et al. (1999)];
   - H1-ASR:: H1 subunit of the asialoglycoprotein receptor [Meier et al. (2000)];
   - MMR-4:: macrophage mannose receptor domain 4 [Feinberg et al. (2000)];
   - IX-A and IX-B:: coagulation factors IX/X-binding protein domain A and B, respectively [Mizuno et al. (1997)];
   - Lit:: lithostatine [Bertrand et al. (1996)];
   - TU14:: tunicate C-type lectin [Poget et al. (1999)].
**Fig. 2** shows an alignment of the nucleotide and amino acid sequences of the coding regions of the mature forms of human and murine tetranectin with an indication of known secondary structural elements.
   hTN: human tetranectin; nucleotide sequence from Berglund and Petersen (1992).
   mTN: murine tetranectin; nucleotide sequence from Sørensen et al. (1995).
   Secondary structure elements from Nielsen et al. (1997). "α" denotes an α-helix; "β" denotes a β-strand; and "L" denotes a loop.
**Fig. 3** shows an alignment of the nucleotide and amino acid sequences of human and murine tlec coding regions. htlec: the sequence derived from hTN; mtlec: the sequence derived from mTN. The position of the restriction endonuclease sites for *Bgl* II, *Kpn* I, and *Mun* I are indicated.
**Fig. 4** shows an alignment of the nucleotide and amino acid sequences of human and murine tCTLD coding regions. htCTLD: the sequence derived from hTN; mtCTLD: the sequence derived from mTN. The position of the restriction endonuclease sites for *Bgl* II, *Kpn* I, and *Mun* I are indicated.
**Fig. 5** shows an outline of the pT7H6FX-htlec expression plasmid. The FX-htlec fragment was inserted into pT7H6 [Christensen et al. (1991)] between the Bam HI and Hind III cloning sites.
**Fig. 6** shows the amino acid sequence (one letter code) of the FX-htlec part of the H6FX-htlec fusion protein produced by pT7H6FX-htlec.
**Fig. 7** shows an outline of the pT7H6FX-htCTLD expression plasmid. The FX-htCTLD fragment was inserted into pT7H6 [Christensen et al. (1991)] between the Bam HI and Hind III cloning sites.
**Fig. 8** shows the amino acid sequence (one letter code) of the FX-htCTLD part of the H6FX-htCTLD fusion protein produced by pT7H6FX-htCTLD.
**Fig. 9** shows an outline of the pPhTN phagemid. The PhTN fragment was inserted into the phagemid pCANTAB 5E (Amersham Pharmacia Biotech, code no. 27-9401-01) between the Sfi I and Not I restriction sites.
**Fig. 10** shows the amino acid sequence (one letter code) of the PhTN part of the PhTN-gene III fusion protein produced by pPhTN.
**Fig. 11** shows an outline of the pPhTN3 phagemid. The PhTN3 fragment was inserted into the phagemid pCANTAB 5E (Amersham Pharmacia Biotech, code no. 27-9401-01) between the Sfi I and Not I restriction sites.
**Fig. 12** shows the amino acid sequence (one letter code) of the PhTN3 part of the PhTN3-gene III fusion protein produced by pPhTN3.
**Fig. 13** shows an outline of the pPhtlec phagemid. The Phtlec fragment was inserted into the phagemid pCANTAB 5E (Amersham Pharmacia Biotech, code no. 27-9401-01) between the Sfi I and Not I restriction sites.
**Fig. 14** shows the amino acid sequence (one letter code) of the Phtlec part of the Phtlec-gene III fusion protein produced by pPhtlec.
**Fig. 15** shows an outline of the pPhtCTLD phagemid. The PhtCTLD fragment was inserted into the phagemid pCANTAB 5E (Amersham Pharmacia Biotech, code no. 27-9401-01) between the Sfi I and Not I restriction sites.
**Fig. 16** shows the amino acid sequence (one letter code) of the PhtCTLD part of the PhtCTLD-gene III fusion protein produced by pPhtCTLD.
**Fig. 17** shows an outline of the pUC-mtlec.
**Fig. 18** shows an outline of the pT7H6FX-mtlec expression plasmid. The FX-mtlec fragment was inserted into pT7H6 [Christensen et al. (1991)] between the Bam HI and Hind III cloning sites.
**Fig. 19** shows the amino acid sequence (one letter code) of the FX-mtlec part of the H6FX-mtlec fusion protein produced by pT7H6FX-mtlec.
**Fig. 20** shows an outline of the pT7H6FX-mtCTLD expression plasmid. The FX-mtCTLD fragment was inserted into pT7H6 [Christensen et al. (1991)] between the Bam HI and Hind III cloning sites.
**Fig. 21** shows the amino acid sequence (one letter code) of the FX-mtCTLD part of the H6FX-mtCTLD fusion protein produced by pT7H6FX-mtCTLD.
**Fig. 22** shows an outline of the pPmtlec phagemid. The Pmtlec fragment was inserted into the phagemid pCANTAB 5E (Amersham Pharmacia Biotech, code no. 27-9401-01) between the Sfi I and Not I restriction sites.
**Fig. 23** shows the amino acid sequence (one letter code) of the Pmtlec part of the Pmtlec-gene III fusion protein produced by pPmtlec.
**Fig. 24** shows an outline of the pPmtCTLD phagemid. The PmtCTLD fragment was inserted into the phagemid pCANTAB 5E (Amersham Pharmacia Biotech, code no. 27-9401-01) between the Sfi I and Not I restriction sites.
**Fig. 25** shows the amino acid sequence (one letter code) of the PmtCTLD part of the PmtCTLD-gene III fusion protein produced by pPmtCTLD.
**Fig. 26** shows an ELISA-type analysis of Phtlec-, PhTN3-, and M13KO7 helper phage binding to anti-tetranectin or BSA. Panel A: Analysis with 3% skimmed milk/5 mM EDTA as blocking reagent. Panel B: Analysis with 3% skimmed milk as blocking reagent.
**Fig. 27** shows an ELISA-type analysis of Phtlec-, PhTN3-, and M13KO7 helper phage binding to plasminogen (Plg) and BSA. Panel A: Analysis with 3% skimmed milk/5 mM EDTA as blocking reagent. Panel B: Analysis with 3% skimmed milk as blocking reagent.
**Fig. 28** shows an ELISA-type analysis of the B series and C series polyclonal populations, from selection round 2, binding to plasminogen (Plg) compared to background.
**Fig. 29** Phages from twelve clones isolated from the third round of selection analysed for binding to hen egg white lysozyme, human β₂-microglobulin and background in an ELISA-type assay.
**Fig. 30** shows the amino acid sequence (one letter code) of the PrMBP part of the PrMBP-gene III fusion protein produced by pPrMBP.
**Fig. 31** shows an outline of the pPrMBP phagemid. The PrMBP fragment was inserted into the phagemid pCANTAB 5E (Amersham Pharmacia Biotech, code no. 27-9401-01) between the Sfi I and Not I restriction sites.
**Fig. 32** shows the amino acid sequence (one letter code) of the PhSP-D part of the PhSP-D-gene III fusion protein produced by pPhSP-D.
**Fig. 33** shows an outline of the pPhSP-D phagemid. The PhSP-D fragment was inserted into the phagemid pCANTAB 5E (Amersham Pharmacia Biotech, code no. 27-9401-01) between the Sfi I and Not I restriction sites.
**Fig. 34****.** Phages from 48 clones isolated from the third round of selection in the #1 series analysed for binding to hen egg white lysozyme and to A-HA in an ELISA-type assay.
**Fig. 35****.** Phages from 48 clones isolated from the third round of selection in the #4 series analysed for binding to hen egg white lysozyme and to A-HA in an ELISA-type assay.

### DETAILED DESCRIPTION OF THE INVENTION

### I. Definitions

The terms "C-type lectin-like protein" and "C-type lectin" are used to refer to any protein present in, or encoded in the genomes of, any eukaryotic species, which protein contains one or more CTLDs or one or more domains belonging to a subgroup of CTLDs, the CRDs, which bind carbohydrate ligands. The definition specifically includes membrane attached C-type lectin-like proteins and C-type lectins, "soluble" C-type lectin-like proteins and C-type lectins lacking a functional transmembrane domain and variant C-type lectin-like proteins and C-type lectins in which one or more amino acid residues have been altered in vivo by glycosylation or any other postsynthetic modification, as well as any product that is obtained by chemical modification of C-type lectin-like proteins and C-type lectins.

In the claims and throughout the specification certain alterations may be defined with reference to amino acid residue numbers of a CTLD domain or a CTLD-containing protein. The amino acid numbering starts at the first N-terminal amino acid of the CTLD or the native or artificial CTLD-containing protein product, as the case may be, which shall in each case be indicated by unambiguous external literature reference or internal reference to a figure contained herein within the textual context.

The terms "amino acid", "amino acids" and "amino acid residues" refer to all naturally occurring L-α-amino acids. This definition is meant to include norleucine, ornithine, and homocysteine. The amino acids are identified by either the single-letter or three-letter designations:

| | |
|---|---|
| Asp D aspartic acid | Ile I isoleucine |
| Thr T threonine | Leu L leucine |
| Ser S serine | Tyr Y tyrosine |
| Glu E glutamic acid | Phe F phenylalanine |
| Pro P proline | His H histidine |
| Gly G glycine | Lys K lysine |
| Ala A alanine | Arg R arginine |
| Cys C cysteine | Trp W tryptophan |
| Val V valine | Gln Q glutamine |
| Met M methionine | Asn N asparagine |
| Nle J norleucine | Orn O ornithine |
| Hcy U homocysteine | Xxx X any L-α-amino acid. |

The naturally occurring L-α-amino acids may be classified according to the chemical composition and properties of their side chains. They are broadly classified into two groups, charged and uncharged. Each of these groups is divided into subgroups to classify the amino acids more accurately:

### A. Charged Amino Acids

| | |
|---|---|
| Acidic Residues: | Asp, Glu |
| Basic Residues: | Lys, Arg, His, Orn |

### B. Uncharged Amino Acids

| | |
|---|---|
| Hydrophilic Residues: | Ser, Thr, Asn, Gln |
| Aliphatic Residues: | Gly, Ala, Val, Leu, Ile, Nle |
| Non-polar Residues: | Cys, Met, Pro, Hcy |
| Aromatic Residues: | Phe, Tyr, Trp |

The terms "amino acid alteration" and "alteration" refer to amino acid substitutions, deletions or insertions or any combinations thereof in a CTLD amino acid sequence. In the CTLD variants of the present invention such alteration is at a site or sites of a CTLD amino acid sequence. Substitutional variants herein are those that have at least one amino acid residue in a native CTLD sequence removed and a different amino acid inserted in its place at the same position. The substitutions may be single, where only one amino acid in the molecule has been substituted, or they may be multiple, where two or more amino acids have been substituted in the same molecule.

The designation of the substitution variants herein consists of a letter followed by a number followed by a letter. The first (leftmost) letter designates the amino acid in the native (unaltered) CTLD or CTLD-containing protein. The number refers to the amino acid position where the amino acid substitution is being made, and the second (righthand) letter designates the amino acid that is used to replace the native amino acid. As mentioned above, the numbering starts with "1" designating the N-terminal amino acid sequence of the CTLD or the CTLD-containing protein, as the case may be. Multiple alterations are separated by a comma (,) in the notation for ease of reading them.

The terms "nucleic acid molecule encoding", "DNA sequence encoding", and "DNA encoding" refer to the order or sequence of deoxyribonucleotides along a strand of deoxyribonucleic acid. The order of these deoxyribonucleotides determines the order of amino acids along the polypeptide chain. The DNA sequence thus encodes the amino acid sequence.

The terms "mutationally randomised sequence", "randomised polypeptide segment", "randomised amino acid sequence", "randomised oligonucleotide" and "mutationally randomised sequence", as well as any similar terms used in any context to refer to randomised sequences, polypeptides or nucleic acids, refer to ensembles of polypeptide or nucleic acid sequences or segments, in which the amino acid residue or nucleotide at one or more sequence positions may differ between different members of the-ensemble of polypeptides or nucleic acids, such that the amino acid residue or nucleotide occurring at each such sequence position may belong to a set of amino acid residues or nucleotides that may include all possible amino acid residues or nucleotides or any restricted subset thereof. Said terms are often used to refer to ensembles in which the number of amino acid residues or nucleotides is the same for each member of the ensemble, but may also be used to refer to such ensembles in which the number of amino acid residues or nucleotides in each member of the ensemble may be any integer number within an appropriate range of integer numbers.

### II. Construction and utility of combinatorial CTLD libraries

Several systems displaying phenotype, in terms of putative ligand binding modules or modules with putative enzymatic activity, have been described. These include: phage display (e.g. the filamentous phage fd [Dunn (1996), Griffiths amd Duncan (1998), Marks et al. (1992)], phage lambda [Mikawa et al. (1996)]), display on eukarotic virus (e.g. baculovirus [Ernst et al. (2000)]), cell display (e.g. display on bacterial cells [Benhar et al. (2000)], yeast cells [Boder and Wittrup (1997)], and mammalian cells [Whitehorn et al. (1995)], ribosome linked display [Schaffitzel et al. (1999)], and plasmid linked display [Gates et al. (1996)].

The most commonly used method for phenotype display and linking this to genotype is by phage display. This is accomplished by insertion of the reading frame encoding the scaffold protein or protein of interest into an intradomain segment of a surface exposed phage protein. The filamentous phage fd (e.g. M13) has proven most useful for this purpose. Polypeptides, protein domains, or proteins are the most frequently inserted either between the "export" signal and domain 1 of the fd gene III protein or into a so-called hinge region between domain 2 and domain 3 of the fd-phage gene III protein. Human antibodies are the most frequently used proteins for the isolation of new binding units, but other proteins and domains have also been used (e.g. human growth hormone [Bass et al. (1990)], alkaline phosphatase [McCafferty et al. (1991)], β-lactamase inhibitory protein [Huang et al. (2000)], and cytotoxic T lymphocyte-associated antigen 4 [Hufton et al. (2000)]. The antibodies are often expressed and presented as scFv or Fab fusion proteins. Three strategies have been employed. Either a specific antibody is used as a scaffold for generating a library of mutationally randomised sequences within the antigen binding clefts [e.g. Fuji et al. (1998)] or libraries representing large ensembles of human antibody encoding genes from nonimmunised hosts [e.g. Nissim et al. (1994)] or from immunised hosts [e.g. Cyr and Hudspeth (2000)] are cloned into the fd phage vector.

The general procedure for accomplishing the generation of a display system for the generation of CTLD libraries comprise essentially
(1) identification of the location of the loop-region, by referring to the 3D structure of the CTLD of choice, if such information is available, or, if not, identification of the sequence locations of the β2-, β3- and β4 strands by sequence alignment with the sequences shown in Fig. 1, as aided by the further corroboration by identification of sequence elements corresponding to the β2 and β3 consensus sequence elements and β4-strand characteristics, also disclosed above;
(2) subcloning of a nucleic acid fragment encoding the CTLD of choice in a protein display vector system with or without prior insertion of endonuclease restriction sites close to the sequences encoding β2, β3 and β4; and
(3) substituting the nucleic acid fragment encoding some or all of the loop-region of the CTLD of choice with randomly selected members of an ensemble consisting of a multitude of nucleic acid fragments which after insertion into the nucleic acid context encoding the receiving framework will substitute the nucleic acid fragment encoding the original loop-region polypeptide fragments with randomly selected nucleic acid fragments. Each of the cloned nucleic acid fragments, encoding a new polypeptide replacing an original loop-segment or the entire loop-region, will be decoded in the reading frame determined within its new sequence context.

Nucleic acid fragments may be inserted in specific locations into receiving nucleic acids by any common method of molecular cloning of nucleic acids, such as by appropriately designed PCR manipulations in which chemically synthesized nucleic acids are copy-edited into the receiving nucleic acid, in which case no endonuclease restriction sites are required for insertion. Alternatively, the insertion/excision of nucleic acid fragments may be facilitated by engineering appropriate combinations of endonuclease restriction sites into the target nucleic acid into which suitably designed oligonucleotide fragments may be inserted using standard methods of molecular cloning of nucleic acids.

It will be apparent that interesting CTLD variants isolated from CTLD libraries in which restriction endonuclease sites have been inserted for convenience may contain mutated or additional amino acid residues that neither correspond to residues present in the original CTLD nor are important for maintaining the interesting new affinity of the CTLD variant. If desirable, e.g. in case the product needs to be rendered as non-immunogenic as possible, such residues may be altered or removed by backmutation or deletion in the specific clone, as appropriate.

The ensemble consisting of a multitude of nucleic acid fragments may be obtained by ordinary methods for chemical synthesis of nucleic acids by directing the step-wise synthesis to add pre-defined combinations of pure nucleotide monomers or a mixture of any combination of nucleotide monomers at each step in the chemical synthesis of the nucleic acid fragment. In this way it is possible to generate any level of sequence degeneracy, from one unique nucleic acid sequence to the most complex mixture, which will represent a complete or incomplete representation of maximum number unique sequences of 4^{N}, where N is the number of nucleotides in the sequence.

Compl,ex ensembles consisting of multitudes of nucleic acid fragments may, alternatively, be prepared by generating mixtures of nucleic acid fragments by chemical, physical or enzymatic fragmentation of high-molecular mass nucleic acid compositions like, e.g., genomic nucleic acids extracted from any organism. To render such mixtures of nucleic acid fragments useful in the generation of molecular ensembles, as described here, the crude mixtures of fragments, obtained in the initial cleavage step, would typically be size-fractionated to obtain fragments of an approximate molecular mass range which would then typically be adjoined to a suitable pair of linker nucleic acids, designed to facilitate insertion of the linker-embedded mixtures of size-restricted oligonucleotide fragments into the receiving nucleic acid vector.

To facilitate the construction of combinatorial CTLD libraries in tetranectin, the model CTLD of the preferred embodiment of the invention, suitable restriction sites located in the vicinity of the nucleic acid sequences encoding β2, β3 and β4 in both human and murine tetranectin were designed with minimal perturbation of the polypeptide sequence encoded by the altered sequences. It was found possible to establish a design strategy, as detailed below, by which identical endonuclease restriction sites could be introduced at corresponding locations in the two sequences, allowing interesting loop-region variants to be readily excised from a recombinant murine CTLD and inserted correctly into the CTLD framework of human tetranectin or *vice versa.*

Analysis of the nucleotide sequence encoding the mature form of human tetranectin reveals (Fig. 2) that a recognition site for the restriction endonuclease *Bgl* II is found at position 326 to 331 (AGATCT), involving the encoded residues Glu109, Ile110, and Trp111 of β2, and that a recognition site for the restriction endonuclease *Kas I* is found at position 382 to 387 (GGCGCC), involving the encoded amino acid residues Gly128 and Ala129 (located C-terminally in loop 2).

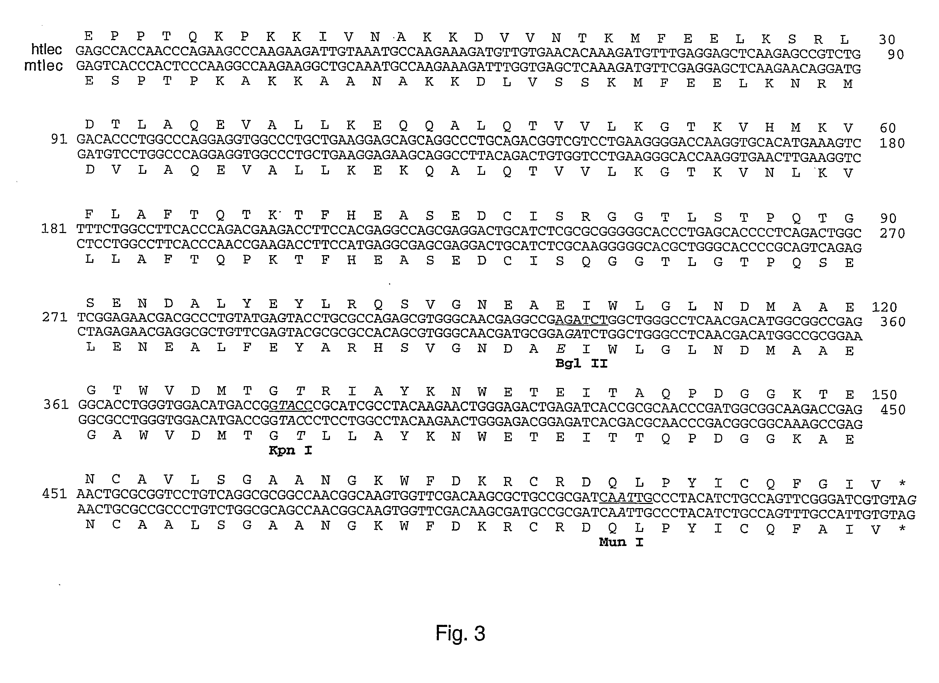

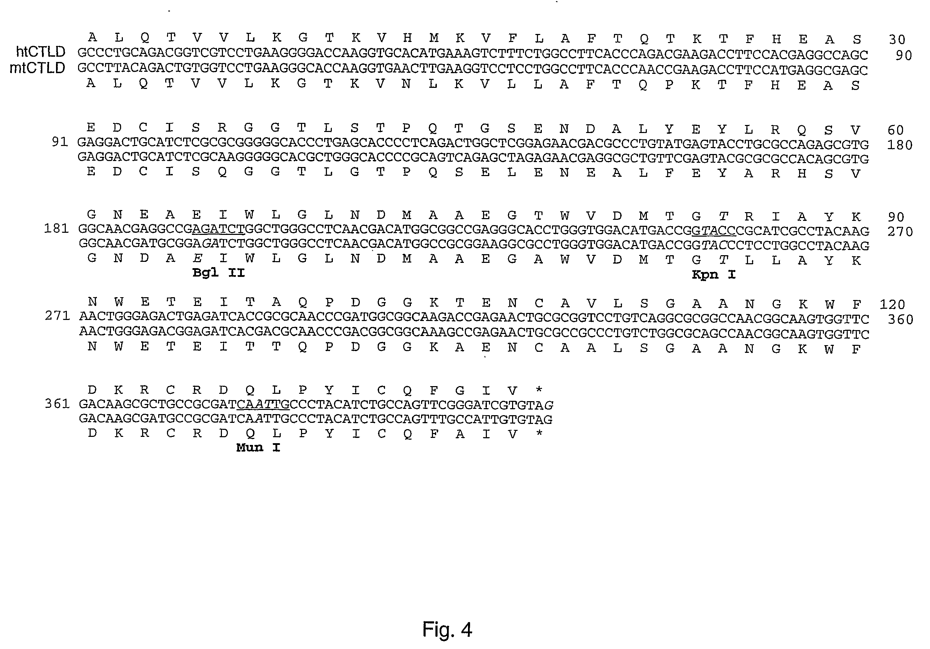

Mutation, by site directed mutagenesis, of G513 to A and of C514 to T in the nucleotide sequence encoding human tetranectin would introduce a *Mun I* restriction endonuclease recognition site therein, located at position 511 to 516, and mutation of G513 to A in the nucleotide sequence encoding murine tetranectin would introduce a *Mun I* restriction endonuclease site therein at a position corresponding to the *Mun I* site in human tetranectin, without affecting the amino acid sequence of either of the encoded protomers. Mutation, by site directed mutagenesis, of C327 to G and of G386 to C in the nucleotide sequence encoding murine tetranectin would introduce a *Bgl* II and a *Kas* I restriction endonuclease recognition site, respectively, therein. Additionally, A325 in the nucleotide sequence encoding murine tetranectin is mutagenized to a G. These three mutations would affect the encoded amino acid sequence by substitution of Asn109 to Glu and Gly129 to Ala, respectively. Now, the restriction endonuclease *Kas I* is known to exhibit marked site preference and cleaves only slowly the tetranectin coding region. Therefore, a recognition site for another restriction endonuclease substituting the *Kas I* site is preferred (e.g. the recognition site for the restriction endonuclease *Kpn I,* recognition sequence GGTACC). The nucleotide and amino acid sequences of the resulting tetranectin derivatives, human tetranectin lectin (htlec) and murine tetranectin lectin (mtlec) are shown in Fig. 3. The nucleotide sequences encoding the htlec and mtlec protomers may readily be subcloned into devices enabling protein display of the linked nucleotide sequence (e.g. phagemid vectors) and into plasmids designed for heterologous expression of protein [e.g. pT7H6, Christensen et al. (1991)]. Other derivatives encoding only the mutated CTLDs of either htlec or mtlec (htCTLD and mtCTLD, respectively) have also been constructed and subcloned into phagemid vectors and expression plasmids, and the nucleotide and amino acid sequences of these CTLD derivatives are shown in Fig. 4.

The presence of a common set of recognition sites for the restriction endonucleases *Bgl* II, *Kas* I or *Kpn* I, and *Mun* I in the ensemble of tetranectin and CTLD derivatives allows for the generation of protein libraries with randomised amino acid sequence in one or more of the loops and at single residue positions in β4 comprising the lectin ligand binding region by ligation of randomised oligonucleotides into properly restricted phagemid vectors encoding htlec, mtlec, htCTLD, or mtCTLD derivatives.

After rounds of selection on specific targets (e.g. eukaryotic cells, virus, bacteria, specific proteins, polysaccharides, other polymers, organic compounds etc.) DNA may be isolated from the specific phages, and the nucleotide sequence of the segments encoding the ligand-binding region determined, excised from the phagemid DNA and transferred to the appropriate derivative expression vector for heterologous production of the desired product. Heterologous production in a prokaryote may be preferred because an efficient protocol for the isolation and refolding of tetranectin and derivatives has been reported (International Patent Application Publication WO 94/18227 A2).

A particular advantage gained by implementing the technology of the invention, using tetranectin as the scaffold structure, is that the structures of the murine and human tetranectin scaffolds are almost identical, allowing loop regions to be swapped freely between murine and human tetranectin derivatives with retention of functionality. Swapping of loop regions between the murine and the human framework is readily accomplished within the described system of tetranectin derivative vectors, and it is anticipated, that the system can be extended to include other species (e.g. rat, old and new world monkeys, dog, cattle, sheep, goat etc.) of relevance in medicine or veterinary medicine in view of the high level of homology between man and mouse sequences, even at the genetic level. Extension of this strategy to include more species may be rendered possible as and when tetranectin is eventually cloned and/or sequenced from such species.

Because the C-type lectin ligand-binding region represents a different topological unit compared to the antigen binding clefts of the antibodies, we envisage that the selected binding specificities will be of a different nature compared to the antibodies. Further, we envisage that the tetranectin derivatives may have advantages compared to antibodies with respect to specificity in binding sugar moieties or polysaccharides. The tetranectin derivatives may also be advantageous in selecting binding specificities against certain natural or synthetic organic compounds.

Several CTLDs are known to bind calcium ions, and binding of other ligands is often either dependent on calcium (e.g. the collectin family of C-type lectins, where the calcium ion bound in site 2 is directly involved in binding the sugar ligand [Weis and Drickamer (1996)]) or sensitive to calcium (e.g. tetranectin, where binding of calcium involves more of the side chains known otherwise to be involved in plasminogen kringle 4 binding [Graversen et al. (1998)]). The calcium binding sites characteristic of the C-type lectin-like protein family are comprised by residues located in loop 1, loop 4 and β-strand 4 and are dependent on the presence of a proline residue (often interspacing loop 3 and loop 4 in the structure), which upon binding is found invariantly in the cis conformation. Moreover, binding of calcium is known to enforce structural changes in the CTLD loop-region [Ng et al. (1998a,b)]. We therefore envisage, that binding to a specific target ligand by members of combinational libraries with preserved CTLD metal binding sites may be modulated by addition or removal of divalent metal ions (e.g. calcium ions) either because the metal ion may be directly involved in binding, because it is a competitive ligand, or because binding of the metal ion enforces structural rearrangements within the putative binding site.

The trimeric nature of several members of the C-type lectin and C-type lectin-like protein family, including tetranectin, and the accompanying avidity in binding may also be exploited in the creation of binding units with very high binding affinity.

As can be appreciated from the disclosure above, the present invention has a broad general scope and a wide area of application. Accordingly, the following examples, describing various embodiments thereof, are offered by way of illustration only, not by way of limitation.

### Example 1

### Construction of tetranectin derived E.coli expression plasmids and phagemids

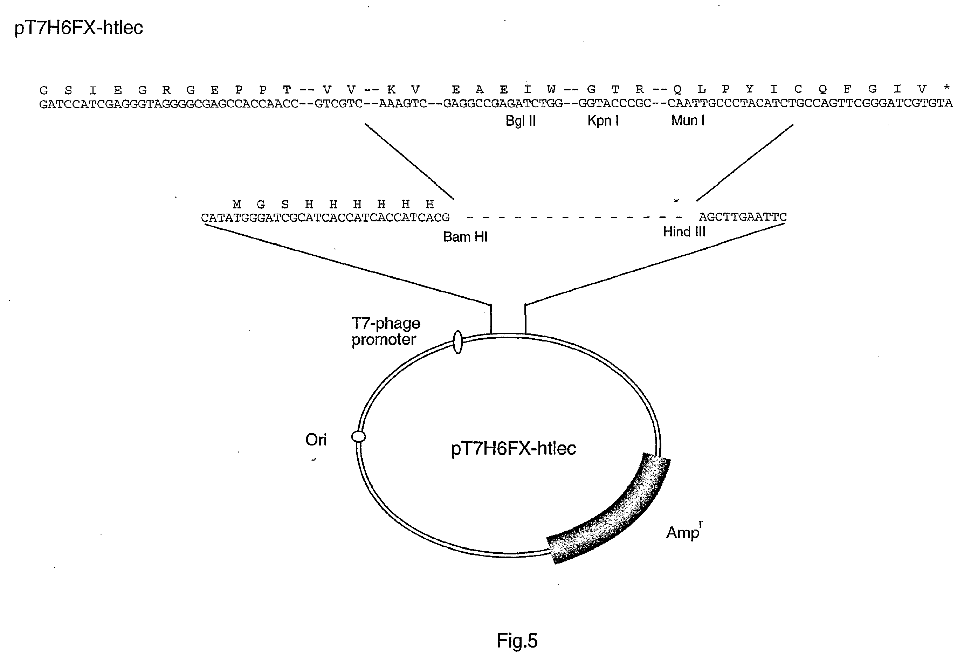

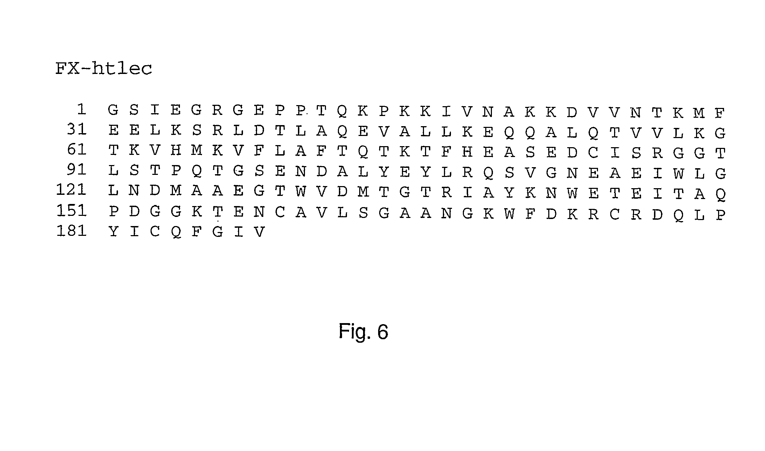

The expression plasmid pT7H6FX-htlec, encoding the FX-htlec (SEQ ID NO:01) part of full length H6FX-htlec fusion protein, was constructed by a series of four consecutive site-directed mutagenesis experiments starting from the expression plasmid pT7H6-rTN 123 [Holtet et al. (1997)] using the QuickChange^{™} Site-Directed Mutagenesis Kit (Stratagene, La Jolla, CA) and performed as described by the manufacturer. Mismatching primer pairs introducing the desired mutations were supplied by DNA Technology (Aarhus, Denmark). An outline of the resulting pT7H6FX-htlec expression plasmid is shown in Fig. 5, and the nucleotide sequence of the FX-htlec encoding insert is given as SEQ ID NO:01. The amino acid sequence of the FX-htlec part of the H6FX-htlec fusion protein is shown in Fig. 6 and given as SEQ ID NO:02.

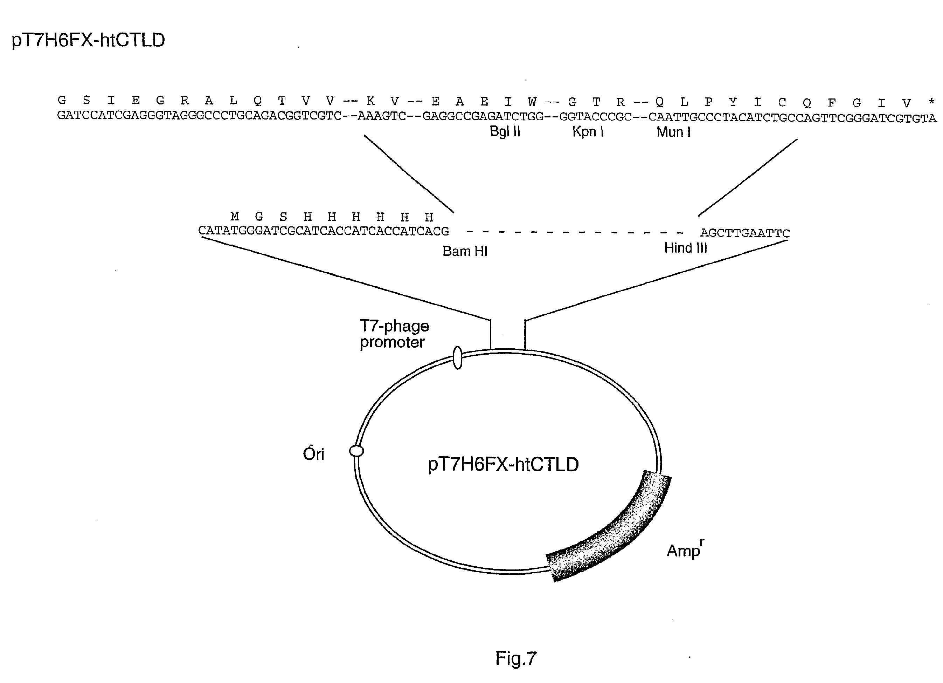

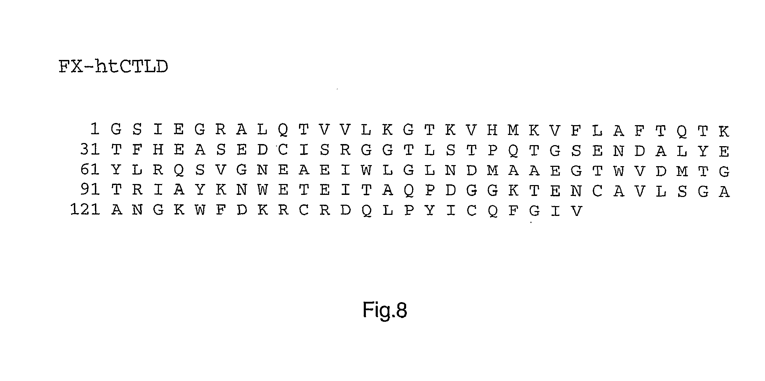

The expression plasmid pT7H6FX-htCTLD, encoding the FX-htCTLD (SEQ ID NO: 03) part of the H6FX-htCTLD fusion protein, was constructed by amplification and subcloning into the plasmid pT7H6 (i.e. amplification in a polymerase chain reaction using the expression plasmid pT7H6-htlec as template, and otherwise the primers, conditions, and subcloning procedure described for the construction of the expression plasmid pT7H6TN3 [Holtet et al. (1997)]. An outline of the resulting pT7H6FX-htCTLD expression plasmid is shown in Fig. 7, and the nucleotide sequence of the FX-htCTLD encoding insert is given as SEQ ID NO:03. The amino acid sequence of the FX-htCTLD part of the H6FX-htCTLD fusion protein is shown in Fig. 8 and given as SEQ ID NO:04.

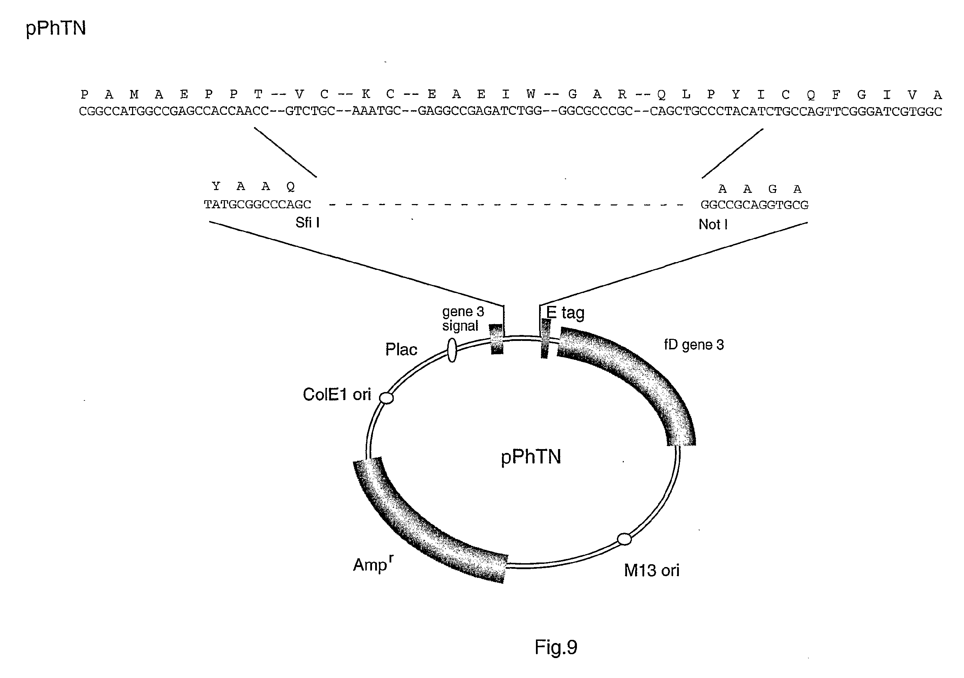

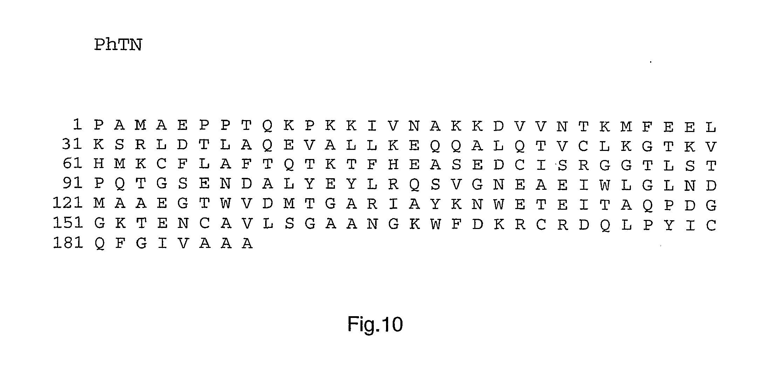

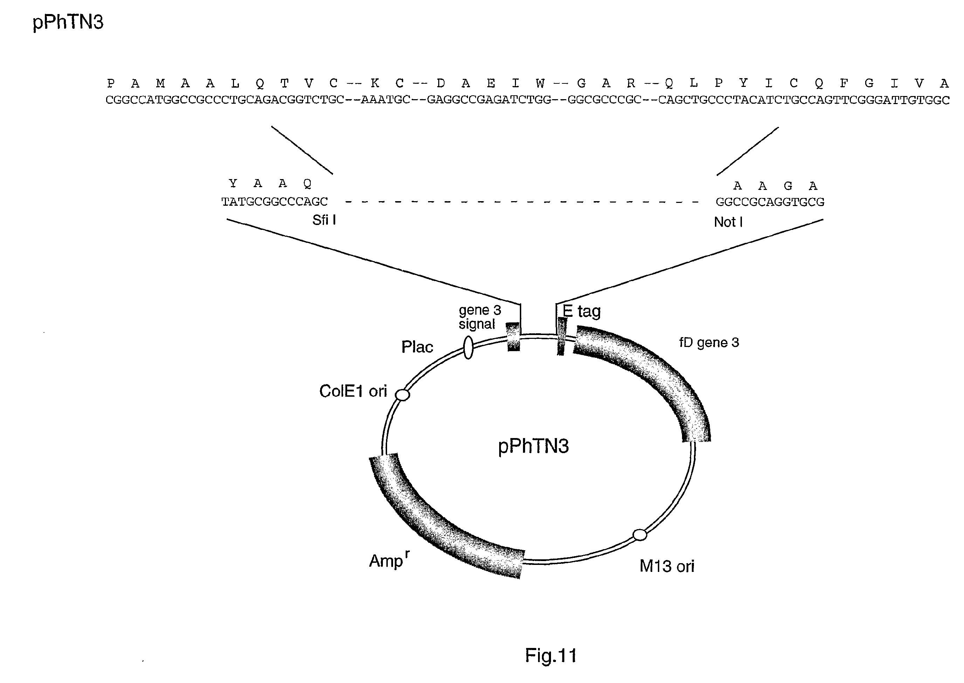

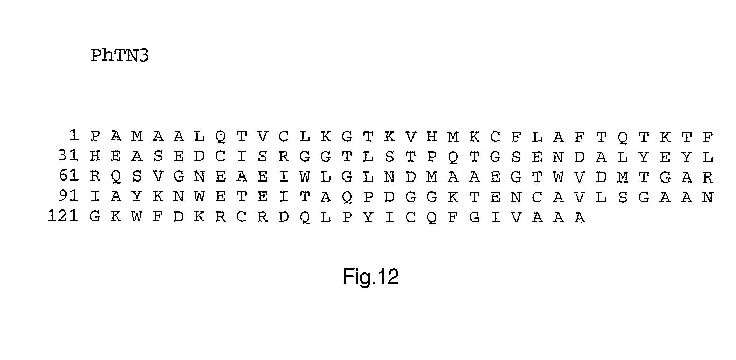

The phagemids, pPhTN and pPhTN3, were constructed by ligation of the *Sfi* I and *Not* I restricted DNA fragments amplified from the expression plasmids pT7H6-rTN 123 (with the oligonucleotide primers 5-CGGCTGAGCGGCCCA-GCCGGCCATGGCCGAGCCACCAACCCAGAAGC-3' [SEQ ID NO:05] and 5'-CCTGCGGCCGCCACGATCCCGAACTGG-3' [SEQ ID NO:06]) and pT7H6FX-htCTLD (with the oligonucleotide primers 5'-CGGCTGAGCGGCCCAGCCGGCCATGGCCGCCCTGCAGACGGTC-3' [SEQ ID NO:07] and 5'-CCTGCGGCCGCCACGATCCCGAACTGG-3' [SEQ ID NO:06]), respectively, into a *Sfi* I and *Not* I precut vector, pCANTAB 5E supplied by Amersham Pharmacia Biotech (code no. 27-9401-01) using standard procedures. Outlines of the resulting pPhTN and pPhTN3 phagemids are shown in Fig. 9 and Fig. 11, respectively, and the nucleotide sequences of the PhTN and PhTN3 inserts are given as SEQ ID NO:08 and SEQ ID NO:10, respectively. The amino acid sequences encoded by the PhTN and PhTN3 inserts are shown in Fig 10 (SEQ ID NO:09) and Fig. 12 (SEQ ID NO:11), respectively.

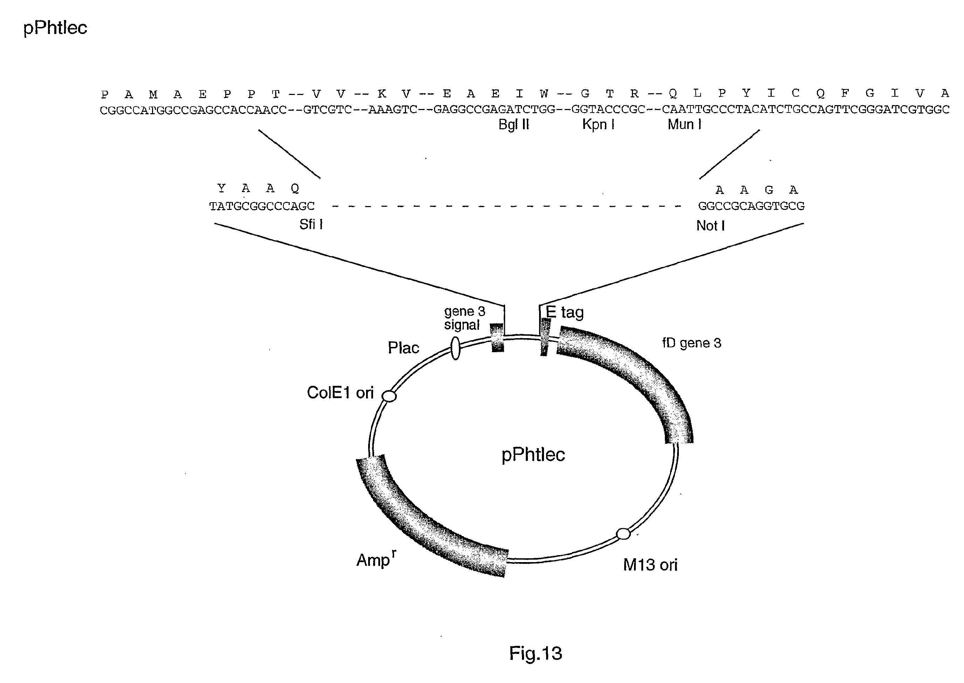

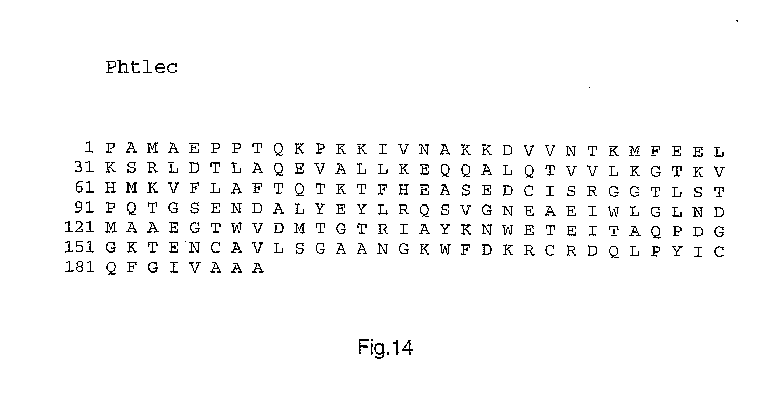

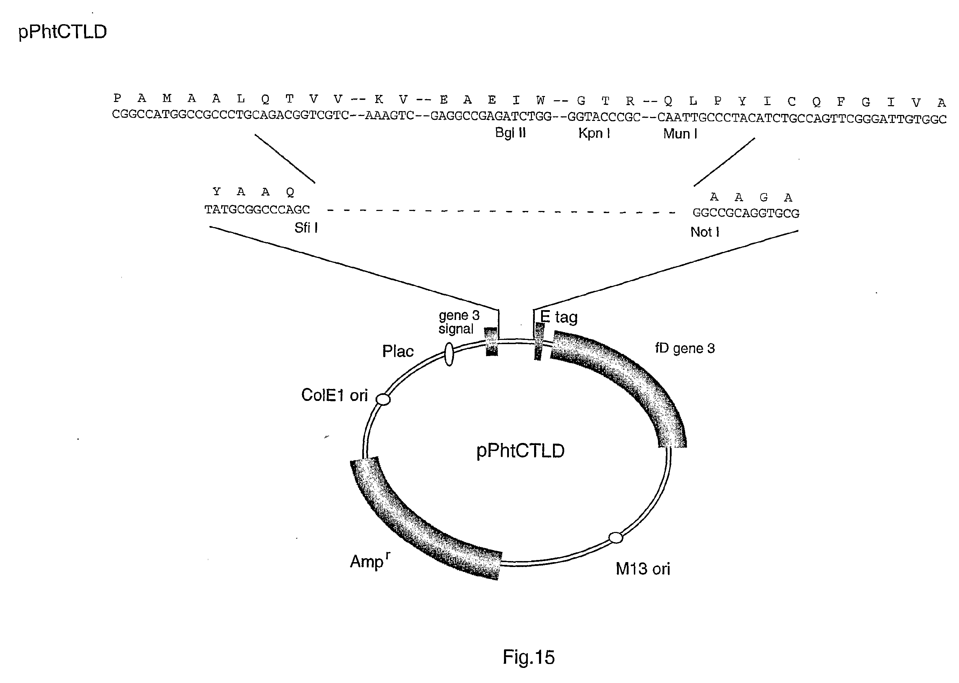

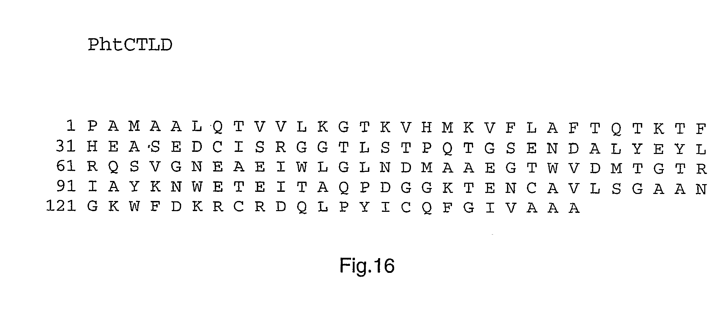

The phagemids, pPhtlec and pPhtCTLD, were constructed by ligation of the *Sfi* I and *Not* I restricted DNA fragments amplified from the expression plasmids pT7H6FX-htlec (with the oligonucleotide primers 5-CGGCTGAGCGGCCCAGCC-GGCCATGGCCGAGCCACCAACCCAGAAGC-3' [SEQ ID NO:05] and 5'-CCTGCGGCCGCCACGATCCCGAACTGG-3' [SEQ ID NO:06]) and pT7H6FX-htCTLD (with the oligonucleotide primers 5'-CGGCTGAGCGGCCCAGCCGGCCATGGCCGCCCTGCAGACGGTC-3' [SEQ ID NO:07] and 5'-CCTGCGGCCGCCACGATCCCGAACTGG-3' [SEQ ID NO:06]), respectively, into a *Sfi* I and *Not* I precut vector, pCANTAB 5E supplied by Amersham Pharmacia Biotech (code no. 27-9401-01) using standard procedures. Outlines of the resulting pPhtlec and pPhtCTLD phagemids are shown in Fig. 13 and Fig. 15, respectively, and the nucleotide sequences of the Phtlec and PhtCTLD inserts are given as SEQ ID NO:12 and SEQ ID NO:14, repectively. The amino acid sequences encoded by the Phtlec and PhtCTLD inserts are shown in Fig. 14 (SEQ ID NO:13) and Fig. 16 (SEQ ID NO:15), respectively.

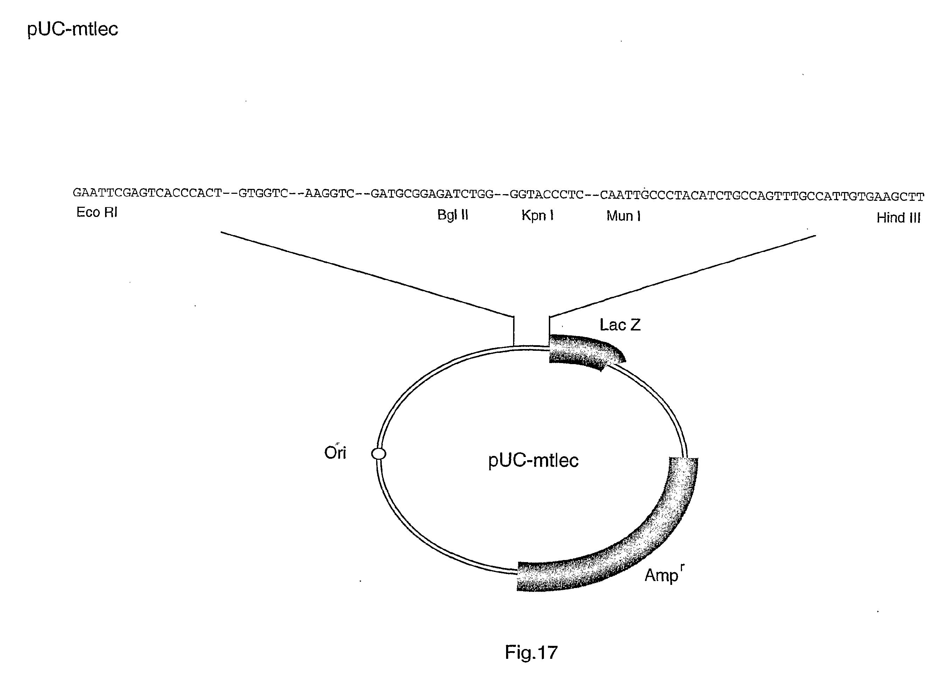

A plasmid clone, pUC-mtlec, containing the nucleotide sequence corresponding to the murine tetranectin derivative mtlec (Fig. 3 and SEQ ID NO:16) was constructed by four succesive subclonings of DNA subfragments in the following way: First, two oligonucleotides 5'-CGGAATTCGAGTCACCCACTCCCAAGGCCAAGAAGGCTGCAAATGCCAAGAAA-GATTTGGTGAGCTCAAAGATGTTC-3' (SEQ ID NO:17) and 5'-GCG-GATCCAGGCCTGCTTCTCCTTCAGCAGGGCCACCTCCTGGGCCAGGACATCCAT-CCTGTTCTTGAGCTCCTCGAACATCTTTGAGCTCACC-3' (SEQ ID NO:18) were annealed and after a filling in reaction cut with the restriction endonucleases Eco RI (GAATTC) and *Bam* HI (GGATCC) and ligated into Eco RI and *Bam* HI precut pUC18 plasmid DNA. Second, a pair of oligonucleotides 5'-GCA-GGCCTTACAGACTGTGTGCCTGAAGGGCACCAAGGTGAACTTGAAGTGCCTCCT-GGCCTTCACCCAACCGAAGACCTTCCATGAGGCGAGCGAG-3' (SEQ ID NO:19) and 5'-CCGCATGCTTCGAACAGCGCCTCGTTCTCTAGCTCTGAC-TGCGGGGTGCCCAGCGTGCCCCCTTGCGAGATGCAGTCCTCGCTCGCCTCATGG-3' (SEQ ID NO:20) was annealed and after a filling in reaction cut with the restriction endonucleases Stu I (AGGCCT) and *Sph* I (GCATGC) and ligated into the Stu I and *Sph* I precut plasmid resulting from the first ligation. Third, an oligonucleotide pair 5'-GGTTCGAATACGCGC-GCCACAGCGTGGGCAACGATGCGGAGATCTAAATGCTCCCAATTGC-3' (SEQ ID NO:21) and 5'-CCAAGCTTCACAATGGCAAACTGGCAGATGTAGGGCAATTGG-GAGCATTTAGATC-3' (SEQ ID NO: 22) was annealed and after a filling in reaction cut with the restriction endonucleases *BstB* I (TTCGAA) and *Hind* III (AAGCTT) and ligated into the BstB I and *Hind* III precut plasmid resulting from the second ligation. Fourth, an oligonucleotide pair 5'-CGGAGATCTGGCTGGGCCTCAACGACATGGCCGCGGAAGGCGCCTGGGTGGA-CATGACCGGTACCCTCCTGGCCTACAAGAACTGG-3' (SEQ ID NO:23) and 5'-GGGCAATTGATCGCGGCATCGCTTGTCGAACCTCTTGCCGTTGGCTGCGCCAG-ACAGGGCGGCGCAGTTCTCGGCTTTGCCGCCGTCGGGTTGCGTCGTGATCTCCGTC-TCCCAGTTCTTGTAGGCCAGG-3' (SEQ ID NO:24) was annealed and after a filling in reaction cut with the restriction endonucleases *Bgl* II (AGATCT) and *Mun* I (CAATTG) and ligated into the *Bgl* II and *Mun* I precut plasmid resulting from the third ligation. An outline of the pUC-mtlec plasmid is shown in Fig. 17, and the resulting nucleotide sequence of the Eco RI. to *Hind* III insert is given as SEQ ID NO:16.

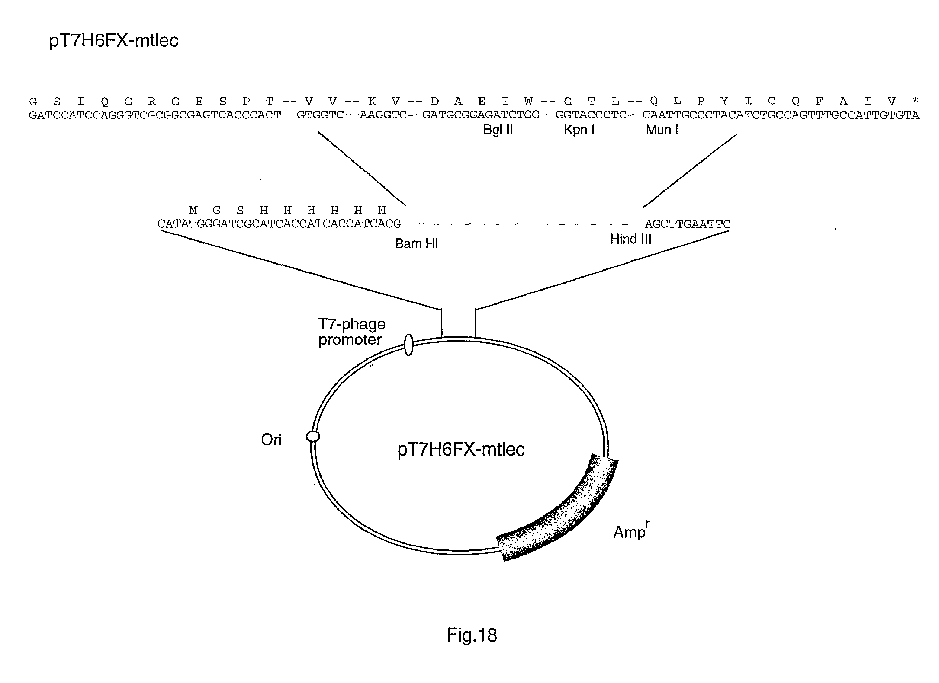

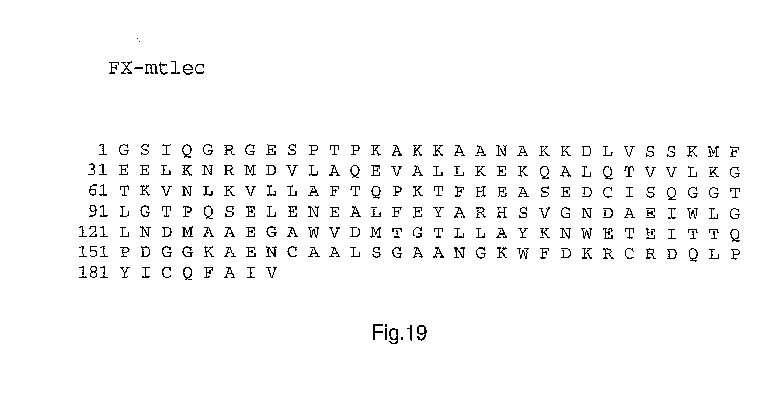

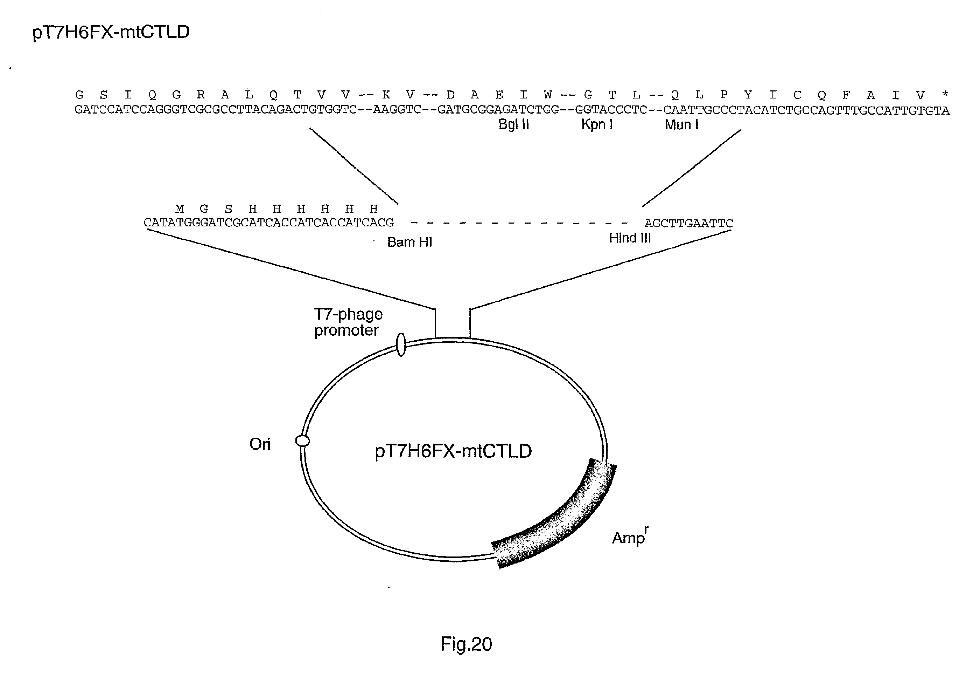

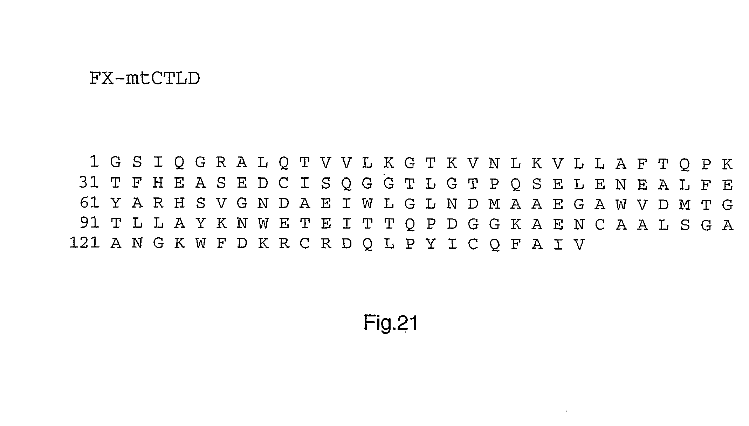

The expression plasmids pT7H6FX-mtlec and pT7H6FX-mtCTLD may be constructed by ligation of the *Bam* HI and *Hind* III restricted DNA fragments, amplified from the pUC-mtlec plasmid with the oligonucleotide primer pair 5-CTGGGATCC-TCCAGGGTCGCGAGTCACCCACTCCCAAGG-3' (SEQ ID NO:25) and 5'-CCGAAGCTTACACAATGGCAAACTGGC-3' (SEQ ID NO:26), and with the oligonucleotide primer pair 5'- CTGGGATCCATCCAGGGTCG-CGCCTTACAGACTGTGGTC-3' (SEQ ID NO:27), and 5'-CCGAAGCTT-ACACAATGGCAAACTGGC-3' (SEQ ID NO:26), respectively, into *Bam* HI and *Hind* III precut pT7H6 vector using standard procedures. An outline of the expression plasmids pT7H6FX-mtlec and pT7H6FX-mtCTLD is shown in Fig. 18 and Fig. 20, respectively, and the nucleotide sequences of the FX-mtlec and FX-mtCTLD inserts are given as SEQ ID NO:28 and SEQ ID NO:30, respectively. The amino acid sequences of the FX-mtlec and FX-mtCTLD parts of the fusion proteins H6FX- mtlec and H6FX-mtCTLD fusion proteins are shown in Fig. 19 (SEQ ID NO:29) and Fig. 21 (SEQ ID NO:31), respectively.

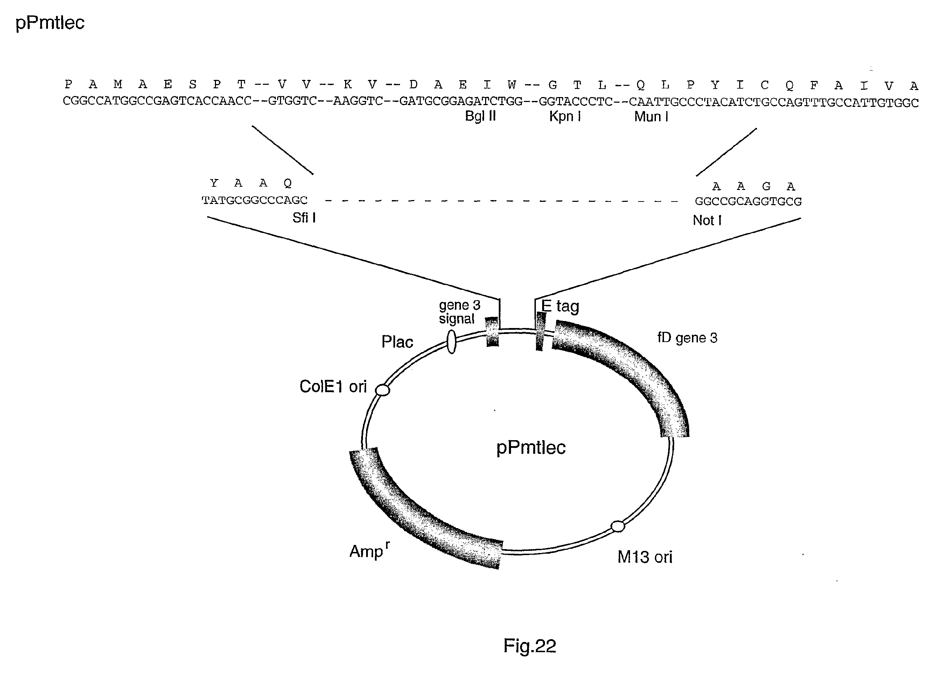

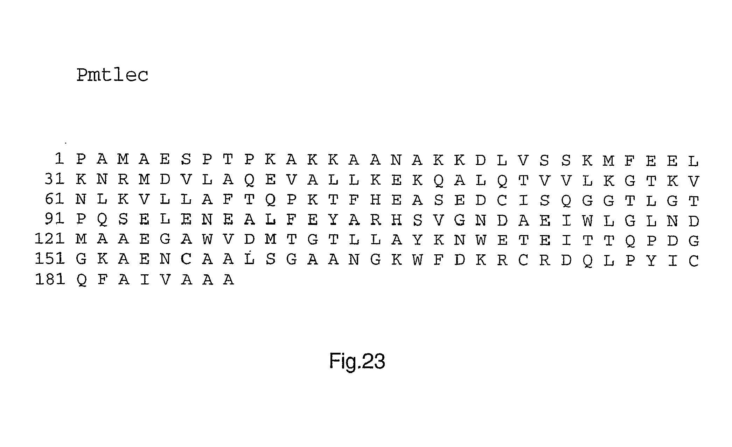

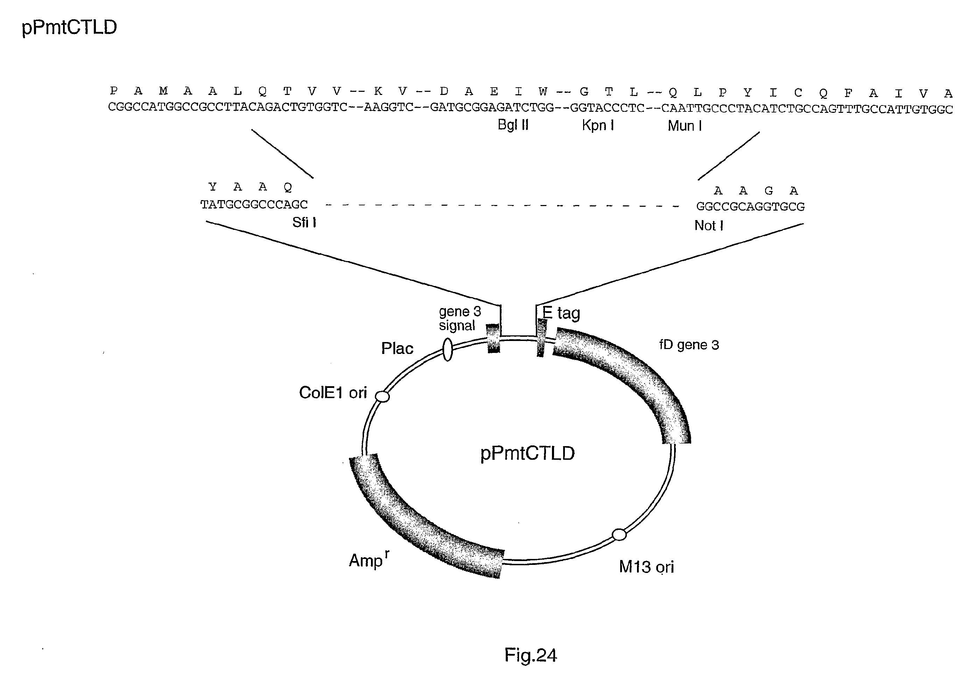

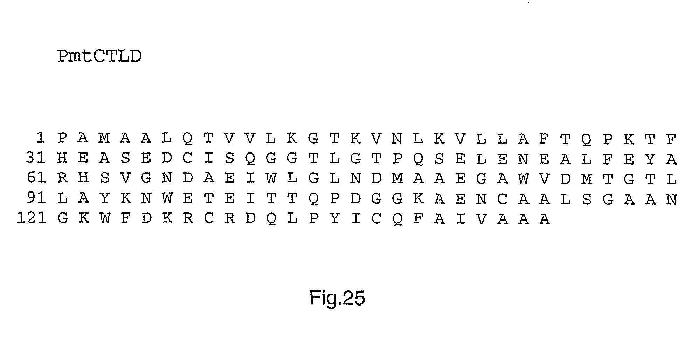

The phagemids pPmtlec and pPmtCTLD may be constructed by ligation of the Sfi I and Not I restricted DNA fragments (amplified from the pUC-mtlec plasmid with the oligonucleotide primer pair 5-CGGCTGAGCGGCCCAGCCGGCCATGGCCGAGTCACCCACTCCCAAGG-3' [SEQ ID NO:32], and 5'-CCTGCGGCCGCCACGATCCCGAACTGG-3' [SEQ ID NO:33] and with the oligonucleotide primers 5'-CGGCTGAGCGGCCCAGCCGGCCATGGCCGCCTTACAGACTGTGGTC-3' [SEQ ID NO:34] and 5'-CCTGCGGCCGCCACGATCCCGAACTGG-3' [SEQ ID NO:33], respectively) into a *Sfi* I and *Not* I precut vector pCANTAB 5E supplied by Amersham Pharmacia Biotech (code no. 27-9401-01) using standard procedures. Outlines of the pPmtlec and pPmtCTLD plasmids are shown in Fig. 22 and Fig. 24, respectively, and the resulting nucleotide sequences of the Pmtlec and PmtCTLD inserts are given as SEQ ID NO:35 and SEQ ID NO:37, repectively. The amino acid sequences encoded by the Pmtlec and PmtCTLD inserts are shown in Fig. 23 (SEQ ID NO: 36) and Fig. 25 (SEQ ID NO: 38), respectively.

### Example 2

### Demonstration of successful display of Phtlec and PhTN3 on phages.

In order to verify that the Phtlec and PhTN3 Gene III fusion proteins can indeed be displayed by the recombinant phage particles, the phagemids pPhtlec and pPhTN3 (described in Example 1) were transformed into *E. coli* TG1 cells and recombinant phages produced upon infection with the helper phage M13KO7. Recombinant phages were isolated by precipitation with poly(ethylene glycol) (PEG 8000) and samples of Phtlec and PhTN3 phage preparations as well as a sample of helper phage were subjected to an ELISA-type sandwich assay, in which wells of a Maxisorb (Nunc) multiwell plate were first incubated with anti-human tetranectin or bovine serum albumin (BSA) and blocked in skimmed milk or skimmed milk/EDTA. Briefly, cultures of pPhtlec and pPhTN3 phagemid transformed TG1 cells were grown at 37 °C in 2xTY-medium supplemented with 2% glucose and 100 mg/L ampicillin until A₆₀₀ reached 0.5. By then the helper phage, M13KO7, was added to a concentration of 5x10⁹ pfu/mL. The cultures were incubated at 37 °C for another 30 min before cells were harvested by centrifugation and resuspended in the same culture volume of 2xTY medium supplemented with 50 mg/L kanamycin and 100 mg/L ampicillin and transferred to a fresh set of flasks and grown for 16 hours at 25 °C. Cells were removed by centrifugation and the phages precipitated from 20 mL culture supernatant by the addition of 6 mL of ice cold 20% PEG 8000, 2.5 M NaCl. After mixing the solution was left on ice for one hour and centrifuged at 4 °C to isolate the precipitated phages. Each phage pellet was resuspended in 1 mL of 10 mM tris-HCl pH 8, 1 mM EDTA (TE) and incubated for 30 min before centrifugation. The phage containing supernatant was transferred to a fresh tube. Along with the preparation of phage samples, the wells of a Maxisorb plate was coated overnight with (70 µL) rabbit anti-human tetranectin (a polyclonal antibody from DAKO A/S, code no. A0371) in a 1:2000 dilution or with (70 µL) BSA (10 mg/mL). Upon coating, the wells were washed three times with PBS (2.68 mM KC1, 1.47 mM KH₂PO₄, 137 mM NaCl, 8.10 mM Na₂HPO₄, pH 7.4) and blocked for one hour at 37 °C with 280 µL of either 3% skimmed milk in PBS, or 3% skimmed milk, 5mM EDTA in PBS. Anti-tetranectin coated and BSA coated wells were then incubated with human Phtlec-, PhTN3-, or helper phage samples for 1 hour and then washed 3 times in PBS buffer supplemented with the appropriate blocking agent. Phages in the wells were detected after incubation with HRP-conjugated anti-phage conjugate (Amersham Pharmacia, code no. 27-9421-01) followed by further washing. HRP activities were then measured in a 96-well ELISA reader using a standard HRP chromogenic substrate assay.

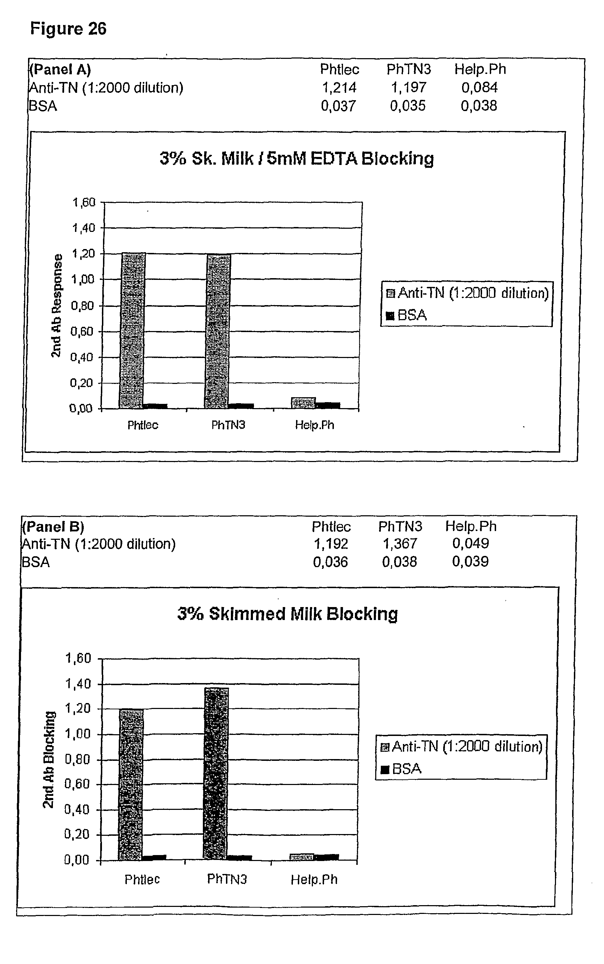

Phtlec and PhTN3 phages produced strong responses (14 times background) in the assay, irrespective of the presence or absence of EDTA in the blocking agent, whereas helper phage produced no response above background readings in either blocking agent. Only low binding to BSA was observed (Fig. 26).

It can therefore be concluded that the human Phtlec and PhTN3 phages both display epitopes that are specifically recognized by the anti-human tetranectin antibody.

### Example 3

### Demonstration of authentic ligand binding properties of Phtlec and PhTN3 displayed on phage

The apo-form of the CTLD domain of human tetranectin binds in a lysine-sensitive manner specifically to the kringle 4 domain of human plasminogen [Graversen et al. (1998)]. Binding of tetranectin to plasminogen can be inhibited by calcium which binds to two sites in the ligand-binding site in the CTLD domain (Kd approx. 0.2 millimolar) or by lysine-analogues like AMCHA (6-amino-cyclohexanoic acid), which bind specifically in the two stronger lysine-binding sites in plasminogen of which one is located in kringle 1 and one is located in kringle 4 (Kd approx. 15 micromolar).

To demonstrate specific AMCHA-sensitive binding of human Phtlec and PhTN3 phages to human plasminogen, an ELISA assay, in outline similar to that employed to demonstrate the presence of displayed Phlec and PhCTLD GIII fusion proteins on the phage particles (cf. Example 2), was devised.

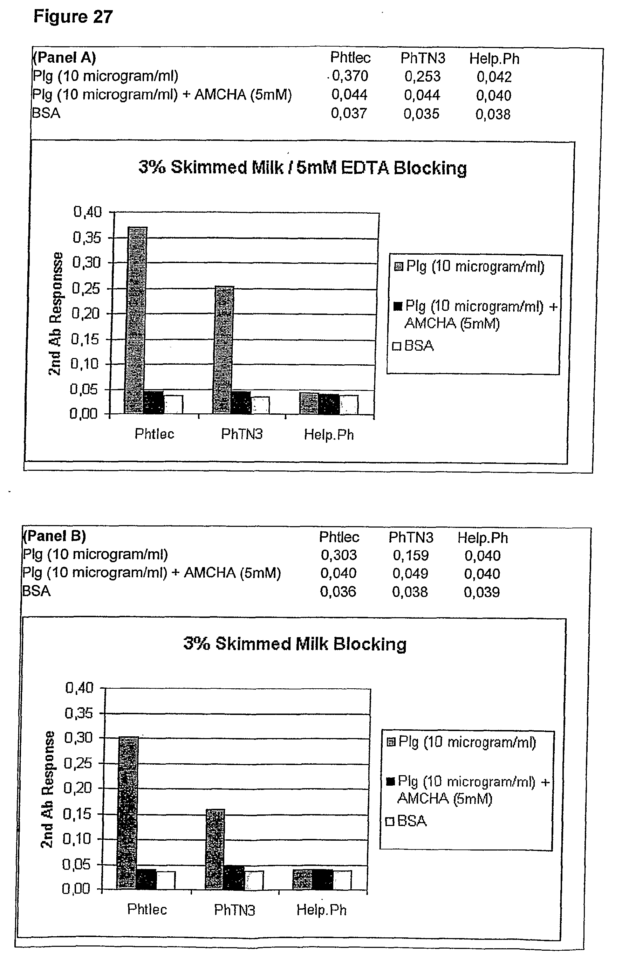

Wells were coated with solutions of human plasminogen (10 µg/mL), with or without addition of 5mM AMCHA. Control wells were coated with BSA. Two identical arrays were established, one was subjected to blocking of excess binding capacity with 3% skimmed milk, and one was blocked using 3% skimmed milk supplemented with 5mM EDTA. Where appropriate, blocking, washing and phage stock solutions were supplemented by 5mM AMCHA. The two arrays of wells were incubated with either Phtlec-, or PhTN3-, or helper phage samples, and after washing the amount of phage bound in each well was measured using the HRP-conjugated antiphage antibody as above. The results are shown in Fig. 27, panels A and B, and can be summarized as follows
(a) In the absence of AMCHA, binding of human Phtlec phages to plasminogen-coated wells generated responses at 8-10 times background levels using either formulation of blocking agent, whereas human PhTN3 phages generated responses at 4 (absence of EDTA) or 7 (presence of EDTA) times background response levels.
(b) In the presence of 5mM AMCHA, binding of human Phtlec- and PhTN3 phages to plasminogen was found to be completely abolished.
(c) Phtlec and PhTN3 phages showed no binding to BSA, and control helper phages showed no binding to any of the immobilized substances.
(d) Specific binding of human Phtlec and PhTN3 phages to a specific ligand at moderate binding strength (about 20 micromolar level) can be detected with high efficiency at virtually no background using a skimmed-milk blocking agent, well-known in the art of combinatorial phage technology as a preferred agent effecting the reduction of non-specific binding.

In conclusion, the results show that the Phtlec and PhTN3 Gene III fusion proteins displayed on the phage particles exhibit plasminogen-binding properties corresponding to those of authentic tetranectin, and that the physical and biochemical properties of Phtlec and PhTN3 phages are compatible with their proposed use as vehicles for the generation of combinatorial libraries from which CTLD derived units with new binding properties can be selected.

### Example 4

### Construction of the phage libraries Phtlec-lb001 and Phtlec-lb002.

All oligonucleotides used in this example were supplied by DNA Technology (Aarhus, Denmark).

The phage library Phtlec-lb001, containing random amino acid residues corresponding to Phtlec (SEQ ID NO: 12) positions 141-146 (loop 3), 150-153 (part of loop 4), and residue 168 (Phe in β4), was constructed by ligation of 20 µg *Kpn*I and *Mun*I restricted pPhtlec phagemid DNA (cf, Example 1) with 10 µg of *Kpn*I and *Mun*I restricted DNA fragment amplified from the oligonucleotide htlec-lib1-tp (SEQ ID NO: 39), where N denotes a mixture of 25% of each of the nucleotides T, C, G, and A, respectively and S denotes a mixture of 50 % of C and G, encoding the appropriately randomized nucleotide sequence and the oligonucleotides htlec-libl-rev (SEQ ID NO: 40) and htleclib1/2-fo (SEQ ID NO: 41) as primers using standard conditions. The ligation mixture was used to transform so-called electrocompetent *E. coli* TG-1 cells by electroporation using standard procedures. After transformation the *E. coli* TG-1 cells were plated on 2xTY-agar plates containing 0.2 mg ampicillin/mL and 2% glucose and incubated over night at 30°C.

The phage library Phtlec-lb002, containing random amino acid residues corresponding to Phtlec (SEQ ID NO: 12) positions 121-123, 125 and 126 (most of loop 1), and residues 150-153 (part of loop 4) was constructed by ligation of 20 µg *Bgl*II and *Mun*I restricted pPhtlec phagemid DNA (cf, EXAMPLE 1) with 15 µg of *Bgl*II and *Mun*I restricted DNA fragment amplified from the pair of oligonucleotides htlec-lib2-tprev (SEQ ID NO: 42) and htlec-lib2-tpfo (SEQ ID NO: 43), where N denotes a mixture of 25% of each of the nucleotides T, C, G, and A, respectively and S denotes a mixture of 50 % of C and G, encoding the appropriately randomized nucleotide sequence and the oligonucleotides htlec-lib2-rev (SEQ ID NO: 44) and htleclib1/2-fo (SEQ ID NO: 41) as primers using standard conditions. The ligation mixture was used to transform so-called electrocompetent E. *coli* TG-1 cells by electroporation using standard procedures. After transformation the E. *coli* TG-1 cells were plated on 2xTY-agar plates containing 0.2 mg ampicillin/mL and 2% glucose and incubated overnight at 30°C.

The titer of the libraries Phtlec-lb001 and -lb002 was determined to 1.4*10⁹ and 3.2*10⁹ clones, respectively. Six clones from each library were grown and phagemid DNA isolated using a standard miniprep procedure, and the nucleotide sequence of the loop-region determined (DNA Technology, Aarhus, Denmark). One clone from each library failed, for technical reasons, to give reliable nucleotide sequence, and one clone from Phtlec-lib001 apparently contained a major deletion. The variation of nucleotide sequences, compared to Phtlec (SEQ ID NO: 12), of the loop-regions of the other nine clones (lb001-1, lb001-2, lb001-3, lb001-4, lb002-1, lb002-2, lb002-3, lb002-4, and lb002-5) is shown in Table 3.

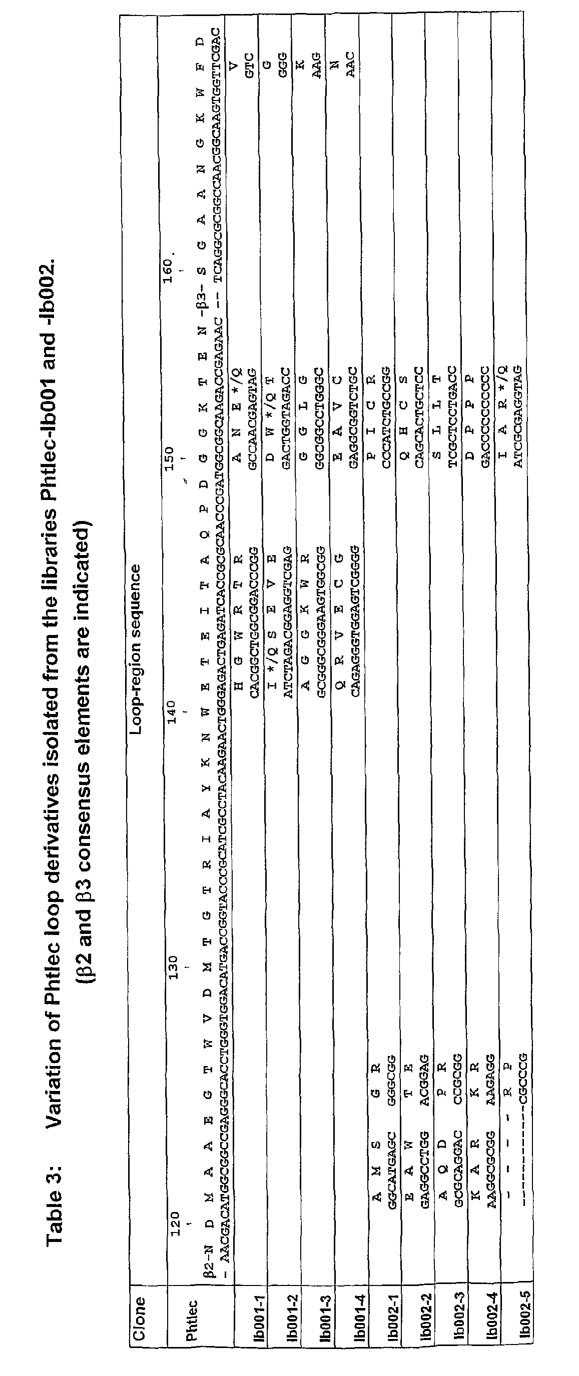

### Example 5

### Construction of the phage library PhtCTLD-lb003

All oligonucleotides used in this example were supplied by DNA Technology (Aarhus, Denmark).

The phage library PhtCTLD-lb003, containing random amino acid residues corresponding to PhtCTLD (SEQ ID NO: 15) positions 77 to 79 and 81 to 82 (loop 1) and 108 to 109 (loop 4) was constructed by ligation of 20 µg *Bgl*II and *Mun*I restricted pPhtCTLD phagemid DNA (cf. Example 1) with 10 µg of a *Bgl*II and *Mun*I restricted DNA fragment population encoding the appropriately randomised loop 1 and 4 regions with or without two and three random residue insertions in loop 1 and with three and four random residue insertions in loop 4. The DNA fragment population was amplified, from six so-called assembly reactions combining each of the three loop 1 DNA fragments with each of the two loop 4 DNA fragments as templates and the oligonucleotides TN-lib3-rev (SEQ ID NO: 45) and loop 3-4-5 tagfo (SEQ ID NO: 46) as primers using standard procedures. Each of the three loop 1 fragments was amplified in a reaction with either the oligonucleotides looplb (SEQ ID NO: 47), loop1c (SEQ ID NO: 48), or loop1d (SEQ ID NO: 49) as template and the oligonucleotides TN-lib3-rev (SEQ ID NO: 45) and TN-KpnI-fo (SEQ ID NO: 50) as primers, and each of the two DNA loop 4 fragments was amplified in a reaction with either the oligonucleotide loop4b (SEQ ID NO: 51) or loop4c (SEQ ID NO: 52) as template and the oligonucleotides loop3-4rev (SEQ ID NO: 53) and loop3-4fo (SEQ ID NO: 54) as primers using standard procedures. In the oligonucleotide sequences N denotes a mixture of 25% of each of the nucleotides T, C, G, and A, respectively and S denotes a mixture of 50 % of C and G, encoding the appropriately randomized nucleotide sequence. The ligation mixture was used to transform so-called electrocompetent *E. coli* TG-1 cells by electroporation using standard procedures. After transformation the E. coli TG-1 cells were plated on 2xTY-agar plates containing 0.2 mg ampicillin/mL and 2% glucose and incubated over night at 30 °C.

The size of the resulting library, PhtCTLD-lb003, was determined to 1.4*10¹⁰ clones. Twenty four clones from the library were grown and phages and phagemid DNA isolated. The nucleotide sequences of the loop-regions were determined (DNA Technology, Aarhus, Denmark) and binding to a polyclonal antibody against tetranectin, anti-TN (DAKO A/S, Denmark), analysed in an ELISA-type assay using HRP conjugated anti-gene VIII (Amersham Pharmacia Biotech) as secondary antibody using standard procedures. Eighteen clones were found to contain correct loop inserts, one clone contained the wild type loop region sequence, one a major deletion, two contained two or more sequences, and two clones contained a frameshift mutation in the region. Thirteen of the 18 clones with correct loop inserts, the wild type clone, and one of the mixed isolates reacted strongly with the polyclonal anti-TN antibody. Three of the 18 correct clones reacted weakly with the antibody, whereas, two of the correct clones, the deletion mutant, one of the mixed, and the two frameshift mutants did not show a signal above background.

### Example 6

### Phage selection by biopanning on anti-TN antibody.

Approximately 10¹¹ phages from the PhtCTLD-lb003 library was used for selection in two rounds on the polyclonal anti-TN antibody by panning in Maxisorb immunotubes (NUNC, Denmark) using standard procedures. Fifteen clones out of 7*10⁷ from the plating after the second selection round were grown and phagemid DNA isolated and the nucleotide sequence determined. All 15 clones were found to encode correct and different loop sequences.

### Example 7

### Model selection of CTLD-phages on plasminogen. I: elution by trypsin digestion after panning.

In order to demonstrate that tetranectin derived CTLD bearing phages can be selected from a population of phages, mixtures of PhtCTLD phages isolated from a E, *coli* TG1 culture transformed with the phagemid pPhtCTLD (cf, EXAMPLE 1) after infection with M13K07 helper phage and phages isolated from a culture transformed with the phagemid pPhtCPB after infection with M13K07 helper phage at ratios of 1:10 and 1:10⁵, respectively were used in a selection experiment using panning in 96-well Maxisorb micro-titerplates (NUNC, Denmark) and with human plasminogen as antigen. The pPhtCPB phagemid was constructed by ligation of the double stranded oligonucleotide (SEQ ID NO: 55) with the appropriate restriction enzyme overhang sequences into KpnI and MunI restricted pPhtCTLD phagemid DNA. The pPhtCBP phages derived upon infection with the helper phages displays only the wild type M13 gene III protein because of the translation termination codons introduced into the CTLD coding region of the resulting pPhtCPB phagemid (SEQ ID NO: 56).

The selection experiments were performed in 96 well micro titer plates using standard procedures. Briefly, in each well 3 µg of human plasminogen in 100 µL PBS (PBS, 0.2 g KCl, 0.2 g KH₂PO₄, 8 g NaCl, 1.44 g Na₂HPO4, 2H₂O, water to 1 L, and adjusted to pH 7.4 with NaOH) or 100 µL PBS (for analysis of non specific binding) was used for over night coating at 4 °C and at 37 °C for one hour. After washing once with PBS, wells were blocked with 400 µL PBS and 3% non fat dried milk for one hour at 37°C. After blocking wells were washed once in PBS and 0.1% Tween 20 and three times with PBS before the addition of phages suspended in 100 µL PBS, 3% non fat dried milk. The phages were allowed to bind at 37 °C for one hour before washing three times with PBS, Tween 20 and three times with PBS. Bound phages were eluted from each well by trypsin digestion in 100 µL (1 mg/mL trypsin in PBS) for 30 min. at room temperature, and used for infection of exponentially growing *E. coli* TG1 cells before plating and titration on 2xTY agar plates containing 2% glucose and 0.1 mg/mL ampicillin.

Initially (round 1), 10¹² PhtCTLD phages (A series), a mixture of 10¹⁰ PhtCTLD phages and 10¹¹ PhtCPB phages (B series), or a mixture of 10⁶ PhtCTLD and 10¹¹ PhtCPB phages (C series) were used. In the following round (round 2) 10¹¹ phages of the output from each series were used. Results from the two rounds of selection are summarised in Table 4.

**Table 4: Selection of mixtures of PhtCTLD and PhtCPB by panning and elution with trypsin.**

| | | Plasminogen | Blank |
|---|---|---|---|
| | | (*10⁵ colonies) | (*10⁵ colonies) |
| Round 1 | A | 113.0 | 19.50 |
| | B | 1.8 | 1.10 |
| | C | 0.1 | 0.30 |
| Round 2 | A | 49 | 0.10 |
| | B | 5.2 | 0.20 |
| | C | 0.3 | 0.04 |

Phagemid DNA from 12 colonies from the second round of plating together with 5 colonies from a plating of the initial phage mixtures was isolated and the nucleotide sequence of the CTLD region determined. From the initial 1/10 mixture (B series) of PhtCTLD/PhtCPB one out of five were identified as the CTLD sequence. From the initial 1/10⁵ mixture (C series) all five sequences were derived from the pPhtCPB phagemid. After round 2 nine of the twelve sequences analysed from the B series and all twelve sequences from the C series were derived from the pPhtCTLD phagemid.

### Example 8

### Model selection of CTLD-phages on plasminogen. II: elution by 0.1 M triethylamine after panning.

In order to demonstrate that tetranectin derived CTLD-bearing phages can be selected from a population of phages, mixtures of PhtCTLD phages isolated from a *E. coli* TG1 culture transformed with the phagemid pPhtCTLD (cf, EXAMPLE 1) after infection with M13K07 helper phage and phages isolated from a culture transformed with the phagemid pPhtCPB (cf, EXAMPLE 6) after infection with M13K07 helper phage at ratios of 1:10² and 1:10⁶, respectively were used in a selection experiment using panning in 96-well Maxisorb micro-titerplates (NUNC, Denmark) and with human plasminogen as antigen using standard procedures.

Briefly, in each well 3 µg of human plasminogen in 100 µL PBS (PBS, 0.2 g KCl, 0.2 g KH₂PO₄, 8 g NaCl, 1.44 g Na₂HPO₄, 2H₂0, water to 1 L, and adjusted to pH 7.4 with NaOH) or 100 µL PBS (for analysis of non specific binding) was used for over night coating at 4 °C and at 37 °C for one hour. After washing once with PBS, wells were blocked with 400 µL PBS and 3% non fat dried milk for one hour at 37 °C. After blocking wells were washed once in PBS and 0.1% Tween 20 and three times with PBS before the addition of phages suspended in 100 µL PBS, 3% non fat dried milk. The phages were allowed to bind at 37 °C for one hour before washing 15 times with PBS, Tween 20, and 15 times with PBS. Bound phages were eluted from each well by 100 µL 0.1 M triethylamine for 10 min at room temperature, and upon neutralisation with 0.5 vol. 1 M Tris-HCl pH 7.4, used for infection of exponentially growing *E. coli* TG1 cells before plating and titration on 2xTY agar plates containing 2% glucose and 0.1 mg/mL ampicillin.

Initially (round 1) 10¹² PhtCTLD phages (A series), a mixture of 10⁹ PhtCTLD phages and 10¹¹ PhtCPB phages (B series), or a mixture of 10⁵ PhtCTLD and 10¹¹ PhtCPB phages (C series) were used. In the following round (round 2) 10¹¹ phages of the output from each series were used. Results from the two rounds of selection are summarised in Table 5.

**Table 5: Selection of mixtures of PhtCTLD and PhtCPB by panning elution with triethylamine.**

| | | Plasminogen | Blank |
|---|---|---|---|
| | | (*10⁴ colonies) | (*10⁴ colonies) |
| Round 1 | A | 18 | 0.02 |
| | B | 0.5 | 0.00 |
| | C | 0.25 | 0.02 |
| Round 2 | A | n.d. | n.d. |
| | B | 5.0 | 0.00 |
| | C | 1.8 | 0.02 |
| Round 3 | A | n.d. | n.d. |
| | B | 11 | 0.00 |
| | C | 6.5 | 0.02 |

| | | | |
|---|---|---|---|
| n.d. = not determined | | | |

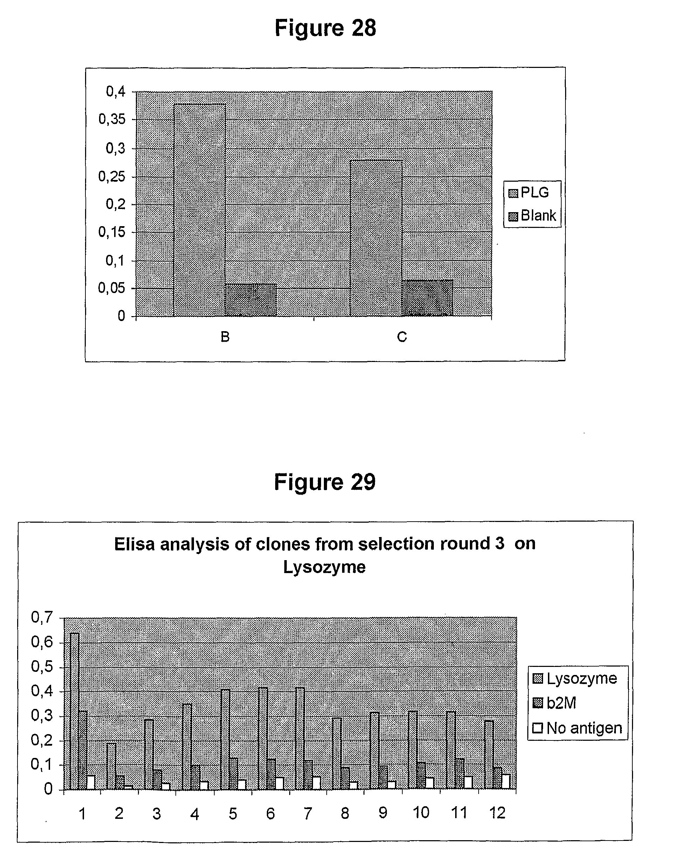

Phage mixtures from the A and the B series from the second round of selection were grown using a standard procedure, and analysed for binding to plasminogen in an ELISA-type assay. Briefly, in each well 3 µg of plasminogen in 100 µL PBS (PBS, 0.2 g KC1, 0.2 g KH2PO4, 8 g NaCl, 1.44 g Na₂HPO₄, 2H₂0, water to 1 L, and adjusted to pH 7.4 with NaOH) or 100 µL PBS (for analysis of non specific binding) was used for over night coating at 4 °C and at 37 °C for one hour. After washing once with PBS, wells were blocked with 400 µL PBS and 3% non fat dried milk for one hour at 370C. After blocking wells were washed once in PBS and 0.1% Tween 20 and three times with PBS before the addition of phages suspended in 100 µL PBS, 3% non fat dried milk. The phage mixtures were allowed to bind at 37 °C for one hour before washing three times with PBS, Tween 20, and three times with PBS. After washing, 50 µL of a 1:5000 dilution of a HRP-conjugated anti-gene VIII antibody (Amersham Pharmacia Biotech) in PBS, 3% non fat dried milk was added to each well and incubated at 37 °C for one hour. After binding of the "secondary" antibody wells were washed three times with PBS, Tween 20, and three times with PBS before the addition of 50 µL of TMB substrate (DAKO-TMB One-Step Substrate System, code: S1600, DAKO, Denmark). Reaction was allowed to proceed for 20 min. before quenching with 0.5 vol. 0.5 M H₂SO₄, and analysis. The result of the ELISA analysis confirmed specific binding to plasminogen of phages in both series (fig. 28).

### Example 9

### Selection of phages from the library Phtlec-lb002 binding to hen egg white lysozyme.

1.2*10¹² phages, approximately 250 times the size of the original library, derived from the Phtlec-lb002 library (cf, EXAMPLE 4) were used in an experimental procedure for the selection of phages binding to hen egg white lysozyme involving sequential rounds of panning using standard procedures.

Briefly, 30 µg of hen egg white lysozyme in 1 mL PBS (PBS, 0.2 g KCl, 0.2 g KH₂PO₄, 8 g NaCl, 1.44 g Na₂HPO₄, 2H₂0, water to 1 L, and adjusted to pH 7.4 with NaOH) or 1 mL PBS (for analysis of non specific binding) was used for over night coating of Maxisorb immunotubes (NUNC, Denmark) at 4 '°C and at 37 °C for one hour. After washing once with PBS, tubes were filled and blocked with PBS and 3% non fat dried milk for one hour at 37°C. After blocking tubes were washed once in PBS, 0.1% Tween 20 and three times with PBS before the addition of phages suspended in 1 mL PBS, 3% non fat dried milk. The phages were allowed to bind at 37 °C for one hour before washing six times with PBS, Tween 20 and six times with PBS. Bound phages were eluted from each well by 1 mL 0.1 M triethylamine for 10 min at room temperature, and upon neutralisation with 1 M Tris-HCl pH 7.4, used for infection of exponentially growing *E. coli* TG1 cells before plating and titration on 2xTY agar plates containing 2% glucose and 0.1 mg/mL ampicillin. In the subsequent rounds of selection approximately 10¹² phages derived from a culture grown from the colonies plated after infection with the phages eluted from the lysozyme coated tube were used in the panning procedure. However, the stringency in binding was increased by increasing the number of washing step after phage panning from six to ten.

The results from the selection procedure is shown in Table 7.

**Table 7: Selection by panning of lysozyme binding phages from Phtlec-lb002 library.**

| | Lysozyme | Blank | Ratio |
|---|---|---|---|
| Round 1 | 2.4*10⁴ | n.a. | n.a. |
| Round 2 | 3.5*10³ | 4.0*10² | 9 |
| Round 3 | 3.2*10³ | 2.5*10² | 1.3*10³ |

| | | | |
|---|---|---|---|
| n.a. = not applicable | | | |

Phages were grown from twelve clones isolated from the third round of selection in order to analyse the specificity of binding using a standard procedure, and analysed for binding to hen egg white lysozyme and human (β₂-microglobulin in an ELISA-type assay. Briefly, in each well 3 µg of hen egg white lysozyme in 100 µL PBS (PBS, 0.2 g KCl, 0.2 g KH₂PO₄, 8 g NaCl, 1.44 g Na₂HPO₄, 2H₂0, water to 1 L, and adjusted to pH 7.4 with NaOH), or 3 µg of human (β₂-microglobulin, or 100 µL PBS (for analysis of non specific binding) was used for over night coating at 4 °C and at 37 °C for one hour. After washing once with PBS, wells were blocked with 400 µL PBS and 3% non fat dried milk for one hour at 37°C. After blocking wells were washed once in PBS and 0.1% Tween 20 and three times with PBS before the addition of phages suspended in 100 µL PBS, 3% non fat dried milk. The phages were allowed to bind at 37 °C for one hour before washing three times with PBS, Tween 20 and three times with PBS. After washing, 50 µL of a 1 to 5000 dilution of a HRP-conjugated anti-gene VIII antibody (Amersham Pharmacia Biotech) in PBS, 3% non fat dried milk was added to each well and incubated at 37 °C for one hour. After binding of the "secondary" antibody wells were washed three times with PBS, Tween 20 and three times with PBS before the addition of 50 µL of TMB substrate (DAKO-TMB One-Step Substrate System, code: S1600, DAKO, Denmark). Reaction was allowed to proceed for 20 min before quenching with 0.5 M H₂SO₄.

Results showing relatively weak but specific binding to lysozyme are summarised in Fig. 29.

### EXAMPLE 10

### Construction of the rat mannose-binding protein CTLD (r-MBP) derived phagemid (pPrMBP) and human lung surfactant protein D CTLD (h-SP-D) derived phagemid (pPhSP-D)

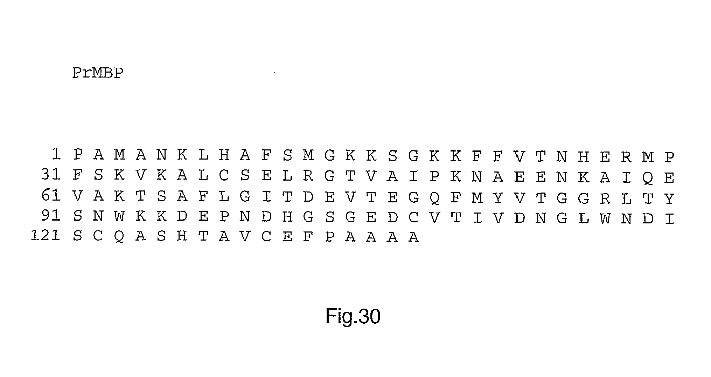

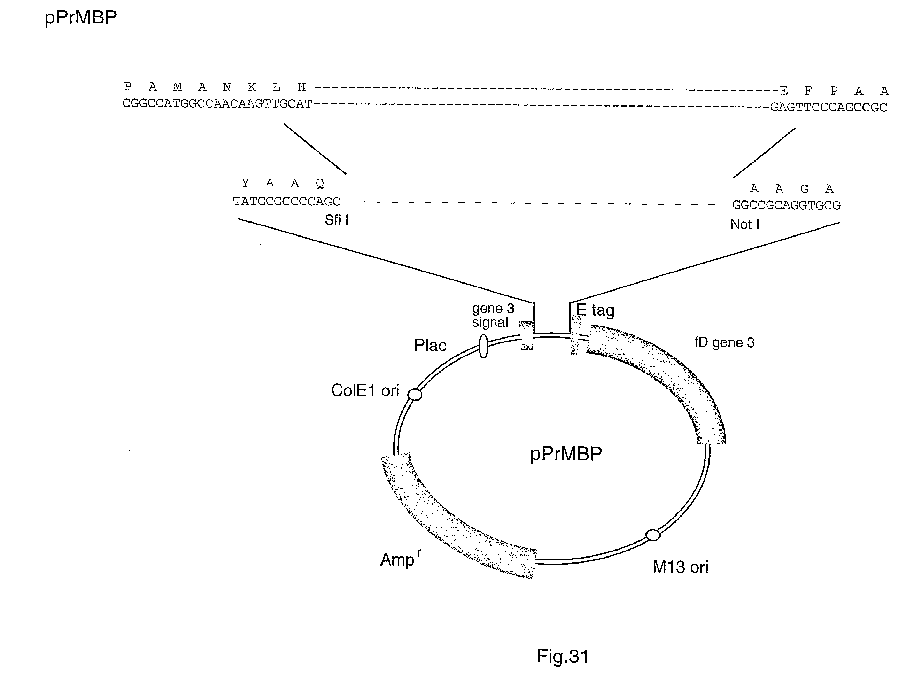

The phagemid, pPrMBP, is constructed by ligation of the *Sfi* I and Not I restricted DNA fragment amplified from cDNA, isolated from rat liver (Drickamer, K., et al., J. Biol. Chem. 1987, 262(6):2582-2589) (with the oligonucleotide primers SfiMBP 5'-CGGCTGAGCGGCCCAGCCGGCCATGGC-CGAGCCAAACAAGTTGCATGCCTTCTCC-3' [SEQ ID NO:62] and NotMBP 5'-GCACTCCTGCGGCCGCGGCTGGGAACTCGCAGAC-3' [SEQ ID NO:63]) into a Sfi I and Not I precut vector, pCANTAB 5E supplied by Amersham Pharmacia Biotech (code no. 27-9401-01) using standard procedures. Outlines of the resulting pPrMBP is shown in Fig. 31 and the nucleotide sequence of PrMBP is given as (SEQ ID NO:58). The amino acid sequence encoded by the PrMBP insert is shown in Fig. 30 (SEQ ID NO:59).

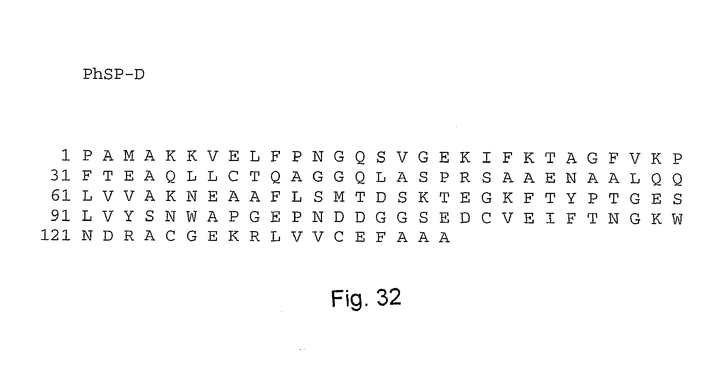

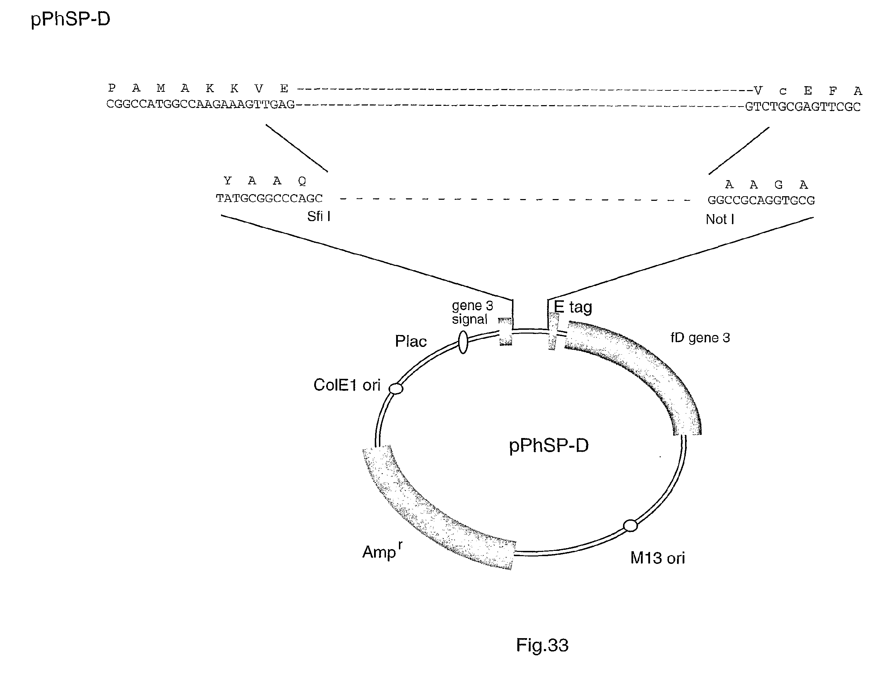

The phagemid,pPhSP-D, is constructed by ligation of the *Sfi* I and *Not* I restricted DNA fragment amplified from cDNA, isolated from human lung (Lu,J., et al., Biochem J. 1992 jun 15; 284:795-802) (with the oligonucleotide primers SfiSP-D 5'-CGGCTGAGCGGCCCAGCCGGCCATGGCCGAGCCAAAGAAAGTTGA-GCTCTTCCC-3' [SEQ ID NO:64] and NotSP-D 5'-GCACTCCTGCGGC-CGCGAACTCGCAGACCACAAGAC-3' [SEQ ID NO:65]) into a *Sfi* I and *Not* I precut vector, pCANTAB 5E supplied by Amersham Pharmacia Biotech (code no. 27-9401-01) using standard procedures. Outlines of the resulting pPhSP-D is shown in Fig. 33 and the nucleotide sequence of PhSP-D, is given as (SEQ ID NO:60). The amino acid sequences encoded by the PhSP-D insert is shown in Fig 32 (SEQ ID NO:61).

### Example 11

### Construction of the phage library PrMBP-lb001

The phage library PrMBP-lb001, containing random amino acid residues corresponding to PrMBP CTLD (SEQ ID NO:59) positions 71 to 73 or 70 to 76 (loop 1) and 97 to 101 or 100 to 101 (loop 4) is constructed by ligation of 20 µg SfiI and *Not*I restricted pPrMBP phagemid DNA (cf. Example 10) with 10 µg of a SfiI and NotI restricted DNA fragment population encoding the appropriately randomised loop 1 and 4 regions. The DNA fragment population is amplified, from nine assembly reactions combining each of the three loop 1 DNA fragments with each of the three loop 4 DNA fragments as templates and the oligonucleotides Sfi-tag 5'-CGGCTGAGCGGCCCA-GC-3' (SEQ ID NO:74) and Not-tag 5'-GCACTCCTGCGGCCGCG-3' (SEQ ID NO:75) as primers using standard procedures. Each of the three loop 1 fragments is amplified in a primary PCR reaction with pPrMBP phagmid DNA (cf. Example 10) as template and the oligonucleotides MBPloop1a fo (SEQ ID NO:66), MBPloop1b fo (SEQ ID NO:67)or MBPloop1c fo (SEQ ID NO:68) and SfiMBP (SEQ ID NO:62) as primers, and further amplified in a secondary PCR reaction using Sfi-tag (SEQ ID NO:74) and MBPloop1-tag fo (SEQ ID NO:69). Each of the three DNA loop 4 fragments is amplified in a primary PCR reaction with pPrMBP phagemid DNA (cf. Example 10) as template and the oligonucleotides MBPloop4a rev (SEQ ID NO:71), MBPloop4b rev (SEQ ID NO:72) or MBPloop4c rev (SEQ ID NO:73) and NotMBP (SEQ ID NO:63) as primers using standard procedures and further amplified in a secondary PCR reaction using MBPloop4-tag rev (SEQ ID NO:70) and Not-tag (SEQ ID NO:63). In the oligonucleotide sequences N denotes a mixture of 25% of each of the nucleotides T, C, G, and A, respectively, and S denotes a mixture of 50 % of C and G, encoding the appropriately randomized nucleotide sequence. The ligation mixture is used to transform so-called electrocompetent *E. coli* TG-1 cells by electroporation using standard procedures. After transformation the *E. coli* TG-1 cells are plated on 2xTY-agar plates containing 0.2 mg ampicillin/mL and 2% glucose and incubated over night at 30 °C.

### Example 12

### Construction of the phage library PhSP-D-lb001

The phage library PhSP-D-lb001, containing random amino acid residues corresponding to PhSP-D CTLD insert (SEQ ID NO:61) positions 74 to 76 or 73 to 79 (loop 1) and 100 to 104 or 103 to 104 (loop 4) is constructed by ligation of 20 µg SfiI and NotI restricted pPhSP-D phagemid DNA (cf. Example 10) with 10 µg of a SfiI and NotI restricted DNA fragment population encoding the appropriately randomised loop 1 and 4 regions. The DNA fragment population is amplified, from nine assembly reactions combining each of the three loop 1 DNA fragments with each of the three loop 4 DNA fragments as templates and the oligonucleotides Sfi-tag 5'-CGGCTGAGCGGCCCAGC-3' (SEQ ID NO:74 ) and Not-tag 5'-GCACTCCTGCGGCCGCG-3' (SEQ ID NO:75) as primers using standard procedures. Each of the three loop 1 fragments is amplified in a primary PCR reaction with pPhSP-D phagemid DNA (cf. Example 10) as template and the oligonucleotides Sp-dloop1a fo (SEQ ID NO:76), Sp-dloop1b fo (SEQ ID NO:77)or Sp-dloop1c fo (SEQ ID NO:78) and SfiSP-D (SEQ ID NO:64) as primers, and further amplified in a PCR reaction using Sfi-tag (SEQ ID NO:74) and Sp-dloopl-tag fo (SEQ ID NO:79) as primers. Each of the three DNA loop 4 fragments is amplified in a primary PCR reaction with pPhSP-D phagemid DNA (cf. Example 10) as template and the oligonucleotides Sp-dloop4a rev (SEQ ID NO:81), Sp-dloop4b rev (SEQ ID NO:82) or Sp-dloop4c rev (SEQ ID NO:83) and NotSP-D (SEQ ID NO:65) as primers using standard procedures and further amplified in a PCR reaction using Sp-dloop4-tag rev (SEQ ID NO:80) and Not-tag (SEQ ID NO:75) as primers. In the oligonucleotide sequences N denotes a mixture of 25% of each of the nucleotides T, C, G, and A, respectively, and S denotes a mixture of 50 % of C and G, encoding the appropriately randomized nucleotide sequence. The ligation mixture is used to transform so-called electrocompetent *E. coli* TG-1 cells by electroporation using standard procedures. After transformation the *E. coli* TG-1 cells are plated on 2xTY-agar plates containing 0.2 mg ampicillin/mL and 2% glucose and incubated over night at 30 °C.

### Example 13

### Construction of the phage library PhtCTLD-lb004

All oligonucleotides used in this example were supplied by DNA Technology (Aarhus, Denmark).

The phage library PhtCTLD-lb004, containing random amino acid residues corresponding to PhtCTLD (SEQ ID NO:15) positions 97 to 102 or 98 to 101(loop 3) and positions 116 to 122 or 118 to 120 (loop 5) was constructed by ligation of 20 µg *Kpn*I and MunI restricted pPhtCTLD phagemid DNA (cf. Example 1) with 10 µg of a *Kpn*I and *Mun*I restricted DNA fragment population encoding the randomised loop 3 and 5 regions. The DNA fragment population was amplified from nine primary PCR reactions combining each of the three loop 3 DNA fragments with each of the three loop 5 DNA fragments. The fragments was amplified with either of the oligonucleotides loop3a (SEQ ID NO:84), loop3b (SEQ ID NO: 85), or loop3c (SEQ ID NO:86) as template and loop5a (SEQ ID NO:87), loop5b(SEQ ID NO:88)or loop5c(SEQ ID NO:89) and loop3-4rev(SEQ ID NO:91) as primers. The DNA fragments were further amplified in PCR reactions, using the primary PCR product as template and the oligonucleotide loop3-4rev (SEQ ID NO:91) and loop3-4-5tag fo (SEQ ID NO:90) as primers. All PCR reactions were performed using standard procedures.

In the oligonucleotide sequences N denotes a mixture of 25% of each of the nucleotides T, C, G, and A, respectively and S denotes a mixture of 50 % of C and G, encoding the appropriately randomised nucleotide sequence. The ligation mixture was used to transform so-called electrocompetent E. coli TG-1 cells by electroporation using standard procedures. After transformation the E. coli TG-1 cells were plated on 2xTY-agar plates containing 0.2 mg ampicillin/mL and 2% glucose and incubated over night at 30 °C.

The size of the resulting library, PhtCTLD-lb004, was determined to 7*109 clones. Sixteen clones from the library were picked and phagemid DNA isolated. The nucleotide sequence of the loop-regions were determined (DNA Technology, Aarhus, Denmark). Thirteen clones were found to contain correct loop inserts and three clones contained a frameshift mutation in the region.

### Example 14

### Selection of Phtlec-phages and PhtCTLD-phages binding to the blood group A sugar moiety immobilised on human serum albumin

Phages grown from glycerol stocks of the libraries Phtlec-lb001 and Phtlec-lb002 (cf. Example 4) and phages grown from a glycerol stock of the library PhtCTLD-lb003 (cf. Example 5), using a standard procedure, were used in an experiment designed for the selection of Phtlec- and PhtCTLD derived phages with specific affinity to the blood group A sugar moiety immobilized on human serum albumin, A-HA, by panning in 96-well Maxisorb micro-titerplates (NUNC, Denmark) using standard procedures.

Initially, the phage supernatants were precipitated with 0.3 vol. of a solution of 20% polyethylene glycol 6000 (PEG) and 2.5 M NaCl, and the pellets re-suspended in TE-buffer (10 mM Tris-HCl pH 8, 1 mM EDTA). After titration on E. coli TG-1 cells, phages derived from Phtlec-lb001 and - lb002 were mixed (#1) in a 1:1 ratio and adjusted to 5*1012 pfu/mL in 2*TY medium, and phages grown from the PhtCTLD-lb003 library (#4) were adjusted to 2.5*1012 pfu/mL in 2*TY medium.

One microgram of the "antigen", human blood group A trisaccharide immobilised on human serum albumin, A-HA, (Glycorex AB, Lund, Sweden) in 100 µL PBS (PBS, 0.2 g KCl, 0.2 g KH₂PO₄, 8 g NaCl, 1.44 g Na₂HPO₄, 2H₂0, water to 1 L, and adjusted to pH 7.4 with NaOH), in each of three wells, was coated over night at 4 °C and at room temperature for one hour, before the first round of panning. After washing once with PBS, wells were blocked with 300 µL BS and 3% non fat dried milk for one hour at room temperature. After blocking wells were washed once in PBS and 0.1% Tween 20 and three times with PBS before the addition of a mixture of 50 µL of the phage suspension and 50 µL PBS, 6% non fat dried milk. The phages were allowed to bind at room temperature for two hours before washing eight times with PBS, Tween 20, and eight times with PBS. Bound phages were eluted from each well by trypsin digestion in 100 µL (1 mg/mL trypsin in PBS) for 30 min. at room temperature, and used for infection of exponentially growing *E. coli* TG1 cells before plating and titration on 2xTY agar plates containing 2% glucose and 0.1 mg/mL ampicillin.

In the second round of selection, 150 µL of crude phage supernatant, grown from the first round output colonies, was mixed with 150 µL PBS, 6% non fat dried milk, and used for panning distributing 100 µL of the mixture in each of three A-HA coated wells, as previously described. Stringency in binding was increased by increasing the number of washing steps from 16 to 32. 300 µL of phage mixture was also used for panning in three wells, which had received no antigen as control.

In the third round of selection, 150 µL of crude phage supernatant, grown from the second round output colonies, was mixed with 150 µL PBS, 6% non fat dried milk, and used for panning distributing 100 µL of the mixture in each of three A-HA coated wells, as previously described. The number of washing steps was again 32. 300 µL of phage mixture was also used for panning in three wells, which had received no antigen as control.

The results from the selection procedure are summarised in Table 8

**Table 8. Selection of Phtlec phages (#1) and PhtCTLD phages (#4) binding to A-HA by panning and elution with trypsin digestion.**

| | | A-HA | Blank | Ratio |
|---|---|---|---|---|
| Round 1 | #1 | 0.8*10³ | n.a. | n.a. |
| | #4 | 1.1*10³ | n.a. | n.a. |
| Round 2 | #1 | 1.0*10³ | 0.5*10² | 20 |
| | #4 | 1.3*10³ | 0.5*10² | 26 |
| Round 3 | #1 | 8.0*10⁴ | 0.5*10² | 1600 |
| | #4 | 9.0*10⁵ | 0.5*10² | 18000 |

| | | | | |
|---|---|---|---|---|
| n.a. not applicable. | | | | |

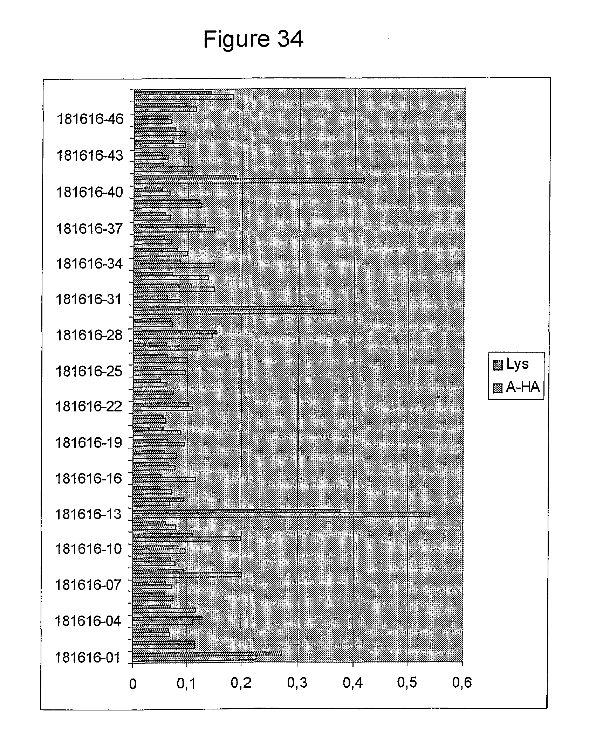

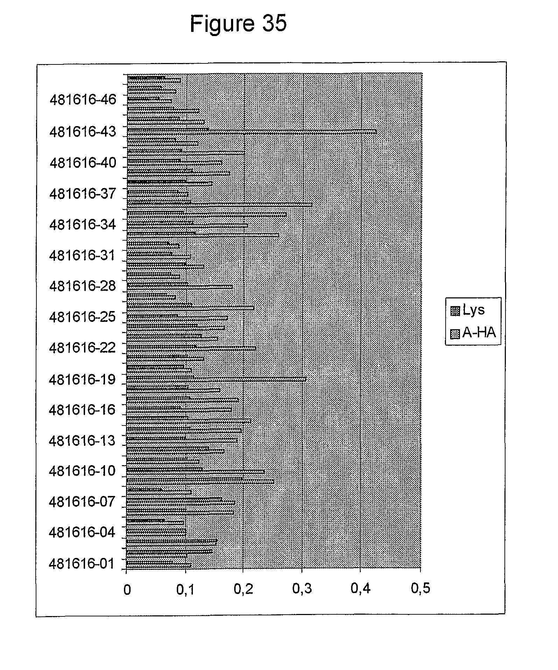

48 clones from each of the #1 and #4 series were picked and grown in a 96 well microtiter tray and phages produced by infection with M13K07 helper phage using a standard procedure. Phages from the 96 phage supernatants were analysed for binding to the A-HA antigen and for non-specific binding to hen egg white lysozyme using an ELISA-type assay. Briefly, in each well 1 µg of A-HA in 100 µL PBS (PBS, 0.2 g KCl, 0.2 g KH₂PO₄, 8 g NaCl, 1.44 g Na₂HPO₄, 2H₂0, water to 1 L, and adjusted to pH 7.4 with NaOH) or 1 µg of hen egg white lysozyme in 100 µL PBS (for analysis of non specific binding) was used for over night coating at 4 °C and at room temperature for one hour. After washing once with PBS, wells were blocked with 300 µL PBS and 3% non fat dried milk for one hour at room temperature. After blocking wells were washed once in PBS and 0.1% Tween 20 and three times with PBS before the addition of 50 µL phage supernatant in 50 µL PBS, 6% non fat dried milk. The phage mixtures were allowed to bind at room temperature for two hours before washing three times with PBS, Tween 20, and three times with PBS. After washing, 50 µL of a 1:5000 dilution of a HRP-conjugated anti-gene VIII antibody (Amersham Pharmacia Biotech) in PBS, 3% non fat dried milk, was added to each well and incubated at room temperature for one hour. After binding of the "secondary" antibody wells were washed three times with PBS, Tween 20, and three times with PBS before the addition of 50 µL of TMB substrate (DAKO-TMB One-Step Substrate System, DAKO, Denmark). Reaction was allowed to proceed for 20 min. before quenching with 0.5 M H₂SO₄, and analysis. The result of the ELISA analysis showed "hits" in terms of specific binding to A-HA of phages in both series (fig. 34 and 35), as judged by a signal ratio between signal on A-HA to signal on lysozyme at or above 1.5, and with a signal above background.

From the #1 series 13 hits were identified and 28 hits were identified from the #4 series.

### REFERENCES

Aspberg, A., Miura, R., Bourdoulous, S., Shimonaka, M., Heinegård, D., Schachner, M., Ruoslahti, E., and Yamaguchi, Y. (1997). "The C-type lectin domains of lecticans, a family of aggregating chondroitin sulfate proteoglycans, bind tenascin-R by protein-protein interactions independent of carbohydrate moiety". Proc. Natl. Acad. Sci. (USA) 94: 10116-10121
Bass, S., Greene, R., and Wells, J.A. (1990). "Hormone phage: an enrichment method for variant proteins with altered binding properties". Proteins 8: 309-314
Benhar, I., Azriel, R., Nahary, L., Shaky, S., Berdichevsky, Y., Tamarkin, A., and Wels, W. (2000). "Highly efficient selection of phage antibodies mediated by display of antigen as Lpp-OmpA' fusions on live bacteria". J. Mol. Biol. 301: 893-904
Berglund, L. and Petersen, T.E. (1992). "The gene structure of tetranectin, a plasminogen binding protein". FEBS Letters 309: 15-19
Bertrand, J.A., Pignol, D., Bernard, J-P., Verdier, J-M., Dagorn, J-C., and Fontecilla-Camps, J.C. (1996). "Crystal structure of human lithostathine, the pancreatic inhibitor of stone formation". EMBO J. 15: 2678-2684
Bettler, B., Texido, G., Raggini, S., Ruegg, D., and Hofstetter, H. (1992). "Immunoglobulin E-binding site in Fc epsilon receptor (Fc epsilon RII/CD23) identified by homolog-scanning mutagenesis". J. Biol. Chem. 267: 185-191
Blanck, O., Iobst, S.T., Gabel, C., and Drickamer, K. (1996)."Introduction of selectin-like binding specificity into a homologous mannose-binding protein". J. Biol. Chem. 271: 7289-7292
Boder, E.T. and Wittrup, K.D. (1997). "Yeast surface display for screening combinatorial polypeptide libraries". Nature Biotech. 15: 553-557
Burrows L, Iobst ST, Drickamer K. (1997) "Selective binding of N-acetylglucosamine to the chicken hepatic lectin". Biochem J. 324:673-680
Chiba, H., Sano, H., Saitoh, M., Sohma, H., Voelker, D.R., Akino, T., and Kuroki, Y. (1999). "Introduction of mannose binding protein-type phosphatidylinositol recognition into pulmonary surfactant protein A". Biochemistry 38: 7321-7331
Christensen, J.H., Hansen, P.K., Lillelund, O., and Th⌀gersen, H.C. (1991). "Sequence-specific binding of the N-terminal three-finger fragment of Xenopus transcription factor IIIA to the internal control region of a 5S RNA gene". FEBS Letters 281: 181-184
Cyr, J.L. and Hudspeth, A.J. (2000). "A library of bacteriophage-displayed antibody fragments directed against proteins of the inner ear". Proc. Natl. Acad. Sci (USA) 97: 2276-2281
Drickamer, K. (1992). "Engineering galactose-binding activity into a C-type mannose-binding protein". Nature 360: 183-186
Drickamer, K. and Taylor, M.E. (1993). "Biology of animal lectins". Annu. Rev. Cell Biol. 9: 237-264
Drickamer, K. (1999). "C-type lectin-like domains". Curr. Opinion Struc. Biol. 9: 585-590
Dunn, I.S. (1996). "Phage display of proteins". Curr. Opinion Biotech. 7: 547-553
Erbe, D.V., Lasky, L.A., and Presta, L.G. "Selection variants". US Patent No. 5593882
Ernst, W.J., Spenger, A., Toellner, L., Katinger, H.,Grabherr, R.M. (2000). "Expanding baculovirus surface display. Modification of the native coat protein gp64 of Autographa californica NPV". Eur. J. Biochem. 267: 4033-4039
Ewart, K.V., Li, Z., Yang, D.S.C., Fletcher, G.L., and Hew, C.L. (1998). "The ice-binding site of Atlantic herring antifreeze protein corresponds to the carbohydrate-binding site of C-type lectins". Biochemistry 37: 4080-4085
Feinberg, H., Park-Snyder, S., Kolatkar, A.R., Heise, C.T., Taylor, M.E., and Weis, W.I. (2000). "Structure of a C-type carbohydrate recognition domain from the macrophage mannose receptor". J. Biol. Chem. 275: 21539-21548
Fujii, I., Fukuyama, S., Iwabuchi, Y., and Tanimura, R. (1998). "Evolving catalytic antibodies in a phage-displayed combinatorial library". Nature Biotech. 16: 463-467
Gates, C.M., Stemmer, W.P.C., Kaptein, R., and Schatz, P.J. (1996). "Affinity selective isolation of ligands from peptide libraries through display on a lac repressor "headpiece dimer". J. Mol. Biol. 255: 373-386
Graversen, J.H., Lorentsen, R.H., Jacobsen, C., Moestrup, S.K., Sigurskjold, B.W., Th⌀gersen, H.C., and Etzerodt, M. (1998). "The plasminogen binding site of the C-type lectin tetranectin is located in the carbohydrate recognition domain, and binding is sensitive to both calcium and lysine". J. Biol. Chem. 273:29241-29246
Graversen, J.H., Jacobsen, C., Sigurskjold, B.W., Lorentsen, R.H., Moestrup, S.K., Th⌀gersen, H.C., and Etzerodt, M. (2000). "Mutational Analysis of Affinity and Selectivity of Kringle-Tetranectin Interaction. Grafting novel kringle affinity onto the tetranectin lectin scaffold". J. Biol. Chem. 275: 37390-37396
Griffiths, A.D. and Duncan, A.R. (1998). "Strategies for selection of antibodies by phage display". Curr. Opinion Biotech. 9: 102-108
Holtet, T.L., Graversen, J.H., Clemmensen, I., Th⌀gersen, H.C., and Etzerodt, M. (1997). "Tetranectin, a trimeric plasminogen-binding C-type lectin". Prot. Sci. 6: 1511-1515
Honma, T., Kuroki, Y., Tzunezawa, W., Ogasawara, Y., Sohma, H., Voelker, D.R., and Akino, T. (1997). "The mannose-binding protein A region of glutamic acid185-alanine221 can functionally replace the surfactant protein A region of glutamic acid195-phenylalanine228 without loss of interaction with lipids and alveolar type II cells". Biochemistry 36: 7176-7184
Huang, W., Zhang, Z., and Palzkill, T. (2000). "Design of potent beta-lactamase inhibitors by phage display of beta-lactamase inhibitory protein". J. Biol. Chem. 275: 14964-14968
Hufton, S.E., van Neer, N., van den Beuken, T., Desmet, J., Sablon, E., and Hoogenboom, H.R. (2000). "Development and application of cytotoxic T lymphocyte-associated antigen 4 as a protein scaffold for the generation of novel binding ligands". FEBS Letters 475: 225-231
Håkansson, K., Lim, N.K., Hoppe, H-J., and Reid, K.B.M. (1999). "Crystal structure of the trimeric alpha-helical coiled-coil and the three lectin domains of human lung surfactant protein D". Structure Folding and Design 7: 255-264
Iobst, S.T., Wormald, M.R., Weis, W.I., Dwek, R.A., and Drickamer, K. (1994). "Binding of sugar ligands to Ca(2+)-dependent animal lectins. I. Analysis of mannose binding by site-directed mutagenesis and NMR". J. Biol. Chem. 269: 15505-15511
Iobst, S.T. and Drickamer, K. (1994). "Binding of sugar ligands to Ca(2+)-dependent animal lectins. II. Generation of high-affinity galactose binding by site-directed mutagenesis". J. Biol, Chem. 269: 15512-15519
Iobst, S.T. and Drickamer, K. (1996). "Selective sugar binding to the carbohydrate recognition domains of the rat hepatic and macrophage asialoglycoprotein receptors". J. Biol. Chem. 271: 6686-6693
Jaquinod, M., Holtet, T. L., Etzerodt, M., Clemmensen, I., Thogersen, H. C., and Roepstorff, P. (1999). "Mass Spectrometric Characterisation of Post-Translational Modification and Genetic Variation in Human Tetranectin". Biol. Chem. 380: 1307-1314
Kastrup, J.S., Nielsen, B.B., Rasmussen, H., Holtet, T.L., Graversen, J.H., Etzerodt, M., Th⌀gersen, H.C., and Larsen, I.K. (1998). "Structure of the C-type lectin carbohydrate recognition domain of human tetranectin". Acta. Cryst. D 54: 757-766
Kogan, T.P., Revelle, B.M., Tapp, S., Scott, D., and Beck, P.J. (1995). "A single amino acid residue can determine the ligand specificity of E-selectin". J. Biol. Chem. 270: 14047-14055
Kolatkar, A.R., Leung, A.K., Isecke, R., Brossmer, R., Drickamer, K., and Weis, W.I. (1998). "Mechanism of N-acetylgalactosamine binding to a C-type animal lectin carbohydrate-recognition domain". J. Biol. Chem. 273: 19502-19508
Lorentsen, R.H., Graversen, J.H., Caterer, N.R., Th⌀gersen, H.C., and Etzerodt, M. (2000). "The heparin-binding site in tetranectin is located in the N-terminal region and binding does not involve the carbohydrate recognition domain". Biochem. J. 347: 83-87
Marks, J.D., Hoogenboom, H.R., Griffiths, A.D., and Winter, G. (1992). "Molecular evolution of proteins on filamentous phage. Mimicking the strategy of the immune system". J. Biol. Chem. 267: 16007-16010
Mann K, Weiss IM, Andre S, Gabius HJ, Fritz M. (2000). "The amino-acid sequence of the abalone (Haliotis laevigata) nacre protein perlucin. Detection of a functional C-type lectin domain with galactose/mannose specificity". Eur. J. Biochem. 267: 5257-5264
McCafferty, J., Jackson, R.H., and Chiswell, D.J. (1991). "Phage-enzymes: expression and affinity chromatography of functional alkaline phosphatase on the surface of bacteriophage". Prot. Eng. 4: 955-961
McCormack, F.X., Kuroki, Y., Stewart, J.J., Mason, R.J., and Voelker, D.R. (1994). "Surfactant protein A amino acids Glu195 and Arg197 are essential for receptor binding, phospholipid aggregation, regulation of secretion, and the facilitated uptake of phospholipid by type II cells". J. Biol. Chem. 269: 29801-29807
McCormack, F.X., Festa, A.L., Andrews, R.P., Linke, M., and Walzer, P.D. (1997). "The carbohydrate recognition domain of surfactant protein A mediates binding to the major surface glycoprotein of Pneumocystis carinii". Biochemistry 36: 8092-8099
Meier, M., Bider, M.D., Malashkevich, V.N., Spiess, M., and Burkhard, P. (2000). "Crystal structure of the carbohydrate recognition domain of the H1 subunit of the asialoglycoprotein receptor". J. Mol. Biol. 300: 857-865
Mikawa, Y.G., Maruyama, I.N., and Brenner, S. (1996). "Surface display of proteins on bacteriophage lambda heads". J. Mol. Biol. 262: 21-30
Mio H, Kagami N, Yokokawa S, Kawai H, Nakagawa S, Takeuchi K, Sekine S, Hiraoka A. (1998). "Isolation and characterization of a CDNA for human mouse, and rat full-length stem cell growth factor, a new member of C-type lectin super-family". Biochem. Biophys. Res. Commun. 249: 124-130
Mizuno, H., Fujimoto, Z., Koizumi, M., Kano, H., Atoda, H., and Morita, T. (1997). "Structure of coagulation factors IX/X-binding protein, a heterodimer of C-type lectin domains". Nat. Struc. Biol. 4: 438-441
Ng, K.K., Park-Snyder, S., and Weis, W.I. (1998a). "Ca2+-dependent structural changes in C-type mannose-binding proteins". Biochemistry 37: 17965-17976
Ng, K.K. and Weis, W.I. (1998b). "Coupling of prolyl peptide bond isomerization and Ca2+ binding in a C-type mannose-binding protein". Biochemistry 37: 17977-17989
Nielsen, B.B., Kastrup, J.S., Rasmussen, H., Holtet, T.L., Graversen, J.H., Etzerodt, M., Th⌀gersen, H.C., and Larsen, I.K. (1997). "Crystal structure of tetranectin, a trimeric plasminogen-binding protein with an alpha-helical coiled coil". FEBS Letters 412: 388-396
Nissim A., Hoogenboom, H.R., Tomlinson, I.M., Flynn, G., Midgley, C., Lane, D., and Winter, G. (1994). "Antibody fragments from a 'single pot' phage display library as immunochemical reagents". EMBO J. 13: 692-698
Ogasawara, Y. and Voelker, D.R. (1995). "Altered carbohydrate recognition specificity engineered into surfactant protein D reveals different binding mechanisms for phosphatidylinositol and glucosylceramide". J. Biol. Chem. 270: 14725-14732
Ohtani, K., Suzuki, Y., Eda, S., Takao, K., Kase, T., Yamazaki, H., Shimada, T., Keshi, H., Sakai, Y., Fukuoh, A., Sakamoto, T., and Wakamiya, N. (1999). "Molecular cloning of a novel human collectin from liver (CL-L1)". J. Biol. Chem. 274: 13681-13689
Pattanajitvilai, S., Kuroki, Y., Tsunezawa, W., McCormack, F.X., and Voelker, D.R. (1998). "Mutational analysis of Arg197 of rat surfactant protein A. His197 creates specific lipid uptake defects". J. Biol. Chem. 273: 5702-5707
Poget, S.F., Legge, G.B., Proctor, M.R., Butler, P.J., Bycroft, M., and Williams, R.L. (1999). "The structure of a tunicate C-type lectin from Polyandrocarpa misakiensis complexed with D-galactose". J. Mol. Biol. 290: 867-879
Revelle, B.M., Scott, D., Kogan, T.P., Zheng, J., and Beck, P.J. (1996). "Structure-function analysis of P-selectinsialyl LewisX binding interactions. Mutagenic alteration of ligand binding specificity". J. Biol. Chem. 271: 4289-4297
Sano, H., Kuroki, Y., Honma, T., Ogasawara, Y., Sohma, H., Voelker, D.R., and Akino, T. (1998). "Analysis of chimeric proteins identifies the regions in the carbohydrate recognition domains of rat lung collectins that are essential for interactions with phospholipids, glycolipids, and alveolar type II cells". J. Biol. Chem. 273: 4783-4789
Schaffitzel, C., Hanes, J., Jermutus, L., and Plücktun, A. (1999). "Ribosome display: an in vitro method for selection and evolution of antibodies from libraries". J. Immunol. Methods 231: 119-135
Sheriff, S., Chang, C.Y., and Ezekowitz, R.A. (1994). "Human mannose-binding protein carbohydrate recognition domain trimerizes through a triple alpha-helical coiled-coil". Nat. Struc. Biol. 1: 789-794
S⌀rensen, C.B., Berglund, L., and Petersen, T.E. (1995). "Cloning of a cDNA encoding murine tetranectin". Gene 152: 243-245
Torgersen, D., Mullin, N.P., and Drickamer, K. (1998). "Mechanism of ligand binding to E- and P-selectin analyzed using selectin/mannose-binding protein chimeras". J. Biol. Chem. 273: 6254-6261
Tormo, J., Natarajan, K., Margulies, D.H., and Mariuzza, R.A. (1999). "Crystal structure of a lectin-like natural killer cell receptor bound to its MHC class I ligand". Nature 402: 623-631
Tsunezawa, W., Sano, H., Sohma, H., McCormack, F.X., Voelker, D.R., and Kuroki, Y. (1998). "Site-directed mutagenesis of surfactant protein A reveals dissociation of lipid aggregation and lipid uptake by alveolar type II cells". Biochim. Biophys. Acta 1387: 433-446
Weis, W.I., Kahn, R., Fourme, R., Drickamer, K., and Hendrickson, W.A. (1991). "Structure of the calcium-dependent lectin domain from a rat mannose-binding protein determined by MAD phasing". Science 254: 1608-1615
Weis, W.I., and Drickamer, K. (1996). "Structural basis of lectin-carbohydrate recognition". Annu. Rev. Biochem. 65: 441-473
Whitehorn, E.A., Tate, E., Yanofsky, S.D., Kochersperger, L., Davis A., Mortensen, R.B., Yonkovic, S., Bell, K., Dower, W.J., and Barrett, R.W. (1995). "A generic method for expression and use of "tagged" soluble versions of cell surface receptors". Bio/Technology 13: 1215-1219
Wragg, S. and Drickamer, K. (1999). "Identification of amino acid residues that determine pH dependence of ligand binding to the asialoglycoprotein receptor during endocytosis". J. Biol. Chem. 274: 35400-35406
Zhang, H., Robison, B., Thorgaard, G.H., and Ristow, S.S. (2000). "Cloning, mapping and genomic organization of a fish C-type lectin gene from homozygous clones of rainbow trout (Oncorhynchos Mykiss)". Biochim. et Biophys. Acta 1494: 14-22

### EMBODIMENTS

The present invention also relates to the following embodiments:
Embodiment 1. A protein having the scaffold structure of C-type lectin-like domains (CTLD), said protein comprising a variant of a model CTLD wherein the α-helices and β-strands and connecting segments are conserved to such a degree that the scaffold structure of the CTLD is substantially maintained, while the loop region is altered by amino acid substitution, deletion, insertion or any combination thereof, with the proviso that said protein is not any of the known CTLD loop derivatives of C-type lectin-like proteins or C-type lectins listed in Table 2 in the description.
Embodiment 2. A protein according to embodiment 1 wherein the model CTLD is defined by having a 3D structure that conforms to the secondary-structure arrangement illustrated in Fig 1 characterized by the following main secondary structure elements:
   five β-strands and two α-helices sequentially appearing in the order β1, α1, α2, β2, β3, β4, and β5, the (β-strands being arranged in two anti-parallel α-sheets, one composed of β1 and β5, the other composed of β2, β3 and β4,
   at least two disulfide bridges, one connecting α1 and β5 and one connecting β3 and the polypeptide segment connecting β4 and β5, and
   a loop region consisting of two polypeptide segments, loop segment A (LSA) connecting β2 and β3 and comprising typically 15-70 or, less typically, 5-14 amino acid residues, and loop segment B (LSB) connecting β3 and β4 and comprising typically 5-12 or less typically, 2-4 amino acid residues.
Embodiment 3. A protein according to embodiment 1 wherein the model CTLD is defined by showing sequence similarity to a previously recognised member of the CTLD family as expressed by an amino acid sequence identity of at least 22 %, preferably at least 25 % and more preferably at least 30 %, and by containing the cysteine residues necessary for establishing the conserved two-disulfide bridge topology (i.e. CYS_{I}, Cys_{II}, Cys_{III} and Cys_{IV}), whereas the loop region and its flanking (β-strand structural elements are identified by inspection of the sequence alignment with the collection of CTLDs shown in Fig. 1 providing identification of the sequence locations of the β2- and (β3-strands with the further corroboration provided by comparison of these sequences with the four-residue consensus sequences, β2cseq and β3cseq, the β4 strand segment being located typically at positions -6 to - 2 and less typically at positions -5 to -2 relative to the conserved Cys_{III} residue and with the characteristic residues at positions -5 and -3 as elucidated from Table 1 and deducted in the description.
Embodiment 4. A protein according to any one of the preceding embodiments wherein up to 10, preferably up to 4, and more preferably 1 or 2, amino acid residues are substituted, deleted or inserted in the α-helices and/or β-strands and/or connecting segments of the model CTLD.
Embodiment 5. A protein according to any one of the preceding embodiments wherein changes of up to 4 residues are made in the β-strands of the model CTLD as a consequence of the introduction of recognition sites for one or more restriction endonucleases in the nucleotide sequence encoding the CTLD to facilitate the excision of part or all of the loop region and the insertion of an altered amino acid sequence instead while the scaffold structure of the CTLD is substantially maintained.
Embodiment 6. A protein according to any one of the preceding embodiments wherein the model CTLD is that of a tetranectin.
Embodiment 7. A protein according to embodiment 6 wherein the model CTLD is that of human tetranectin comprising amino acid residues 59 to 180 in the monomer native peptide sequence thereof.
Embodiment 8. A protein according to embodiment 6 wherein the model CTLD is that of murine tetranectin comprising amino acid residues 59 to 180 in the monomer native peptide sequence thereof.
Embodiment 9. A protein according to any one of the preceding embodiments which further comprises N-terminal and/or C-terminal extensions of the CTLD variant.
Embodiment 10. A protein according to embodiment 9 wherein said N-terminal and/or C-terminal extensions contain effector, enzyme, further binding and/or multimerising functions.
Embodiment 11. A protein according to embodiment 9 or 10 wherein said N-terminal and/or C-terminal extensions are the non-CTLD-portions of a native C-type lectin-like protein or C-type lectin or a "soluble" variant thereof lacking a functional transmembrane domain.
Embodiment 12. A protein according to any one of the preceding embodiments which is a multimer of a moiety comprising the CTLD variant.
Embodiment 13. A protein according to embodiment 12 which is derived from the native tetranectin trimer.
Embodiment 14. A protein according to embodiment 7 which is derived from the polypeptide htlec having the amino acid sequence from position 5 Glu to position 185 Val in SEQ IN NO:13 by altering the amino acid sequence of the loop region.
Embodiment 15. A protein according to embodiment 7 which is derived from the polypeptide htCTLD having the amino acid sequence from position 5 Ala to position 141 Val in SEQ IN NO:15 by altering the amino acid sequence of the loop region.
Embodiment 16. A protein according to embodiment 7 which is derived from the polypeptide hTN having the amino acid sequence from position 5 Glu to position 185 Val in SEQ IN NO:09 by altering the amino acid sequence of the loop region.
Embodiment 17. A protein according to embodiment 7 which is derived from the polypeptide hTN3 having the amino acid sequence from position 5 Ala to position 141 Val in SEQ IN NO:11 by altering the amino acid sequence of the loop region.
Embodiment 18. A protein according to embodiment 8 which is derived from the polypeptide mtlec having the amino acid sequence from position 5 Glu to position 185 Val in SEQ IN NO:36 by altering the amino acid sequence of the loop region.
Embodiment 19. A protein according to embodiment 8 which is derived from the polypeptide mtCTLD having the amino acid sequence from position 5 Ala to position 141 Val in SEQ IN NO:38 by altering the amino acid sequence of the loop region.
Embodiment 20. A combinatorial library of proteins having the scaffold structure of C-type lectin-like domains (CTLD), said proteins comprising variants of a model CTLD wherein the α-helices and β-strands are conserved to such a degree that the scaffold structure of the CTLD is substantially maintained, while the loop region or parts of the loop region of the CTLD is randomised with respect to amino acid sequence and/or number of amino acid residues.
Embodiment 21. A combinatorial library according to embodiment 20 wherein the model CTLD is defined by having a 3D structure that conforms to the secondary-structure arrangement illustrated in Fig. 1 characterised by the following main secondary structure elements:
   five β-strands and two α-helices sequentially appearing in the order β1, α1, α2, β2, β3, β4, and β5, the β-strands being arranged in two anti-parallel β-sheets, one composed of β1 and β5, the other composed of β2, β3 and β4,
   at least two disulfide bridges, one connecting α1 and β5 and one connecting β3 and the polypeptide segment connecting β4 and β5, and
   a loop region consisting of two polypeptide segments, loop segment A (LSA) connecting β2 and β3 and comprising typically 15-70 or, less typically, 5-14 amino acid residues, and loop segment B (LSB) connecting β3 and β4 and comprising typically 5-12 or less typically, 2-4 amino acid residues.
Embodiment 22. A combinatorial library according to embodiment 20 wherein the model CTLD is defined by showing sequence similarity to a previously recognised member of the CTLD family as expressed by an amino acid sequence identity of at least 22 %, preferably at least 25 % and more preferably at least 30 %, and by containing the cysteine residues necessary for establishing the conserved two-disulfide bridge topology (i.e. Cys_{I}, Cys_{II}, Cys_{III} and Cys_{IV}), whereas the loop region and its flanking β-strand structural elements are identified by inspection of the sequence alignment with the collection of CTLDs shown in Fig. 1 providing identification of the sequence locations of the β2- and β3-strands with the further corroboration provided by comparison of these sequences with the four-residue consensus sequences, β2cseq and β3cseq, the β4 strand segment being located typically at positions -6 to - 2 and less typically at positions -5 to -2 relative to the conserved Cys_{III} residue and with the characteristic residues at positions -5 and -3 as elucidated from Table 1 and deducted in the description.
Embodiment 23. A combinatorial library according to any one of embodiments 20-22 of proteins comprising CTLD variants wherein up to 10, preferably up to 4, and more preferably 1 or 2, amino acid residues are substituted, deleted or inserted in the α,-helices and β-strands and connecting segments of the model CTLD.
Embodiment 24. A combinatorial library according to any one of embodiments 20-23 of proteins comprising CTLD variants wherein changes of up to 4 residues are made in the model CTLD as a consequence of the introduction of recognition sites for one or more restriction endonucleases in the nucleotide sequence encoding the CTLD to facilitate the excision of part or all of a DNA segment encoding the loop region and the insertion of members of an ensemble of DNA fragments that collectively encode a randomised amino acid sequence instead while the scaffold structure of the CTLD is substantially maintained.
Embodiment 25. A combinatorial library according to any one of embodiments 20-24 of proteins wherein the model CTLD is that of a tetranectin.
Embodiment 26. A combinatorial library according to embodiment 25 of proteins wherein the model CTLD is that of human tetranectin comprising amino acid residues 59 to 180 in the monomer native peptide sequence thereof.
Embodiment 27. A combinatorial library according to embodiment 25 of proteins wherein the model CTLD is that of murine tetranectin comprising amino acid residues 59 to 180 in the monomer native peptide sequence thereof.
Embodiment 28. A combinatorial library according to any one of embodiments 20-27 of proteins which further comprise N-terminal and/or C-terminal extensions of the CTLD-variant.
Embodiment 29. A combinatorial library according to embodiment 28 of proteins wherein said N-terminal and/or C-terminal extensions contain effector, enzyme, further binding and/or multimerising functions.
Embodiment 30. A combinatorial library according to embodiment 28 or 29 of proteins wherein said N-terminal and/or C-terminal extensions are the non-CTLD-portions of a native C-type lectin-like protein or C-type lectin or a "soluble" variant thereof lacking a functional transmembrane domain.
Embodiment 31. A combinatorial library according to any one of embodiments 20-30 of proteins which are multimers of a moiety comprising the CTLD variant.
Embodiment 32. A combinatorial library according to embodiment 31 of proteins which are derived from the native tetranectin trimer.
Embodiment 33. A combinatorial library according to embodiment 27 of proteins which are derived from the peptide htlec having the amino acid sequence from position 5 Glu to position 185 Val in SEQ IN NO:13 by randomising the loop region with respect to amino acid sequence and/or number of amino acid residues.
Embodiment 34. A combinatorial library according to embodiment 27 of proteins which are derived from the peptide htCTLD having the amino acid sequence from position 5 Ala to position 141 Val in SEQ IN NO:15 by randomising the loop region with respect to amino acid sequence and/or number of amino acid residues.
Embodiment 35. A combinatorial library according to embodiment 27 of proteins which are derived from the peptide hTN having the amino acid sequence from position 5 Glu to position 185 Val in SEQ IN NO:09 by randomising the loop region with respect to amino acid sequence and/or number of amino acid residues.
Embodiment 36. A combinatorial library according to embodiment 27 of proteins which are derived from the peptide hTN3 having the amino acid sequence from position 5 Ala to position 141 Val in SEQ IN NO:11 by randomising the loop region with respect to amino acid sequence and/or number of amino acid residues.
Embodiment 37. A combinatorial library according to embodiment 28 of proteins which are derived from the peptide mtlec having the amino acid sequence from position 5 Glu to position 185 Val in SEQ IN NO:36 by randomising the loop region with respect to amino acid sequence and/or number of amino acid residues.
Embodiment 38. A combinatorial library according to embodiment 28 of proteins which are derived from the peptide mtCTLD having the amino acid sequence from position 5 Ala to position 141 Val in SEQ IN NO:38 by randomising the loop region with respect to amino acid sequence and/or number of amino acid residues.
Embodiment 39. A derivative of a native tetranectin wherein up to 10, preferably up to 4, and more preferably 1 or 2, amino acid residues are substituted, deleted or inserted in the α-helices and/or β-strands and/or connecting segments of its CTLD with the proviso that said derivative is not any of the known CTLD derivatives of human tetranectin (hTN) listed in Table 2 in the description.
Embodiment 40. A derivative of human tetranectin, termed htlec, having the amino acid sequence from position 5 Glu to position 185 Val in SEQ IN NO:13.
Embodiment 41. A derivative of human tetranectin, termed htCTLD, having the amino acid sequence from position 5 Ala to position 141 Val in SEQ IN NO:15.
Embodiment 42. A derivative of human tetranectin, termed PhTN, having the amino acid sequence given in SEQ IN NO:09.
Embodiment 43. A derivative of human tetranectin, termed PhTN3, having the amino acid sequence given in SEQ IN NO:11.
Embodiment 44. A derivative of human tetranectin, termed Phtlec, having the amino acid sequence given in SEQ IN NO:13.
Embodiment 45. A derivative of human tetranectin, termed PhtCTLD, having the amino acid sequence given in SEQ IN NO:15.
Embodiment 46. A derivative of human tetranectin, termed FX-htlec, having the amino acid sequence given in SEQ IN NO:02.
Embodiment 47. A derivative of human tetranectin, termed FX-htCTLD, having the amino acid sequence given in SEQ IN NO:04.
Embodiment 48. A derivative of murine tetranectin, termed mtlec, having the amino acid sequence from position 5 Glu to position 185 Val in SEQ IN NO:36.
Embodiment 49. A derivative of murine tetranectin, termed mtCTLD, having the amino acid sequence from position 5 Ala to position 141 Val in SEQ IN NO:38.
Embodiment 50. A derivative of murine tetranectin, termed Pmtlec, having the amino acid sequence given in SEQ IN NO:36.
Embodiment 51. A derivative of murine tetranectin, termed PmtCTLD, having the amino acid sequence given in SEQ IN NO:38.
Embodiment 52. A derivative of murine tetranectin, termed FX-mtlec, having the amino acid sequence given in SEQ IN NO:29. Embodiment 53. A derivative of murine tetranectin, termed FX-mtCTLD, having the amino acid sequence given in SEQ IN NO:31.
Embodiment 54. Nucleic acid comprising a nucleotide sequence encoding a htlec insert as shown from nucleotide 20 to nucleotide 562 in SEQ ID NO:12.
Embodiment 55. Nucleic acid comprising a nucleotide sequence encoding a htCTLD insert as shown from nucleotide 20 to nucleotide 430 in SEQ ID NO:14.
Embodiment 56. Nucleic acid comprising a nucleotide sequence encoding a mtlec insert as shown from nucleotide 20 to nucleotide 562 in SEQ ID NO:35.
Embodiment 57. Nucleic acid comprising a nucleotide sequence encoding a mtCTLD insert as shown from nucleotide 20 to nucleotide 430 in SEQ ID NO:37.
Embodiment 58. Nucleic acid comprising any nucleotide sequence encoding a protein according to any one of embodiments 1-19.
Embodiment 59. A library of nucleic acids encoding proteins of a combinatorial library according to any one of embodiments 20-38, in which the members of the ensemble of nucleic acids, that collectively constitute said library of nucleic acids, are able to be expressed in a display system, which provides for a logical, physical or chemical link between entities displaying phenotypes representing properties of the displayed expression products and their corresponding genotypes.
Embodiment 60. A library of nucleic acids according to embodiment 49, wherein the display system is selected from
   (I) a phage display system such as
      (1) a filamentous phage fd in which the library of nucleic acids is inserted into
         (a) a phagemid vector,
         (b) the viral genome of a phage
         (c) purified viral nucleic acid in purified single- or double-stranded form, or
      (2) a phage lambda in which the library is inserted into
         (a) purified phage lambda DNA, or
         (b) the nucleic acid in lambda phage particles; or
   (II) a viral display system in which the library of nucleic acids is inserted into the viral nucleic acid of a eukaryotic virus such as baculovirus; or
   (III) a cell-based display system in which the library of nucleic acids is inserted into, or adjoined to, a nucleic acid carrier able to integrate either into the host genome or into an extrachromosomal element able to maintain and express itself within the cell and suitable for cell-surface display on the surface of
      (a) bacterial cells,
      (b) yeast cells, or
      (c) mammalian cells; or
   (IV) a nucleic acid entity suitable for ribosome linked display into which the library of nucleic acid is inserted; or
   (V) a plasmid suitable for plasmid linked display into which the library of nucleic acid is inserted.
Embodiment 61. A library of nucleic acids according to embodiment 60 wherein said phagemid vector is the vector "pCANTAB 5 E" supplied by Amersham Pharmacia Biotech (code no. 27-9401-01) for use with their "Recombinant Phage Antibody System".
Embodiment 62. A method of preparing a protein according to any one of embodiments 1-19, wherein the protein comprises at least one or more, identical or not identical, CTLD domains with novel loop-region sequences which has (have) been isolated from one or more CTLD libraries by screening or selection.
Embodiment 63. A method of preparing a protein according to embodiment 62, wherein at least one CTLD domain has been further modified by mutagenesis.
Embodiment 64. A method of preparing a protein according to embodiment 62 or 63, wherein the protein containing at least one CTLD domain is assembled from two or more components by chemical or enzymatic coupling or crosslinking.
Embodiment 65. A method of preparing a combinatorial library according to any one of embodiments 20-38 comprising the following steps:
   1) inserting nucleic acid encoding a protein comprising a model CTLD into a suitable vector,
   2) if necessary, introducing restriction endonuclease recognition sites by site directed mutagenesis, said recognition sites being properly located in the sequence at or close to the ends of the sequence encoding the loop region of the CTLD or part thereof,
   3) excising the DNA fragment encoding the loop region or part thereof by use of the proper restriction endonucleases,
   4) ligating mixtures of DNA fragments into the restricted vector, and
   5) inducing the vector to express randomised proteins having the scaffold structure of CTLDs in a suitable medium.
Embodiment 66. A method of constructing a tetranectin derivative adapted for the preparation of a combinatorial library according to any one of embodiments 20-38, wherein the nucleic acid encoding the tetranectin derivative has been modified to generate endonuclease restriction sites within nucleic acid segments encoding β2, β3 or β4, or up to 30 nucleotides upstream or downstream in the sequence from any nucleotide which belongs to a nucleic acid segment encoding β2, β3or β4.
Embodiment 67. The use of a nucleotide sequence encoding a tetranectin, or a derivative thereof wherein the scaffold structure of its CTLD is substantially maintained, for preparing a library of nucleotide sequences encoding related proteins by randomising part or all of the nucleic acid sequence encoding the loop region of its CTLD.
Embodiment 68. The use according to embodiment 67 wherein the nucleotide sequence encodes a mammalian tetranectin,
Embodiment 69. The use according to embodiment 67 wherein the nucleotide sequence encodes human or murine tetranectin. Embodiment 70. The use according to embodiment 67 wherein the nucleotide sequence encodes a derivative of a native tetranectin wherein up to 10, preferably up to 4, and more preferably 1 or 2, amino acid residues are substituted, deleted or inserted in the α-helices and β-strands and connecting segments of its CTLD.
Embodiment 71. The use according to embodiment 67 wherein the nucleotide sequence encodes a derivative of human tetranectin, termed htlec, as shown from nucleotide 20 to nucleotide 562 in SEQ ID NO:12.
Embodiment 72. The use according to embodiment 67 wherein the nucleotide sequence encodes a derivative of human tetranectin, termed htCTLD, as shown from nucleotide 20 to nucleotide 430 in SEQ ID NO:14.
Embodiment 73. The use according to embodiment 67 wherein the nucleotide sequence encodes a derivative of murine tetranectin, termed mtlec, as shown from nucleotide 20 to nucleotide 562 in SEQ ID NO:35.
Embodiment 74. The use according to embodiment 67 wherein the nucleotide sequence encodes a derivative of murine tetranectin, termed mtCTLD, as shown from nucleotide 20 to nucleotide 430 in SEQ ID NO:37.
Embodiment 75. A method of screening a combinatorial library according to any one of embodiments 20-38 for binding to a specific target which comprises the following steps:
   1) expressing a nucleic acids library according to any one of embodiments 59-61 to display the library of proteins in the display system;
   2) contacting the collection of entities displayed with a suitably tagged target substance for which isolation of a CTLD-derived exhibiting affinity for said target substance is desired;
   3) harvesting subpopulations of the entities displayed that exhibit affinity for said target substance by means of affinity-based selective extractions, utilizing the tag to which said target substance is conjugated or physically attached or adhering to as a vehicle or means of affinity purification, a procedure commonly referred to in the field as "affinity panning", followed by re-amplification of the sub-library;
   4) isolating progressively better binders by repeated rounds of panning and re-amplification until a suitably small number of good candidate binders is obtained; and,
   5) if desired, isolating each of the good candidates as an individual clone and subjecting it to ordinary functional and structural characterisation in preparation for final selection of one or more preferred product clones.
Embodiment 76. A method of reformatting a protein according to any one of embodiments 1-19 or selected from a combinatorial library according to any one of embodiments 20-38 and containing a CTLD variant exhibiting desired binding properties, in a desired alternative species-compatible framework by excising the nucleic acid fragment encoding the loop region-substituting polypeptide and any required single framework mutations from the nucleic acid encoding said protein using PCR technology, site directed mutagenesis or restriction enzyme digestion and inserting said nucleic acid fragment into the appropriate location(s) in a display- or protein expression vector that harbours a nucleic acid sequence encoding the desired alternative CTLD framework.

## Claims

1. A method for identifying a CTLD polypeptide variant capable of specifically binding to a target molecule comprising the steps of screening a combinatorial library comprising a collection of variant CTLD polypeptides having the scaffold structure of a C-type lectin-like domain (CTLD) and a randomized loop region, wherein the scaffold structure comprises α-helices, β-strand, and connecting segments that are conserved to such a degree that the scaffold structure of the CTLD is substantially maintained and the randomized loop region is altered from the wildtype sequence by random amino acid substitution, deletion, insertion, or any combination thereof, and identifying a polypeptide variant that specifically binds to the target molecule.

2. The method of claim 1 comprising the steps of:
(1) providing a combinatorial library comprising a collection of variant CTLD polypeptides having the scaffold structure of a C-type lectin-like domain (CTLD) and a randomized loop region, wherein the scaffold structure comprises α-helices, β-strand, and connecting segments that are conserved to such a degree that the scaffold structure of the CTLD is substantially maintained and the randomized loop region is altered from the wildtype sequence by random amino acid substitution, deletion, insertion, or any combination thereof;
(2) contacting the collection of polypeptides in step (1) with said target molecule; and
(3) identifying a polypeptide that is capable of specifically binding to said target molecule from the collection of variant CTLD polypeptides in step (2).

3. The method according to claim or claim 2, wherein the scaffold structure of the variant CTLD polypeptides of the combinatorial library comprises the following structural elements:
(a) five β-strand and two α-helices sequentially appearing in the order β1, α1, α2, β2, β3, β4, and β5, the β-strand being arranged in two anti-parallel β-sheets, one (β-sheer composed of β1 and β5, the other β-sheet composed of β2, β3 and β4, and
(b) at least two disulfide bridges, one connecting α1 and β5 and one connecting β3 and a polypeptide segment connecting β4 and β5
and wherein the randomized loop region comprises two loop polypeptide segments: loop segment A (LSA) connecting β2 and β3 and comprising loops 1-4, and loop segment B (LSB) connecting β3 and β4 and comprising loop 5.

4. The method according to any of claims 1-3, wherein the amino acid residues differ between different members of said collection of variant CTLD polypeptides at least at two amino acid sequence positions in the loop region.

5. The method according to any of claims 1-7, wherein at least one amino acid residue is substituted in, deleted from, or inserted into the α-helices and/or β-strands and/or connecting segments in the CTLD scaffold structure in each of the variant CTLD polypeptides of the library.

6. The method according to any of claims 1-5, wherein the collection of variant CTLD polypeptides is derived from a polypeptide selected from the group consisting of tetranectin, mannose binding protein, surfactant protein-D, the NK receptor (LY49A), H1 subunit of the asialoglycoprotein receptor (HI-ASR), macrophage mannose receptor domain 4 (MMR-4), coagulation factors IX/X-binding protein, lithostatine (Lit), and tunicate C-type lectin (TU14).

7. The method according to any of claims 1-6, wherein the variant CTLD polypeptides of the library further comprise N-terminal and/or C-terminal extensions of the CTLD.

8. The method according to claim 7, wherein said N-terminal and/or C-terminal extensions contain effector, enzyme, further binding, and/or multimerising functions.

9. The method according to claim 7, wherein said N-terminal and/or C-terminal extensions are the non-CTLD portions of a native C-type lectin-like protein, a C-type lectin, or a C-type lectin lacking a functional transmembrane domain.

10. A method for the production of a CTLD polypeptide variant comprising the scaffold structure of a CTLD and a randomized loop region, wherein the CTLD scaffold is derived from a different polypeptide than the randomized loop region, comprising the steps of:
(1) providing a combinatorial library comprising a collection of variant CTLD nucleic acids encoding an ensemble of polypeptides having the scaffold structure of a C-type lectin-like domain (CTLD) and a randomized loop region, wherein the scaffold structure comprises α-helices, β-strand, and connecting segments that are conserved to such a degree that the scaffold structure of the CTLD is substantially maintained and the randomized loop region is altered from the wildtype sequence by random amino acid substitution, deletion, insertion, or any combination thereof;
(2) expressing the collection of nucleic acids to produce an ensemble of polypeptides;
(3) contacting the ensemble of polypeptides in step (2) with a target molecule of interest;
(4) identifying a polypeptide that binds specifically to said target molecule from the ensemble of variant CTLD polypeptides in step (3);
(5) excising a nucleic acid fragment encoding the loop region from the nucleic acid encoding the polypeptide identified in step (4); and
(6) inserting the nucleic acid fragment excised in step (5) into the appropriate location in an expression vector that contains a nucleic acid encoding a CTLD scaffold structure, wherein the CTLD scaffold structure is derived from a different polypeptide than that of the randomized loop region.

11. A variant CTLD polypeptide having the scaffold structure of a C-type lectin-like domain and a CTLD loop region, produced by the method of any of claims 1-10.

12. The variant CTLD polypeptide of claim 11 produced by a method comprising the steps of:
(1) providing a combinatorial library comprising a collection of variant CTLD polypeptides having the scaffold structure of a C-type lectin-like domain (CTLD) and randomized loop region, wherein the scaffold structure comprises α-helices, β-strand, and connecting segments that are conserved to such a degree that the scaffold structure of the CTLD is substantially maintained and the randomized loop region is altered from the wildtype sequence by random amino acid substitution, deletion, insertion, or any combination thereof;
(2) contacting the collection of polypeptides in step (1) with a target molecule of interest; and
(3) isolating a polypeptide that specifically binds to said target molecule from the collection of variant CTLD polypeptides in step (2).

13. The variant CTLD polypeptide of claim 11 or claim 12, wherein the loop region of the variant CTLD polypeptide is derived from human tetranectin (hTN), mannose binding protein (MBP), or surfactant protein D (SP-D) and contains at least two amino acid mutations in one or more of loop 1, loop 2, loop 3, loop 4, and/or loop 5 of the loop region.

14. The variant CTLD polypeptide of any of claims 11-13, wherein the mutant polypeptide binds to a non-natural target of human tetranectin, mannose binding protein, or surfactant protein D with detectable affinity.

15. The variant CTLD polypeptide of any of claims 11-14, wherein the polypeptide is additionally modified by mutagenesis.

16. The variant CTLD polypeptide of any of claims 11-15, wherein the polypeptide is assembled from two or more components by chemical or enzymatic coupling or crosslinking.

17. A mutant human tetranectin (hTN) polypeptide having the scaffold structure of a C-type lectin-like domain and a CTLD loop region comprising two loop polypeptide segments: loop segment A (LSA) comprising loops 1-4, and loop segment B (LSB) comprising loop 5, wherein the mutant polypeptide has amino acid mutations in one or more of loop 1, loop 2, loop 3, loop 4, and/or loop 5 of the hTN CTLD loop region.

18. The mutant hTN polypeptide of claim 17, wherein the mutant polypeptide binds to a non-natural target of tetranectin with detectable affinity.

19. The mutant tetranectin polypeptide of claim 17 or claim 18, wherein the CTLD scaffold comprises the following structural elements:
five β-strand and two α-helices sequentially appearing in the order β1, αI, α2, β2, β3, β4, and β5, the β-strand being arranged in two anti-parallel β-sheets, one β-sheet composed of βI and β5, the other β-sheet composed of β2, β3 and β4 and
at least two disulfide bridges, one disulfide bridge connecting αI and β5 and one disulfide bridge connecting β3 and a polypeptide segment connecting β4 and β5;
and wherein the randomized CTLD loop region consists of two loop polypeptide segments, loop segment A (LSA) connecting β2 and β3, and loop segment B (LSB) connecting β3 and β4.

20. The mutant tetranectin polypeptide of any of claims 17-19, wherein the amino acid mutations are at two or more of any of amino acids 73-78, 93-105, and 114-117 with respect to the loop region of the wildtype amino acid sequence of human tetranectin as set forth in hTN in Figure 1.

21. A mutant mannose binding protein polypeptide having the scaffold structure of a C-type lectin-like domain and a CTLD loop region comprising amino acid mutations with respect to the loop region of the wildtype amino acid sequence of mannose binding protein as set forth in MBP in Figure 1, wherein the amino acid mutations are at two or more of any of amino acids 66, 67, 68, 69, 70, 71, 72, 93, 94, 95, 96, and 97.

22. A mutant surfactant protein-D polypeptide having the scaffold structure of a C-type lectin-like domain and a CTLD loop region comprising amino acid mutations with respect to the loop region of the wildtype amino acid sequence of human surfactant protein-D as set forth in SP-D in Figure 1, wherein the amino acid mutations are at two or more of any of amino acids 69, 70, 71, 72, 73, 74, 75, 96, 97, 98, 99, and 100.
